(19) Europäisches Patentamt
European Patent Office
Office européen des brevets

(11) Veröffentlichungsnummer: **0 146 893**
**B1**

(12) # EUROPÄISCHE PATENTSCHRIFT

(45) Veröffentlichungstag der Patentschrift: **24.08.88**

(51) Int. Cl.$^4$: **C 07 D 279/16,** C 07 D 417/12, A 61 K 31/54

(21) Anmeldenummer: **84115466.9**

(22) Anmeldetag: **14.12.84**

(54) **Neue Benzothiazin-Derivate, Verfahren zu ihrer Herstellung, sie enthaltende Arzneimittel und deren Verwendung.**

(30) Priorität: **27.12.83 DE 3347173**

(43) Veröffentlichungstag der Anmeldung:
**03.07.85 Patentblatt 85/27**

(45) Bekanntmachung des Hinweises auf die Patenterteilung:
**24.08.88 Patentblatt 88/34**

(84) Benannte Vertragsstaaten:
**AT BE CH DE FR GB IT LI LU NL SE**

(56) Entgegenhaltungen:
**EP - A - 0 116 368**
**DE - A - 1 545 702**
**DE - A - 2 912 445**
**GB - A - 1 304 588**
**GB - A - 1 373 537**

**JOURNAL OF MEDICINAL CHEMISTRY, Band 6, 1963; J. KRAPCHO et al. "Synthesis of Substituted 2-Phenyl-1,4-benzothiazin-3(4H)-ones and their Activity as Inhibitors of 1,4-Dipyrrolidino-2-butyne" Seiten 214-216**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **Henning, Rainer, Dr., Rotenhofstrasse 31, D-6234 Hattersheim am Main (DE)**
Erfinder: **Lerch, Ulrich, Dr., Schwalbenweg 19, D-6238 Hofheim am Taunus (DE)**
Erfinder: **Kaiser, Joachim, Dr., Fichtestrasse 12, D-6000 Frankfurt am Main (DE)**

EP 0 146 893 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Es ist bekannt, dass Verbindungen, die das Einströmen von Calcium-Ionen in Zellen behindern, als Therapeutika zur Behandlung von verschiedenen Krankheiten, insbesondere des Herz-Kreislauf-Systems beim Menschen und anderen Warmblütern eingesetzt werden können (siehe z. B. R. A. Janis und D.J. Triggle, J. Med. Chem. 26, 775 (1983)).

In der deutschen Offenlegungsschrift 2 912 445 werden 2-Methylen-2,3-dihydro-3-oxo-1,4-benzothiazin-1,1-dioxid-Verbindungen zur Verwendung bei der Bekämpfung von Herz- und Kreislaufleiden, insbesondere von Herzrhythmusstörungen beschrieben.

Die britische Patentschrift 1 374 283 beschreibt Benzothiazin-Derivate mit tranquillisierender, depressiver und antibakterieller Wirkung.

In der britischen Patentschrift 1 388 054 werden Antiinflammatorika mit Benzothiazinstruktur beschrieben; ähnliche Verbindungen finden sich auch im J. Med. Chem. 12 (1969) 290–294.

In der britischen Patentschrift 1 373 537 finden sich Aminoalkyl-benzyliden)-2H-benzothiazin-3(4H)-one und verwandte Verbindungen mit antidepressiver Wirkung.

In J. Med. Chem 6 (1963) 214–216 werden substituierte 2-Phenyl-1,4-benzothiazin-3(4H)-one als Inhibitoren von 1,4-Dipyrrolidino-2-butin erwähnt.

Schliesslich werden in J. Med. Chem. 16 (1973) 776–779 1,4-Benzothiazin-Derivate mit potentieller antihypertensiver Wirkung beschrieben.

In der prioritätsälteren europäischen Patentanmeldung Veröffentlichungs-Nr. 116 368 A1 werden Benzothiazinonderivate mit den erfindungsgemässen Verbindungen ähnlicher Struktur beschrieben. Diese bereits vorgeschlagenen Verbindungen unterscheiden sich von den erfindungsgemässen Verbindungen jedoch durch das Fehlen des Substituenten in 2-Stellung des Benzothiazinongerüstes, d. h. des Substituenten R(3) nach der Erfindung.

Gegenstand der vorliegenden Erfindung sind Benzothiazin-Derivate der Formel I, die eine solche Wirkung aufweisen,

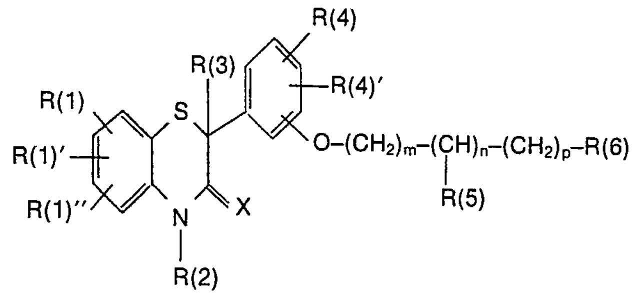

(I)

und in welcher

R(1), R(1)′ und R(1)″ gleich oder verschieden und voneinander unabhängig sind und Wasserstoff, $(C_1–C_4)$-Alkyl, $(C_1–C_3)$-Alkoxy, Halogen, Nitro, Hydroxy, Acetamido oder Amino,

R(2) Wasserstoff, $(C_1–C_{10})$-Alkyl, geradkettig oder verzweigt, $(C_3–C_{10})$-Alkenyl, geradkettig oder verzweigt, Phenyl, das gegebenenfalls durch einen, zwei oder drei Substituenten der Gruppe $(C_1–C_4)$-Alkyl, $(C_1–C_3)$-Alkoxy, Halogen, $(C_1–C_2)$-Alkylendioxy oder Nitro substituiert sein kann, Phenyl-$(C_1–C_4)$-alkyl, wobei der Phenylring durch einen, zwei oder drei Substituenten der Gruppe $(C_1–C_4)$-Alkyl, $(C_1–C_3)$-Alkoxy, Halogen, $(C_1–C_2)$-Alkylendioxy oder Nitro substituiert sein kann, $(C_4–C_8)$-Cycloalkyl-$(C_1–C_4)$-alkyl, $(C_4–C_8)$-Cycloalkyl,

R(3), $(C_1–C_{10})$-Alkyl, geradkettig oder verzweigt, $(C_3–C_{10})$-Alkenyl, geradkettig oder verzweigt, Phenyl-$(C_1–C_4)$-alkyl, wobei der Phenylrest durch einen, zwei oder drei Substituenten der Gruppe $(C_1–C_4)$-Alkyl, $(C_1–C_3)$-Alkoxy, Halogen, $(C_1–C_2)$-Alkylendioxy oder Nitro substituiert sein kann, $(C_4–C_8)$-Cycloalkyl, $(C_4–C_8)$-Cycloalkyl-$(C_1–C_4)$-alkyl,

R(4) und R(4)′ gleich oder verschieden und voneinander unabhängig Wasserstoff, $(C_1–C_4)$-Alkyl, $(C_1–C_3)$-Alkoxy, Halogen, Nitro, Hydroxy, Acetamido oder Amino,

R(5) Wasserstoff oder $(C_1–C_3)$-Alkyl,

R(6) eine Teilstruktur aus der folgenden Gruppe,

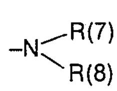

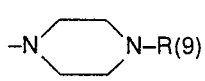

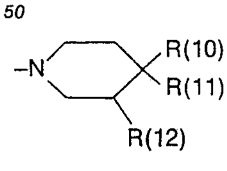

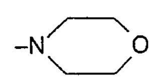

worin

R(7) und R(8) gleich oder verschieden voneinander unabhängig Wasserstoff, $(C_1–C_{10})$-Alkyl, $(C_4–C_8)$-Cycloalkyl, $(C_4–C_8)$-Cycloalkyl-$(C_1–C_4)$-alkyl, Phenyl-$(C_1–C_6)$-alkyl, wobei der Phenylrest durch einen, zwei oder drei Reste aus der Gruppe $(C_1–C_4)$-Alkyl, $(C_1–C_4)$-Alkoxy, $(C_1–C_2)$-Alkylendioxy, Halogen oder Hydroxy substituiert sein kann, Pyridyl-$(C_1–C_4)$-alkyl,

R(9) Wasserstoff, $(C_1–C_{10})$-Alkyl, geradkettig oder verzweigt, Phenyl, Phenyl-$(C_1–C_4)$-alkyl, wobei der Phenylrest jeweils durch einen, zwei oder drei Reste aus der Gruppe $(C_1–C_4)$-Alkyl, $(C_1–C_4)$-Alkoxy, $(C_1–C_2)$-Alkylendioxy, Halogen oder Hydroxy substituiert sein kann, Pyridyl, Pyrimidinyl, $(C_1–C_5)$-Alkanoyl, Phenyl-$(C_1–C_4)$-alkanoyl, Benzoyl, wobei der Phenylrest jeweils durch einen, zwei oder drei Reste aus der Gruppe $(C_1–C_4)$-

Alkyl, ($C_1$–$C_4$)-Alkoxy, ($C_1$–$C_2$)-Alkylendioxy, Halogen oder Hydroxy substituiert sein kann,

R(10) Wasserstoff, ($C_1$–$C_{10}$)-Alkyl, Phenyl, Phenyl-($C_1$–$C_4$)-alkyl, wobei der Phenylrest jeweils durch einen, zwei oder drei Reste aus der Gruppe ($C_1$–$C_4$)-Alkyl, ($C_1$–$C_4$)-Alkoxy, ($C_1$–$C_2$)-Alkylendioxy, Halogen oder Hydroxy substituiert sein kann,

R(11) Wasserstoff, Hydroxy, ($C_1$–$C_4$)-Alkoxy oder zusammen mit R(12) eine Bindung, und

R(12) Wasserstoff oder zusammen mit R(11) eine Bindung bedeuten;
in welcher Formel I weiterhin

m 1, 2, 3 oder 4,
n 0 oder 1,
p 0, 1, 2, 3 oder 4, und
X Sauerstoff bedeuten,

sowie die Salze der Verbindungen der Formel I mit physiologisch verträglichen Säuren.

Bevorzugt werden Verbindungen der Formel I, in welcher

R(1) und R(1)′ gleich oder verschieden sind und voneinander unabhängig Wasserstoff, Methyl, Ethyl, Methoxy, Ethoxy, Chlor, Brom, Nitro oder Acetamido,

R(1)″ Wasserstoff,

R(2) Wasserstoff, ($C_1$–$C_6$)-Alkyl, geradkettig oder verzweigt, Benzyl, Phenethyl, Allyl, Phenyl, 4-Methoxyphenyl, 3-Methoxyphenyl, 3,4-Methylendioxyphenyl, 3,4,5-Trimethoxyphenyl 3,4-Dimethoxyphenyl, Cyclohexylmethyl, Cyclopentyl, Cyclohexyl, Cycloheptyl, 4-Methoxybenzyl, 3,4-Dimethoxybenzyl, 3,4,5-Trimethoxybenzyl, 3,4-Methylendioxybenzyl,

R(3) ($C_1$–$C_6$)-Alkyl, geradkettig oder verzweigt, Benzyl, Phenylethyl, Allyl, Cyclopentyl, Cyclohexyl,

R(4) Wasserstoff, Methyl, Methoxy, Ethoxy, Chlor, Nitro, Hydroxy, Acetamido oder Amino,

R(4)′ Wasserstoff,

R(5) Wasserstoff oder Methyl,

R(6) eine Teilstruktur aus der folgenden Gruppe,

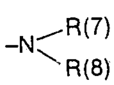
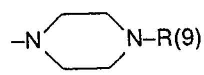
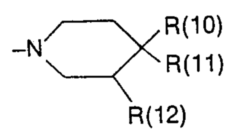

worin

R(7) Wasserstoff, Methyl, Ethyl, Propyl, Isopropyl,

R(8) Wasserstoff, Methyl, Ethyl, Propyl, Isopropyl, Cyclopentylethyl, Cyclohexylethyl, Phenyl-($C_1$–$C_4$)-alkyl, wobei der Phenylrest durch einen, zwei oder drei Reste aus der Gruppe ($C_1$–$C_4$)-Alkyl, ($C_1$–$C_4$)-Alkoxy, ($C_1$–$C_2$)-Alkylendioxy, Halogen oder Hydroxy substituiert sein kann, Pyridyl-($C_1$–$C_4$)-alkyl,

R(9) wie oben angegeben definiert ist,

R(10) Phenyl, das gegebenenfalls durch einen, zwei oder drei Reste aus der Gruppe ($C_1$–$C_4$)-Alkyl, ($C_1$–$C_4$)-Alkoxy, ($C_1$–$C_2$)-Alkylendioxy, Halogen oder Hydroxy substituiert sein kann, Phenyl-($C_1$–$C_4$)-alkyl, wobei der Phenylrest durch einen, zwei oder drei Reste aus der Gruppe ($C_1$–$C_4$)-Alkyl, ($C_1$–$C_4$)-Alkoxy, ($C_1$–$C_2$)-Alkylendioxy, Halogen oder Hydroxy substituiert sein kann,

R(11) Wasserstoff und Hydroxy, Methoxy oder zusammen mit R(12) eine Bindung,

R(12) Wasserstoff oder zusammen mit R(11) eine Bindung bedeuten;
in welcher Formel I weiterhin

m 1, 2 oder 3,
n 0 oder 1,
p 1, 2 oder 3, und
X Sauerstoff bedeuten,

sowie die Salze dieser Verbindungen der Formel I mit physiologisch verträglichen Säuren.

Besonders bevorzugt werden Verbindungen der Formel I, in welcher

R(1) Wasserstoff, Methyl, Methoxy, Fluor oder Chlor,

R(1)′ und R(1)″ Wasserstoff,

R(2) Wasserstoff, Methyl, Ethyl, Propyl, Isopropyl, Butyl, sec. Butyl, Isobutyl, Benzyl, Phenethyl, 4-Methoxyphenyl, 3-Methoxyphenyl, 3,4-Dimethoxyphenyl, 3,4,5-Trimethoxyphenyl, Cyclohexylmethyl, Cyclopenthyl, Cyclohexyl, Cycloheptyl, 4-Methoxybenzyl, 3,4-Dimethoxybenzyl, 3,4,5-Trimethoxybenzyl, 3,4-Methylendioxybenzyl,

R(3) Methyl, Ethyl, Propyl, Isopropyl, Butyl, sec. Butyl, Isobutyl, Benzyl, Phenylethyl, Allyl, Cyclopentyl, Cyclohexyl,

R(4) Wasserstoff, Methoxy, Methyl, Chlor, Nitro oder Hydroxy,

R(4)′ Wasserstoff,

R(5) Wasserstoff,

R(6) eine Teilstruktur aus der folgenden Gruppe,

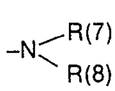
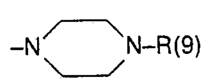
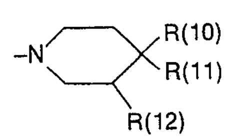

worin

R(7) Wasserstoff oder Methyl,

R(8) Phenyl-($C_1$–$C_4$)-alkyl, wobei der Phenylrest durch einen, zwei oder drei Reste aus der Gruppe Methyl, Methoxy, Chlor, Methylendioxy oder Hydroxy substituiert sein kann,

R(9) wie oben definiert ist,

R(10) Phenyl, wobei der Phenylrest durch einen zwei oder drei Reste aus der Gruppe Methyl, Methoxy, Chlor, Methylendioxy oder Hydroxy substituiert sein kann,

R(11) Wasserstoff, Hydroxy, Methoxy oder zusammen mit R(12) eine Bindung, und

R(12) Wasserstoff oder zusammen mit R(11) eine Bindung bedeuten;
in welcher Formel I weiterhin

m 1, 2 oder 3,
n 0,
p 0, 1 oder 2, und
X Sauerstoff bedeuten,

sowie die Salze dieser Verbindungen der Formel I mit physiologisch verträglichen Säuren.

Als solche Säuren kommen anorganische Säuren wie Salzsäure, Bromwasserstoffsäure, Jodwasserstoffsäure, Schwefelsäure, Phosphorsäure oder Salpetersäure oder organische Säuren wie Weinsäure, Äpfelsäure, Milchsäure, Maleinsäure, Fumarsäure, Malonsäure, Oxalsäure, Gluconsäure, Camphersulfonsäure, Benzolsulfonsäure, Essigsäure, Propionsäure oder p-Toluolsulfonsäure in Betracht.

Insbesondere bevorzugt sind Verbindungen der Formel I wie vorstehend definiert, in denen

R(1), R(1)′ und R(1)″ wie vorstehend definiert sind,

R(2) Wasserstoff, Methyl, Ethyl, Propyl, Isopropyl, Phenyl,

R(3) Methyl, Ethyl, Propyl, Isopropyl, Butyl, sec. Butyl, Isobutyl, Cyclopentyl, Cyclohexyl,

R(4) Wasserstoff, Methoxy, Methyl oder Chlor,

R(4)′ und R(5) wie vorstehend definiert sind,

R(6) eine Teilstruktur aus der folgenden Gruppe

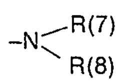

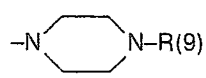

R(7) Methyl,
R(8) wie oben angegeben definiert ist,
R(9) Phenyl-($C_1$–$C_4$)-alkyl, wobei der Phenylrest durch einen, zwei oder drei Reste aus der Gruppe ($C_1$–$C_2$)-Alkoxy, ($C_1$–$C_2$)-Alkylendioxy oder Hydroxy substituiert sein kann,
m 3,
X Sauerstoff,
p 0 oder 1 sind und deren physiologisch verträglichen Salze.

Halogen bedeutet, wenn nicht anders angegeben Fluor oder Chlor.

Die Verbindungen der Formel I weisen asymmetrische C-Atome auf und können daher als Enantiomere oder Diastereomere auftreten. Die Erfindung umfasst sowohl die reinen Isomeren als auch deren Gemische. Gemische von Diastereomeren können nach gebräuchlichen Methoden, zum Beispiel selektive Kristallisation aus geeigneten Lösungsmitteln oder Chromatographie an Kieselgel oder Aluminiumoxid in die Komponenten aufgetrennt werden. Racemate können nach üblichen Methoden gegebenenfalls in die einzelnen Enantiomeren aufgetrennt werden, so zum Beispiel durch Salzbildung mit optisch aktiven Säuren wie Camphersulfonsäure oder Dibenzoylweinsäure und selektive Kristallisation, oder durch Derivatisierung mit geeigneten optisch aktiven Reagenzien, Trennung der diastereomeren Derivate und Rückspaltung.

Die Erfindung betrifft weiterhin ein Verfahren zur Herstellung von Verbindungen der Formel I, das dadurch gekennzeichnet ist, dass man

a) eine Verbindung der Formel II,

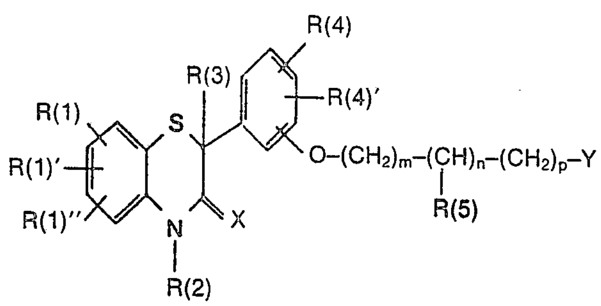

(II)

in welcher R(1), R(1)′, R(1)″, R(2), R(3), R(4), R(4)′, R(5), X, m, n und p die gleiche Bedeutung wie in Formel I haben, und in welcher Y eine Abgangsgruppe, die nucleophil verdrängt werden kann, insbesondere ein Halogenatom, ein Sulfonsäurerest, vorzugsweise einen Methansulfonylrest, einen Benzolsulfonylrest, einen Toluolsulfonylrest oder einen Trifluormethansulfonylrest bedeutet, mit einer der Verbindungen der Formeln IIIa, IIIb, IIIc oder IIId,

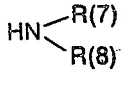

(IIIa)

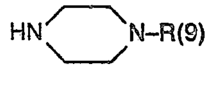

(IIIb)

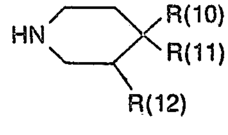

(IIIc)

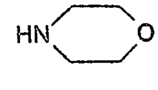

(IIId)

in welchen R(7), R(8), R(9), R(10), R(11) und R(12) die gleiche Bedeutung wie in Formel I haben, unter Bedingungen einer nucleophilen Substitution, vorzugsweise in einem polaren organischen Lösungsmittel wie einem Alkohol, vorzugsweise Methanol, Ethanol, Propanol oder Isopropanol oder einem niederen Keton, vorzugsweise Aceton oder Methylethylketon oder Dimethylformamid,

Dimethylsulfoxid oder Sulfolan oder einen Kohlenwasserstoff, vorzugsweise Toluol, mit oder ohne Gegenwart einer Hilfsbase zum Abfangen der sich bildenden Säure, vorzugsweise in Gegenwart von Kaliumcarbonat, Natriumcarbonat, Triethylamin, N-Ethylmorpholin oder Pyridin, bei einer Temperatur zwischen 0 und 160 °C, vorzugsweise zwischen 20 und 120 °C, umsetzt, oder dass man

b) eine Verbindung der Formel IV,

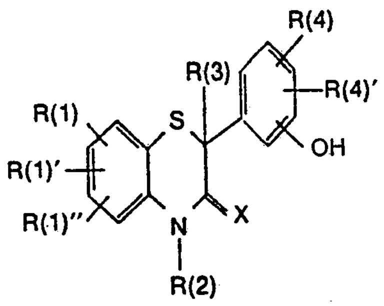

(IV)

in welcher R(1), R(1)', R(1)'', R(2), R(3), R(4), R(4)' und X die gleiche Bedeutung wie in Formel I haben, mit einer Verbindung der Formel V,

$$Z{-}(CH_2)_m{-}\underset{\displaystyle R(5)}{\underset{|}{(CH)_n}}{-}(CH_2)_p{-}R(6) \qquad (V)$$

in welcher Z gleich wie Y in Formel II definiert ist und in welcher R(5), R(6), R(7), R(8), R(9), R(10), R(11), R(12), m, n und p die gleiche Bedeutung wie Formel I haben, entweder in einem polaren aprotischen Lösungsmittel wie Dimethylformamid, Dimethylsulfoxid, Tetrahydrofuran, Sulfolan oder N-Methylpyrrolidon, in Gegenwart einer starken Base wie Natriumhydrid, Kaliumhydrid, Natriumamid, Lithiumdiisopropylamid, Butyllithium oder Lithiumhexamethyldisilazid, bei einer Temperatur zwischen −40 und +60 °C, vorzugsweise zwischen −10 und −30 °C, oder in einem protischen oder aprotischen polaren organischen Lösungsmittel wie einem niederen Alkohol, beispielsweise Methanol, Ethanol, Isopropanol oder einem niederen Keton, vorzugsweise Aceton oder Methylethylketon oder in Dimethylformamid, in Gegenwart einer schwachen bis mittelstarken Base wie einem Alkali- oder Erdalkalimetallhydroxid oder -carbonat oder einem Amin wie beispielsweise Triethylamin, N-Ethylmorpholin, N-Methyldiisopropylamin oder Pyridin, bei einer Temperatur 0 und zwischen 160 °C, vorzugsweise zwischen 20 und 120 °C, umsetzt.

Verbindungen der Formel II, die ebenfalls neu und Gegenstand der Erfindung sind, erhält man aus substituierten Aminothiophenolen der Formel VI,

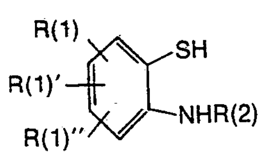

(VI)

in welcher R(1), R(1)', R(1)'' die gleiche Bedeutung wie in Formel I haben, durch Umsetzung mit einer Verbindung der Formel VII,

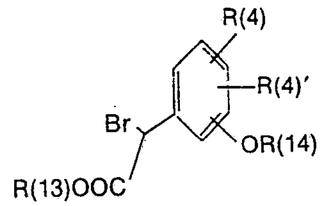

(VII)

in welcher R(4) und R(4)' die gleiche Bedeutung wie in Formel I haben und in welcher R(13) einen Niederalkylrest und R(14) eine unter milden Bedingungen abspaltbare Schutzgruppe wie beispielsweise eine Methyl-, Benzyl- oder Acetylgruppe darstellt, ohne Lösungsmittel oder in einem polaren organischen Lösungsmittel, wie beispielsweise Dimethylformamid, bei 0 bis 60 °C, wobei Verbindungen der Formel VIII erhalten werden (analog Ber. Dt. Chem. Ges. 30, 2389 (1897), und

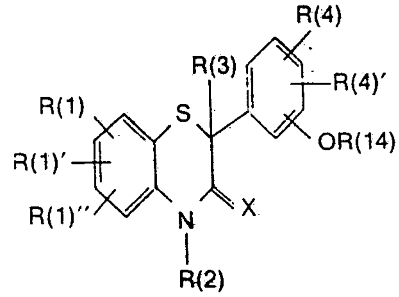

(VIII)

anschliessende Abspaltung der Schutzgruppe R(14) unter geeigneten Bedingungen, so beispielsweise durch katalytische Hydrierung für die Benzylgruppe, Umsetzung mit Bortribromid, Trimethyljodsilan oder Pyridinhydrochlorid für die Methylgruppe, oder Kaliumcarbonat in alkoholischer Lösung für die Acetylgruppe.

Falls Verbindungen der Formel VI mit R(2) = H eingesetzt werden, können Reste R(2), die nicht H bedeuten, in die Verbindungen der Formel VIII durch Alkylierung in Gegenwart einer Base wie beispielsweise Kaliumcarbonat oder durch Arylierung mit einem Halogenaromaten in Gegenwart eines Kupfer-Katalysators eingeführt werden.

In Verbindungen der Formel VIII können Reste R(3) durch Alkylierung mit einem Alkylhalogenid in Gegenwart einer starken Base wie Natriumhydrid oder einem Lithiumamid eingeführt werden. Dabei erhält man Verbindungen der Formel VIIIa mit R(2) = R(3), falls man von Verbindungen der Formel VIII mit R(2) = H ausgeht.

Bei Verwendung von Verbindungen der Formel VIII mit R(2) ≠ H können auch Verbindungen der Formel VIIIa mit R(2) ≠ R(3) erhalten werden.

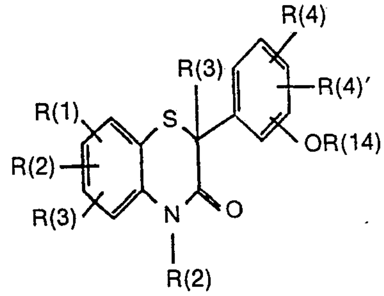

(VIIIa)

Die Verbindungen der Formel IV können unter den für Verfahrensvariante b) beschriebenen Bedingungen mit einer Verbindung der Formel IX,

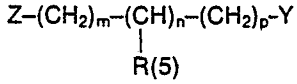

(IX)

in welcher R(5), m, n und p die gleiche Bedeutung wie in Formel I haben, Y die gleiche Bedeutung wie in Formel II hat und Z eine Abgangsgruppe bedeutet, die gleich oder verschieden von Y sein kann und ansonsten die gleiche Bedeutung wie Y hat, zu den Verbindungen der Formel II umgesetzt werden.

Verbindungen der Formel V erhält man in an sich bekannter Weise aus Verbindungen der Formeln IIIa, IIIb, IIIc oder IIId durch Umsetzung mit Verbindungen der Formel IX unter den bei Verfahrensvariante a) beschriebenen Bedingungen.

Verbindungen der Formel VII sind zum Teil aus der Literatur bekannt (siehe z.B. J. Med. Chem. 16, 1043 (1973) oder werden aus den entsprechenden substituierten Mandelsäureestern der Formel X,

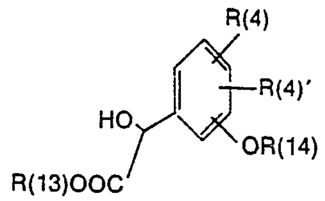

(X)

die man aus den entsprechenden Benzaldehyden der Formel XI über die Cyanhydrine analog Arch. Pharm. 308, 338 (1975) erhält, in analoger Weise durch Umsetzung mit Phosphortribromid erhalten.

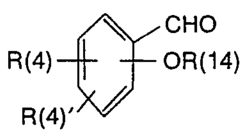

(XI)

Die erfindungsgemässen Verbindungen der Formel I weisen pharmakologische und biochemische Wirkungen, insbesondere calciumantagonistische Wirkungen auf und können daher zur Behandlung aller Krankheitszustände, die auf einer Störung in dem Calciumhaushalt eines Warmblüters beruhen, verwendet werden.

Ihre calciumantagonistische Wirksamkeit kann an dem biochemischen Testmodell der Verdrängung von tritiummarkiertem Nitrendipin gezeigt werden.

Hierbei werden Membranpräparationen, die isolierte Calciumkanäle enthalten, mit der markierten Substanz beladen. Nach Inkubation mit der Testsubstanz wird die freigesetzte Radioaktivität im Überstand bestimmt. In diesem Modell weisen die erfindungsgemässen Verbindungen der Formel I $IC_{50}$-Werte von $10^{-6}$ molar bis $10^{-10}$ molar auf. Die besten der Verbindungen werden in ihrer Wirkung nur von Verbindungen mit Dihydropyridinstruktur übertroffen.

In weiteren Testmodellen, mit denen calciumantagonistische Wirkung nachgewiesen werden kann, z.B. an der Coronardurchströmung am isolierten Meerschweinchenherzen oder am Aktionspotential des isolierten Meerschweinchenpapillarmuskels sind die Verbindungen der Formel I ebenfalls stark wirksam.

Die erfindungsgemässen Verbindungen der Formel I und ihre pharmakologisch verträglichen Salze vermindern das Einströmen von Calcium-Ionen in Zellen und eignen sich daher zu Behandlung des Herz-Kreislaufsystems bei entsprechenden Beschwerden z.B. bei verschiedenen Formen der Angina pectoris, Tachycardie, Herzrhythmusstörungen und Bluthochdruck. Sie sind innerhalb eines breiten Dosisbereichs wirksam. Die Höhe der verabreichten Dosis ist abhängig von der Art der gewünschten Behandlung, von der Verabreichungsweise, vom Zustand, vom Typ und von der Grösse des behandelten Säugers. Bei oraler Dosierung werden befriedigende Ergebnisse mit Dosen von 0,01 bis 100 mg, vorzugsweise 0,1 bis 20 mg, insbesondere 0,5–15 mg, einer Verbindung der Formel I pro kg Körpergewicht erreicht. Beim Menschen variiert die tägliche Dosis zwischen 10 und 800 mg, vorzugsweise 20 bis 500 mg, wobei Einzeldosen von 5 bis 200 mg, insbesondere 5–100 mg, vorzugsweise ein- bis dreimal täglich, gegeben werden können.

Für intravenöse und intramuskuläre Anwendung beträgt die Dosis 1 bis 300 mg, vorzugsweise 5 bis 150 mg täglich.

Die pharmakologisch verwendbaren Verbindungen der vorliegenden Erfindung und ihre Salze können zur Herstellung von pharmazeutischen Präparaten verwendet werden, welche eine wirksame Menge der Aktivsubstanz zusammen mit Trägerstoffen enthalten und die sich zur enteralen und parenteralen Verabreichung eignen. Vorzugsweise verwendet werden Tabletten oder Gelatinekapseln, welche den Wirkstoff zusammen mit Verdünnungsmitteln, z.B. Lactose, Dextrose, Rohrzucker, Mannitol, Sorbitol, Cellulose und/oder Glycin und Gleitmitteln wie Kieselerde, Talk, Stearinsäure oder deren Salze, wie Magnesium- oder Calciumstearat, und/oder Polyethylenglykol enthalten. Tabletten enthalten ebenfalls Bindemittel wie Magnesiumaluminiumsilicat, Stärke, Gelatine, Traganth, Methylcellulose, Natriumcarboxymethylcellulose und/oder Polyvinylpyrrolidon und, falls benötigt, Farbstoffe, Geschmacksstoffe und Süssmittel. Injizierbare Lösungen sind vorzugsweise isotonische wässrige Lösungen oder Suspensionen, die sterilisiert sein können und Hilfsstoffe wie Konservier-, Stabilisierungs-, Netz- und/oder Emulgiermittel, Löslichkeitsvermittler, Salze zur Regulierung des osmotischen Drucks und/oder Puffersubstanzen enthalten können. Die erfindungsgemässen pharmazeutischen Präparate, die, wenn erwünscht, weitere pharmakologisch wertvolle Stoffe enthalten können, werden z.B. mittels konventioneller Misch-, Granulier- und Dragierverfahren, hergestellt und enthalten 0,1% bis etwa 75%, bevorzugt etwa 1% bis etwa 50% des Wirkstoffs.

Die im Anschluss folgenden Beispiele sollen die Erfindung erläutern, ohne sie auf diese Beispiele zu begrenzen.

Beispiel 1

2,3-Dihydro-2,4-dimethyl-2-[2-[4-[N-[2-(3,4-dimethoxyphenyl)-ethyl]-N-methyl]-amino]-butoxy]-phenyl]-benzothiazin-3-on-Hydrochlorid

a) 2-Methoxy-mandelsäure-methylester

65,1 g (1 Mol) Kaliumcyanid und 129,3 g 2-Methoxybenzaldehyd werden mit 775 ml trockenem Diethylether gemischt. Unter gutem Kühlen und heftigem Rühren tropft man 500 ml 2 N HCl zu. Die Etherphase wird abgetrennt und mit 332,5 ml Methanol versetzt. HCl-Gas wird eingeleitet, bis die Lösung gesättigt ist. Der entstandene Niederschlag wird abgesaugt, mit Ether gewaschen und in 600 ml Wasser gelöst. Das entstandene Öl wird mit Ether extrahiert, die Etherphase mit $Na_2SO_4$ getrocknet und eingeengt. Man erhält 109,7 g farbloses Öl.

b) 2-Brom-2-(2-methoxyphenyl)-essigsäure-methylester

109,7 g (0,562 Mol) 2-Methoxy-mandelsäuremethylester werden unter guter Kühlung tropfenweise mit 146,6 ml (1,57 Mol) Phosphortribromid versetzt. Nach 3 Stunden Rühren bei Raumtemperatur wird in 3 N NaCl-Lösung gegossen und mit Methylenchlorid extrahiert. Nach Waschen der organischen Phase mit Wasser wird mit $Na_2SO_4$ getrocknet und eingeengt. Man erhält 140,7 g der Titelverbindung als gelbliches Öl, das ohne Reinigung weiter verwendet wird.

$^1$H-NMR($CDCl_3$): $\delta$ = 7,5–6,7 (m,4H); 5,8 (s,1H); 3,75 (s,3H); 3,67 (s,3H) ppm.

c) 2,3-Dihydro-2-(2-methoxyphenyl)benzothiazin-3-on

50 g 2-Brom-2-(2-methoxyphenyl)-essigsäuremethylester werden in 200 ml trockenem Dimethylformamid gelöst. Unter Rühren und Kühlen werden 24 ml (0,1929 Mol) o-Aminothiophenol zugetropft. Nach Aufwärmen auf Raumtemperatur wird 24 Stunden nachgerührt. Das Lösungsmittel wird i.vac. entfernt, der Rückstand mit Isopropylether verrieben und abgesaugt. Man erhält 30,8 g (60%) der Titelverbindung als farblose Kristalle vom Schmelzpunkt 171 °C.

$^1$H-NMR($CDCl_3$): $\delta$ = 7,3–6,6 (m,8H); 4,92 (s,1H); 3,72 (s,3H) ppm.

| | | | |
|---|---|---|---|
| Ber. ($C_{15}H_{13}NO_2S$) | C 66,4 | H 4,8 | N 5,2 |
| Gef. | C 66,6 | H 4,8 | N 5,0 |

d) 2,3-Dihydro-2,4-dimethyl-2-(2-methoxyphenyl)-benzothiazin-3-on

4,8 g Natriumhydrid (50% in Öl) werden 3× mit Hexan gewaschen, um das Öl zu entfernen, und anschliessend im $N_2$-Strom getrocknet. Nach Suspendieren in 40 ml trockenem Dimethylformamid werden 10 g (36,9 mmol) 2,3-Dihydro-2-(2-methoxyphenyl)-benzothiazin-3-on in 10 ml trockenem DMF zugetropft. Nach 30 min Rühren werden unter Kühlung 6,8 ml Methyljodid zugegeben und noch 2,5 Stunden gerührt. Die Mischung wird auf Eis gegossen und mit $CH_2Cl_2$ extrahiert. Die organische Phase wird mit $Na_2SO_4$ getrocknet und eingeengt. Das Rohprodukt wird an Kieselgel mit Essigester/Cyclohexan (1 : 3) chromatographiert. Man erhält 10,1 g der Titelverbindung als farblose Kristalle vom Schmelzpunkt 78 °C.

$^1$H-NMR($CDCl_3$): $\delta$ = 7,5–6,5 (m,8H); 3,77 (s,3H); 3,15 (s,3H); 1,83 (s,3H) ppm.

| | | | |
|---|---|---|---|
| Ber. ($C_{17}H_{17}NO_2S$) | C 68,2 | H 5,7 | N 4,6 |
| Gef. | C 68,5 | H 5,7 | N 4,6 |

e) 2,3-Dihydro-2,4-dimethyl-2-(2-hydroxyphenyl)-benzothiazin-3-on

1,85 g (6,2 mmol) 2,3-Dihydro-2,4-dimethyl-2-(2-methoxyphenyl)-benzothiazin-3-on werden in 20 ml absolutem Methylenchlorid bei 0 Grad Celsius mit 5,2 ml Bortribromid-Lösung (1 M in Hexan) versetzt. Nach 2 Stunden bei Raumtemperatur wird auf Wasser gegossen und der ausgefallene farblose Feststoff mit Essigester extrahiert. Nach Trocknen mit $MgSO_4$ wird eingeengt und mit Isopropylether verrieben. Man erhält 1,45 g farblose Kristalle vom Schmelzpunkt 164 bis 167 °C.

$^1$H-NMR($CDCl_3$): $\delta$ = 7,5–6,5 (m,9H); 3,53 (s,3H); 1,90 (s,3H) ppm.

| | | | |
|---|---|---|---|
| Ber. ($C_{16}H_{15}NO_2S$) | C 67,3 | H 5,3 | N 4,9 |
| Gef. | C 66,5 | H 5,4 | N 4,9 |

f) 2,3-Dihydro-2,4-dimethyl-2-[2-(4-chlorbutoxy)-phenyl]-benzothiazin-3-on

1,4 g (5 mmol) der Verbindung aus e) werden zusammen mit 0,58 ml (5 mmol) 1-Brom-4-chlorbutan und 0,97 g (7 mmol) Kaliumcarbonat in 30 ml Methylethylketon gelöst und 8 Stunden am Rückfluss erhitzt. Nach Abfiltrieren und Einengen erhält man 2,04 g der Titelverbindung als gelbes Öl, das ohne weitere Reinigung eingesetzt werden kann.

$^1$H-NMR($CDCl_3$): $\delta$ = 7,4–6,4 (m8H); 3,9 (t,3H); 3,6 (t,3H); 3,4 (s,3H); 2,2–1,8 (m,4H); 1,85 (s,3H) ppm.

g) 2,3-Dihydro-2,4-dimethyl-2-[2-[4-[N-[2-(3,4-dimethoxyphenyl)ethyl-N-methyl]-amino]-butoxy]-phenyl]-benzothiazin-3-on-Hydrochlorid

2,04 g der Verbindung aus f) und 1,37 g N-Methylhomoveratrylamin sowie 1,38 g Kaliumcarbonat werden in 30 ml Toluol 40 Stunden am Rückfluss gekocht. Nach Verdünnen mit Essigester wird mit gesättigter NaCl-Lösung gewaschen, mit $Na_2SO_4$ getrocknet und eingeengt. Das Rohprodukt wird an 230 g Kieselgel mit $CH_2Cl_2$/MeOH (15 : 1) chromatographiert. Man erhält nach Verreiben mit Essigester/Ether 1,2 g Produkt vom Schmelzpunkt 72–75 °C.

$^1$H-NMR($CDCl_3$): $\delta$ = 7,6–6,5 (m,11H); 3,93 (t,3H); 3,85 (s,6H); 3,43 (s,3H); 3,5–3,0 (m,6H); 2,87 (s,3H); 2,5–1,6 (m,4H); 1,75 (s,3H) ppm.

Zur Überführung in das HCl-Salz wird in Aceton aufgenommen und mit 2,5 N ethanolischer HCl versetzt. Nach Einengen und Verreiben mit Essigester erhält man 1,2 Gramm Kristalle vom Schmelzpunkt 147 bis 150 °C.

| | | | |
|---|---|---|---|
| Ber. ($C_{31}H_{39}ClN_2O_4S \cdot H_2O$) | C 63,2 | H 7,0 | N 4,8 |
| Gef. | C 63,4 | H 6,8 | N 4,7 |

Beispiel 2

2,3-Dihydro-2,4-dimethyl-2-[2-[3-[N-[2-(3,4-dimethoxyphenyl)-ethyl-N-methyl]-amino]-propoxy]-phenyl]-benzothiazin-3-on

a) 2,3-Dihydro-2,4-dimethyl-2-[2-(3-chlorpropoxy)-phenyl]-benzothiazin-3-on

3,35 g 2,3-Dihydro-2,4-dimethyl-2-(2-hydroxyphenyl)-benzothiazin-3-on werden zusammen mit 2,4 g (15,27 mmol) 1-Brom-3-chlorpropan und 3,25 g Kaliumcarbonat in 30 ml Methylethylketon gelöst und 15 Stunden am Rückfluss erhitzt. Der Niederschlag wird abgesaugt und das Filtrat eingeengt. Man erhält 4,8 g Rohprodukt, das beim Stehen kristallisiert, mit Hexan verrieben und abgesaugt wird; Schmelzpunkt 92 °C.

b) 2,3-Dihydro-2,4-dimethyl-2-[2-[3-[N-[2-(3,4-dimethoxyphenyl)-ethyl-N-methyl]-amino]-propoxy]-phenyl]-benzothiazin-3-on

1,45 g (4 mmol) der Verbindung aus Beispiel 2a) werden zusammen mit 1,37 g N-Methylhomoveratrylamin und 1,38 g Kaliumcarbonat in 30 ml Toluol gegeben und 40 Stunden am Rückfluss erhitzt. Nach Verdünnen mit Essigester wird mit Wasser gewaschen, noch 1 × mit Essigester extrahiert, mit gesättigter Natriumchloridlösung gewaschen, über Natriumsulfat getrocknet und eingeengt. Nach Chromatographie an Kieselgel mit Methylenchlorid/Methanol (13 : 1) als Laufmittel erhält man 1,16 g (56%) der Titelverbindung als Öl.

$^1$H-NMR($CDCl_3$): δ = 7,5–6,5 (m,11H); 3,97 (t,3H); 3,85 (s,6H); 3,47 (s,3H); 2,75 (mc,6H); 2,4 (s,3H); 2,1–1,8 (m,2H); 1,83 (s,3H) ppm.

Beispiel 3

2,3-Dihydro-2,4-dimethyl-2-[2-[4-[4-[2-(3,4,5-trimethoxyphenyl)-ethyl]-piperazinyl]-butoxy]-phenyl]-benzothiazin-3-on-Bis-Maleinat

1,5 g (4 mmol) der Verbindung aus Beispiel 1f) werden zusammen mit 1,12 g (4 mmol) 3,4,5-Trimethoxyphenylethyl-piperazin 4 Stunden auf 120 °C erhitzt. Das erhaltene Öl wird in Essigester/ 1 N NaOH aufgenommen, noch 2 × mit Essigester extrahiert, mit Natriumsulfat getrocknet, eingeengt und das Rohprodukt an Kieselgel mit Methylenchlorid/Methanol (12 : 1) als Laufmittel chromatographiert. Das erhaltene Öl (0,6 g) kristallisiert beim Stehen aus Essigester/Ether, Schmelzpunkt 176–179 °C.

$^1$H-NMR($CDCl_3$): δ = 7,6–6,5 (m,8H); 6,44 (s,2H); 4,1–3,9 (m,2H); 3,83 + 3,80 (2s,9H); 3,47 (s,3H); 3,4–2,7 (m,14H); 2,5–1,7 (m,4H); 1,77 (s,3H) ppm.

Die freie Base wird in Methylenchlorid aufgenommen, mit 120 mg Maleinsäure in 10 ml Aceton versetzt, die Lösung eingeengt, in Aceton aufgenommen und abgesaugt, Schmelzpunkt 158–160 °C.

Beispiel 4

2,3-Dihydro-2,4-dimethyl-2-[2-[3-[4-[2-(3,4,5-trimethoxyphenyl)-ethyl]-piperazinyl]-propoxy]-phenyl]-benzothiazin-3-on-Bis-Maleinat

1,45 g (4 mmol) der Verbindung aus Beispiel 2a) werden zusammen mit 1,12 g (4 mmol) 3,4,5-Trimethoxyphenylethyl-piperazin 4 Stunden auf 120 °C erhitzt. Nach Aufarbeitung analog Beispiel 3 erhält man 0,5 g Öl.

$^1$H-NMR($CDCl_3$): δ = 7,6–6,5 (m,8H); 6,43 (s,2H); 4,1–3,9 (m,2H); 3,83 + 3,80 (2s,9H); 3,47 (s,3H); 3,2–2,5 (m,14H); 2,4–2,0 (m,2H); 1,80 (s,3H) ppm.

Nach Aufnehmen in $CH_2Cl_2$ wird mit 193 mg Maleinsäure in 10 ml Aceton versetzt, 2 × mit Aceton eingeengt und in Aceton/Diethylether aufgenommen, schliesslich abgesaugt, farblose Kristalle vom Schmelzpunkt 157–159 °C.

| | | | |
|---|---|---|---|
| Ber. ($C_{47}H_{53}N_3O_{13}S$) | C 62,7 | H 5,9 | N 4,7 |
| Gef. | C 62,6 | H 5,9 | N 4,9 |

Beispiel 5

2,3-Dihydro-2,4-dimethyl-2-[3-[3-[N-[3-(3,4-dimethoxyphenyl)-1-methyl-propyl-N-methyl]-amino]-propoxy]-phenyl]-benzothiazin-3-on

1,6 g der Verbindung aus Beispiel 2a) werden zusammen mit 0,99 g N,1-Dimethyl-3,4-dimethoxyphenyl-propylamin und 0,6 ml N-Ethylmorpholin in 20 ml DMF 15 Stunden auf 100 °C erhitzt, mit Wasser und Methylenchlorid verdünnt und mit Methylenchlorid 2 × extrahiert. Die vereinigten organischen Phasen werden mit Wasser 5 × gewaschen, mit Natriumsulfat getrocknet und eingeengt. Chromatographie an Kieselgel mit Methylenchlorid/ Methanol (10 : 1) liefert 0,57 g der Titelverbindung als gelbliches Harz.

$^1$H-NMR($CDCl_3$): δ = 7,5–6,5 (m,11H); 3,99 (t,3H); 3,83 (s,6H); 3,43 (s,3H); 3,1–2,0 (m,5H); 2,4 (s,3H); 1,80 (s,3H); 1,10 + 1,08 (2d,3H) ppm.

Beispiel 6

2,3-Dihydro-2,4-dimethyl-2-[2-[4-[N-[2-(3,4-dimethoxyphenyl)-ethyl-N-methyl]-amino]-butoxy]-phenyl]-benzothiazin-3-on-Hydrochlorid

1,4 g (5 mmol) der Verbindung aus Beispiel 1e) werden zusammen mit 1,42 g (5 mmol) 4-Chlor-N-[2-(3,4-dimethoxyphenyl)-ethyl-N-methyl]-butylamin und 0,97 g (7 mmol) Kaliumcarbonat in 20 ml Isopropanol 18 Stunden am Rückfluss erhitzt. Nach üblicher Aufarbeitung wird wie in Beispiel 1g) weiterverfahren. Das erhaltene Produkt stimmt in seinen analytischen Daten mit der Verbindung aus Beispiel 1g) überein.

Beispiel 7

2,3-Dihydro-2,4-dimethyl-2-[2-[4-[4-[2-(3,4,5-trimethoxyphenyl)-ethyl]-piperazinyl]-butoxy]-phenyl]-benzothiazin-3-on-Bis-Maleinat

1,4 g (5 mmol) der Verbindung aus Beispiel 1e) werden zusammen mit 1,85 g (5 mmol) $N_1$-[2-(3,4,5-Trimethoxyphenyl)-ethyl]-$N_2$-4-chlor-1-butyl-piperazin und 0,97 g (7 mmol) Kaliumcarbonat 14 Stunden am Rückfluss erhitzt. Nach üblicher Aufarbeitung und Chromatographie, wie in Beispiel 3 beschrieben, erhält man die Titelverbindung, deren physikalische Daten mit denen von Beispiel 3 übereinstimmen.

Unter Verwendung geeigneter Ausgangsstoffe und Reagenzien erhält man die im folgenden tabellarisch aufgeführten Verbindungen unter Anwendung der in den Beispielen 1 bis 7 beschriebenen Verfahrensweisen.

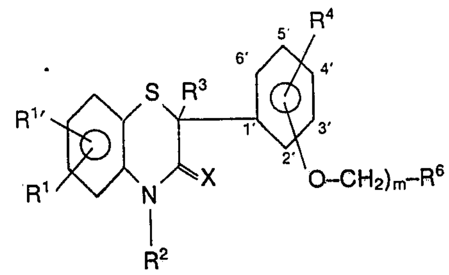

| Bsp.-Nr. | $R^1$ | $R^{1'}$ | $R^2$ | $R^3$ | $R^4$ | $R^6$ | m | X | Seitenkette in Position | NMR (δ, ppm) |
|---|---|---|---|---|---|---|---|---|---|---|
| 8 | H | H | $CH_3$ | $CH_3$ | H | 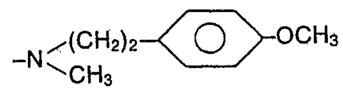 | 3 | O | 2′ | 7.5–6.5 (m, 12H); 3.95 (t, 2H); 3.87 (s, 3H); 3.47 (s, 3H); 2.9–2.5 (m, 6H); 2.4 (s, 3H); 2.1–1.8 (m, 2H); 1.86 (s, 3H). |
| 9 | H | H | $CH_3$ | $CH_3$ | H | 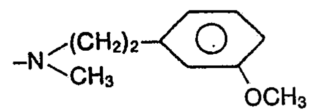 | 3 | O | 2′ | 7.6–6.4 (m, 12H); 3.92 (t, 2H); 3.82 (s, 3H); 3.47 (s, 3H); 2.9–2.5 (m, 6H); 2.42 (s, 3H); 2.1–1.7 (m, 2H); 1.84 (s, 3H). |
| 10 | H | H | $CH_3$ | $CH_3$ | H | 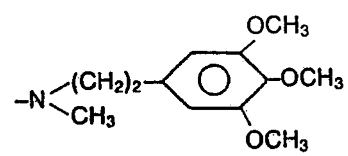 | 3 | O | 2′ | 7.4–6.6 (m, 10H); 3.87 + 3.81 (2s, 9H), 4.05–3.8 (m, 2H); 3.45 (s, 3H); 2.9–2.5 (m, 6H); 2.46 (s, 3H); 2.2–1.9 (m, 2H); 1.84 (s, 3H). |
| 11 | H | H | $CH_3$ | $CH_3$ | H | 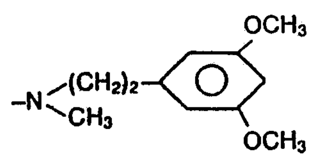 | 3 | O | 2′ | 7.5–6.6 (m, 11H); 3.95 (t, 2H); 3.84 (s, 6H); 3.47 (s, 3H); 2.9–2.5 (m, 6H); 2.43 (s, 3H); 2.1–1.8 (m, 2H); 1.83 (s, 3H). |
| 12 | H | H | $CH_3$ | $CH_3$ | H | 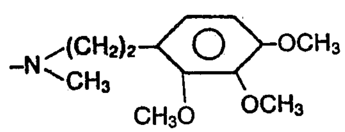 | 3 | O | 2′ | 7.4–6.5 (m, 10H); 3.92 (t, 2H); 3.9–3.8 (3s, 3H); 3.47 (s, 3H); 2.9–2.5 (m, 6H); 2.41 (s, 3H); 2.1–1.8 (m, 2H); 1.86 (s, 3H). |

| Bsp.-Nr. | $R^1$ | $R^{1'}$ | $R^2$ | $R^3$ | $R^4$ | $R^6$ | m | X | Seitenkette in Position | NMR (d, ppm) |
|---|---|---|---|---|---|---|---|---|---|---|
| 13 | H | H | $CH_3$ | $CH_3$ | H | 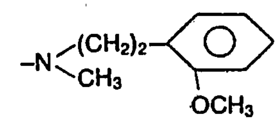 | 3 | O | 2′ | 7.6–6.5 (m, 12H); 3.93 (t, 2H); 3.84 (s, 3H); 3.47 (s, 3H); 2.95–2.5 (m, 6H); 2.42 (s, 3H); 2.1–1.8 (m, 2H); 1.85 (s, 3H). |
| 14 | H | H | $CH_3$ | $CH_3$ | H | 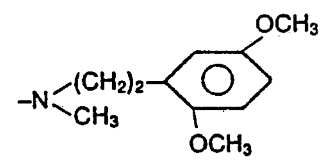 | 3 | O | 2′ | 7.5–6.6 (m, 11H); 3.95 (t, 2H); 3.84 (s, 6H); 3.44 (s, 3H); 2.9–2.5 (m, 6H); 2.47 (s, 3H); 2.1–1.8 (m, 2H); 1.82 (s, 3H). |
| 15 | H | H | $CH_3$ | $CH_3$ | H | 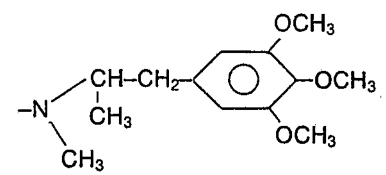 | 3 | O | 2′ | 7.5–6.4 (m, 10H); 3.93 (t, 2H); 3.87 (s, 9H); 3.47 (s, 3H); 2.9–2.5 (m, 5H); 2.47 (s, 3H); 2.1–1.8 (m, 2H); 1.84 (s, 3H); 1.15 (d, 3H). |
| 16 | H | H | $CH_3$ | $CH_3$ | H | 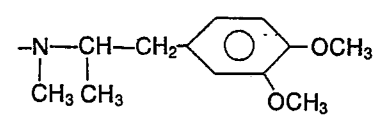 | 3 | O | 2′ | 7.5–6.5 (m, 11H); 3.93 (t, 2H); 3.84 (s, 6H); 3.47 (s, 3H); 2.9–2.5 (m, 5H); 2.43 (s, 3H); 2.1–1.8 (m, 2H); 1.87 (s, 3H); 1.14 (d, 3H). |
| 17 | H | H | $CH_3$ | $CH_3$ | H | 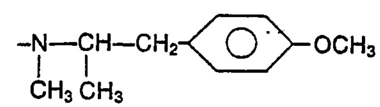 | 3 | O | 2′ | 7.6–6.5 (m, 12H); 3.97 (t, 2H); 3.87 (s, 3H); 3.44 (s, 3H); 2.9–2.5 (m, 5H); 2.47 (s, 3H); 2.1–1.8 (m, 2H); 1.81 (s, 3H); 1.19 (d, 3H). |
| 18 | H | H | $CH_3$ | $CH_3$ | H | 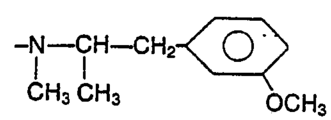 | 3 | O | 2′ | 7.6–6.5 (m, 12H); 3.94 (t, 2H); 3.84 (s, 3H); 3.47 (s, 3H); 2.9–2.5 (m, 5H); 2.41 (s, 3H); 2.1–1.8 (m, 2H); 1.84 (s, 3H); 1.21 (d, 3H). |
| 19 | H | H | $CH_3$ | $CH_3$ | H | 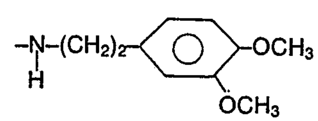 | 3 | O | 2′ | 7.5–6.5 (m, 11H); 3.92 (t, 2H); 3.87 (s, 6H); 3.47 (s, 3H); 2.9–2.5 (m, 6H); 2.1–1.8 (m, 2H); 1.83 (s, 3H). |

| | | | | | | | | | | |
|---|---|---|---|---|---|---|---|---|---|---|
| 20 | H | H | $CH_3$ | $CH_3$ | H | 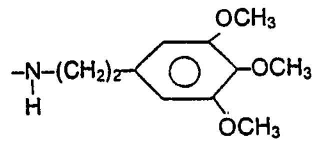 | 3 | O | 2′ | 7.4–6.4 (m, 10H); 3.9 (t, 2H); 3.83 (s, 9H); 3.47 (s, 3H); 2.9–2.5 (m, 6H); 2.1–1.8 (m, 2H); 1.84 (s, 3H). |
| 21 | H | H | $CH_3$ | $CH_3$ | H | 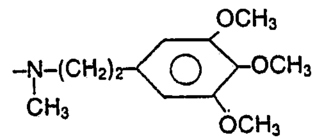 | 4 | O | 2′ | 7.4–6.4 (m, 10H); 3.9 (t, 2H); 3.87+3.81 (2s, 9H); 3.45 (s, 3H); 2.9–2.5 (m, 6H); 2.47 (s, 3H); 2.3–1.8 (m, 4H); 1.84 (s, 3H). |
| 22 | H | H | $CH_3$ | $CH_3$ | H | 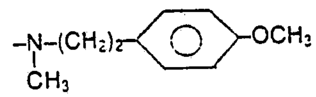 | 4 | O | 2′ | 7.5–6.5 (m, 12H); 3.95 (t, 2H); 3.87 (s, 3H); 3.47 (s, 3H); 2.9–2.5 (m, 6H); 2.4 (s, 3H); 2.3–1.8 (m, 4H); 1.86 (s, 3H). |
| 23 | H | H | $CH_3$ | $CH_3$ | H | 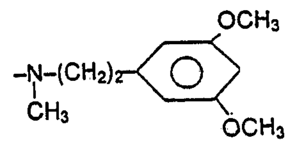 | 4 | O | 2′ | 7.5–6.6 (m, 11H); 3.95 (t, 2H); 3.84 (s, 6H); 3.47 (s, 3H); 2.9–2.5 (m, 6H); 2.43 (s, 3H); 2.3–1.7 (m, 4H); 1.83 (s, 3H). |
| 24 | H | H | $CH_3$ | $CH_3$ | H | 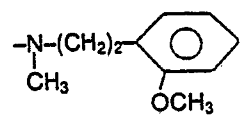 | 4 | O | 2′ | 7.5–6.5 (m, 12H); 3.95 (t, 2H); 3.82 (s, 3H); 3.47 (s, 3H); 2.9–2.5 (m, 6H); 2.41 (s, 3H); 2.3–1.7 (m, 4H); 1.84 (s, 3H). |
| 25 | H | H | $CH_3$ | $CH_3$ | H | 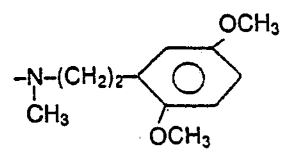 | 4 | O | 2′ | 7.5–6.5 (m, 11H); 3.94 (t, 2H); 3.82 (s, 6H); 3.46 (s, 3H); 2.9–2.5 (m, 6H); 2.40 (s, 3H); 2.3–1.7 (m, 4H); 1.86 (s, 3H). |
| 26 | H | H | $CH_3$ | $CH_3$ | H | 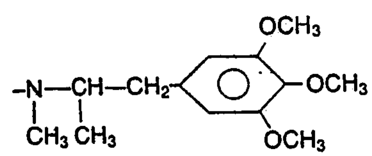 | 4 | O | 2′ | 7.5–6.4 (m, 10H); 3.93 (t, 2H); 3.89+3.83 (2s, 9H); 3.47 (s, 3H); 2.9–2.5 (m, 5H); 2.46 (s, 3H); 2.1–1.7 (m, 4H); 1.84 (s, 3H); 1.05 (d, 3H). |
| 27 | H | H | $CH_3$ | $CH_3$ | H | 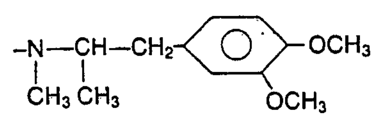 | 4 | O | 2′ | 7.5–6.5 (m, 11H); 3.93 (t, 2H); 3.84 (s, 6H); 3.47 (s, 3H); 2.9–2.5 (m, 5H); 2.44 (s, 3H); 2.3–1.7 (m, 4H); 1.86 (s, 3H); 1.07 (d, 3H). |

| Bsp.-Nr. | $R^1$ | $R^{1'}$ | $R^2$ | $R^3$ | $R^4$ | $R^6$ | m | X | Seitenkette in Position | NMR (δ, ppm) |
|---|---|---|---|---|---|---|---|---|---|---|
| 28 | H | H | $CH_3$ | $CH_3$ | H | 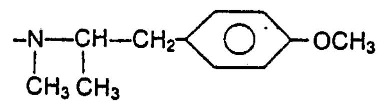 | 4 | O | 2′ | 7.5–6.5 (m, 12H); 3.90 (t, 2H); 3.86 (s, 3H); 3.46 (s, 3H); 2.9–2.5 (m, 5H); 2.44 (s, 3H); 2.3–1.7 (m, 4H); 1.84 (s, 3H); 1.04 (d, 3H). |
| 29 | H | H | $CH_3$ | $CH_3$ | H | 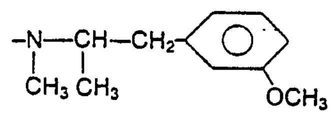 | 4 | O | 2′ | 7.5–6.4 (m, 12H); 3.94 (t, 2H); 3.81 (s, 3H); 3.47 (s, 3H); 2.9–2.5 (m, 5H); 2.46 (s, 3H); 2.3–1.7 (m, 4H); 1.86 (s, 3H); 1.08 (d, 3H). |
| 30 | H | H | $CH_3$ | $CH_3$ | H | 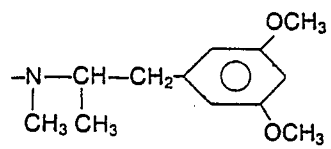 | 4 | O | 2′ | 7.5–6.5 (m, 11H); 3.94 (t, 2H); 3.84 (s, 6H); 3.47 (s, 3H); 2.9–2.5 (m, 5H); 2.40 (s, 3H); 2.3–1.7 (m, 4H); 1.84 (s, 3H); 1.06 (d, 3H). |
| 31 | H | H | $CH_3$ | $CH_3$ | H | 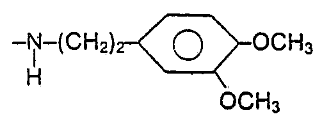 | 4 | O | 2′ | 7.5–6.5 (m, 11H); 3.92 (t, 2H); 3.87 (s, 6H); 3.47 (s, 3H); 2.9–2.5 (m, 6H); 2.5–1.8 (m, 4H); 1.83 (s, 3H). |
| 32 | H | H | $CH_3$ | $CH_3$ | H | 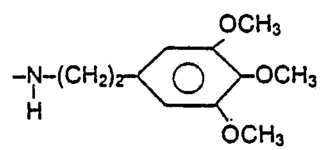 | 4 | O | 2′ | 7.5–6.4 (m, 10H); 3.94 (t, 2H); 3.88+3.82 (2s, 9H); 3.47 (s, 3H); 2.9–2.5 (m, 6H); 2.5–1.8 (m, 4H); 1.83 (s, 3H). |
| 33 | H | H | $CH_3$ | $CH_3$ | H | 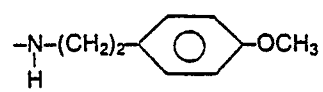 | 4 | O | 2′ | 7.5–6.5 (m, 12H); 3.92 (t, 2H); 3.84 (s, 3H); 3.44 (s, 3H); 3.2–2.8 (m, 6H); 2.5–1.8 (m, 4H); 1.83 (s, 3H). |
| 34 | H | H | $CH_3$ | $CH_3$ | H | 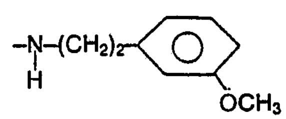 | 4 | O | 2′ | 7.5–6.4 (m, 12H); 3.94 (t, 2H); 3.86 (s, 3H); 3.48 (s, 3H); 3.2–2.7 (m, 6H); 2.4–1.7 (m, 4H); 1.79 (s, 3H). |

| | | | | | | | | | | |
|---|---|---|---|---|---|---|---|---|---|---|
| 35 | H | H | $CH_3$ | $CH_3$ | H | 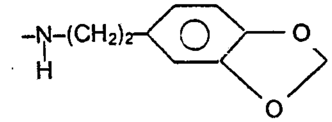 | 4 | O | 2′ | 7.5–6.4 (m, 11H); 5.05 (s, 2H); 3.90 (t, 2H); 3.48 (s, 3H); 3.1–2.7 (m, 6H); 2.4–1.8 (m, 4H); 1.80 (s, 3H). |
| 36 | H | H | $CH_3$ | $CH_3$ | H | 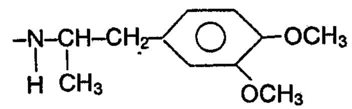 | 4 | O | 2′ | 7.5–6.5 (m, 11H); 3.96 (t, 2H); 3.84 (s, 6H); 3.52 (s, 3H); 3.4–3.0 (m, 6H); 2.4–1.8 (m, 4H); 1.84 (s, 3H); 1.12 (d, 3H). |
| 37 | H | H | $CH_3$ | $CH_3$ | H | 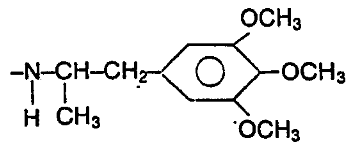 | 4 | O | 2′ | 7.5–6.3 (m, 10H); 3.92 (t, 2H); 3.83+3.80 (2s, 9H); 3.50 (s, 3H); 3.4–2.9 (m, 6H); 2.4–1.8 (m, 4H); 1.82 (s, 3H); 1.05 (d, 3H). |
| 38 | H | H | $CH_3$ | $CH_3$ | H | 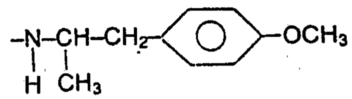 | 4 | O | 2′ | 7.5–6.6 (m, 12H); 3.94 (t, 2H); 3.84 (s, 3H); 3.51 (s, 3H); 3.4–2.8 (m, 6H); 2.4–1.8 (m, 4H); 1.82 (s, 3H); 1.08 (d, 3H). |
| 39 | H | H | $CH_3$ | $CH_3$ | H | 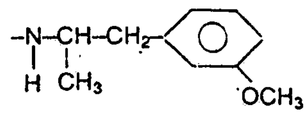 | 4 | O | 2′ | 7.5–6.6 (m, 12H); 3.91 (t, 2H); 3.84 (s, 3H); 3.47 (s, 3H); 4.3–2.7 (m, 6H); 2.4–1.8 (m, 4H); 1.84 (s, 3H); 1.04 (d, 3H). |
| 40 | H | H | $CH_3$ | $CH_3$ | H | 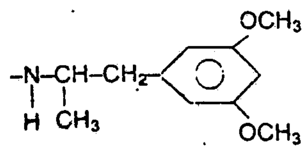 | 4 | O | 2′ | 7.5–6.5 (m, 11H); 3.94 (t, 2H); 3.82 (s, 6H); 3.46 (s, 3H); 3.4–2.9 (m, 6H); 2.3–1.7 (m, 4H); 1.79 (s, 3H); 1.14 (d, JH). |
| 41 | H | H | $CH_3$ | $CH_3$ | H | 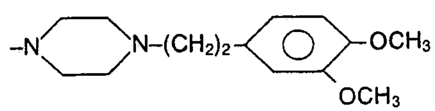 | 3 | O | 2′ | 7.6–6.5 (m, 11H); 4.1–3.9 (m, 2H); 3.84 (s, 6H); 3.47 (s, 3H); 3.2–2.5 (m, 14H); 2.4–2.0 (m, 2H); 1.81 (s, 3H). |
| 42 | H | H | $CH_3$ | $CH_3$ | H | 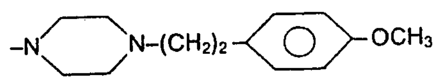 | 3 | O | 2′ | 7.6–6.5 (m, 12H); 4.1–3.9 (m, 2H); 3.86 (s, 3H); 3.42 (s, 3H); 3.2–2.5 (m, 14H); 2.4–2.0 (m, 2H); 1.79 (s, 3H). |

| Bsp.-Nr. | $R^1$ | $R^{1'}$ | $R^2$ | $R^3$ | $R^4$ | $R^6$ | m | X | Seitenkette in Position | NMR (δ, ppm) |
|---|---|---|---|---|---|---|---|---|---|---|
| 43 | H | H | $CH_3$ | $CH_3$ | H | 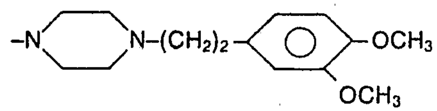 | 4 | O | 2′ | 7.4–6.5 (m, 11H); 4.1–3.8 (m, 2H); 3.83 (s, 6H); 3.47 (s, 3H); 3.4–2.6 (m, 14H); 2.5–1.7 (m, 4H); 1.78 (s, 3H). |
| 44 | H | H | $CH_3$ | $CH_3$ | H | 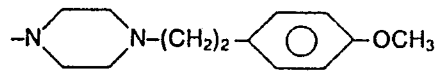 | 4 | O | 2′ | 7.6–6.5 (m, 12H); 4.1–3.9 (m, 2H); 3.81 (s, 3H); 3.46 (s, 3H); 3.1–2.1 (m, 14H); 2.0–1.5 (m, 4H); 1.80 (s, 3H). |
| 45 | H | H | $CH_3$ | $CH_3$ | H | 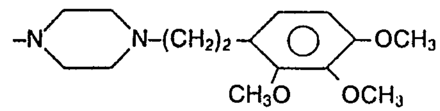 | 4 | O | 2′ | 7.6–6.4 (m, 10H); 4.1–3.9 (m, 2H); 3.80 (s, 9H); 3.47 (s, 3H); 3.4–2.6 (m, 14H); 2.4–1.7 (m, 4H); 1.76 (s, 3H). |
| 46 | H | H | $CH_3$ | $CH_3$ | H | 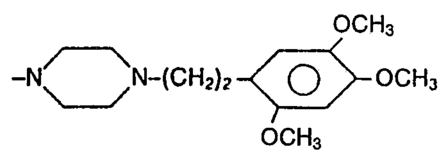 | 4 | O | 2′ | 7.5–6.4 (m, 10H); 4.1–3.9 (m, 2H); 3.82 (s, 9H); 3.45 (s, 3H); 3.3–2.6 (m, 14H); 2.4–1.7 (m, 4H); 1.80 (s, 3H). |
| 47 | H | H | $CH_3$ | $CH_3$ | H | 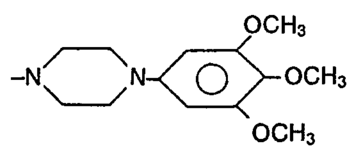 | 4 | O | 2′ | 7.5–6.2 (m, 10H); 4.1–3.9 (m, 2H); 3.86+3.81 (2s, 9H); 3.47 (s, 3H); 3.4–2.5 (m, 10H); 2.4–1.7 (m, 4H); 1.78 (s, 3H). |
| 48 | H | H | $CH_3$ | $CH_3$ | H | 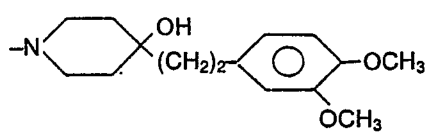 | 4 | O | 2′ | 7.5–6.5 (m, 11H); 4.1–3.9 (m, 2H); 3.83 (s, 6H); 3.47 (s, 3H); 3.4–2.9 (m, 8H); 2.4–1.5 (m, 10H); 1.80 (s, 3H). |
| 49 | H | H | $CH_3$ | $CH_3$ | H | 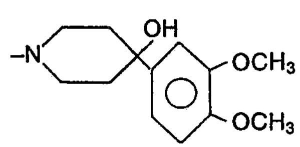 | 4 | O | 2′ | 7.5–6.5 (m, 11H); 4.1–3.9 (m, 2H); 3.84 (s, 6H); 3.45 (s, 3H); 3.4–2.7 (m, 6H); 2.4–1.5 (m, 4H); 1.77 (s, 3H); |

| | | | | | | | | | | |
|---|---|---|---|---|---|---|---|---|---|---|
| 50 | H | H | $CH_3$ | $CH_3$ | H | 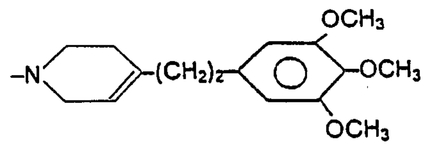 | 4 | O | 2′ | 7.5–6.4 (m, 10H); 5.7 (m, 1H); 4.1–3.9 (m, 2H); 3.85+3.80 (2s, 9H); 3.45 (s, 3H); 3.4–2.6 (m, 12H); 2.4–1.5 (m, 4H); 1.79 (s, 3H); |
| 51 | H | H | $CH_3$ | $CH_3$ | H | 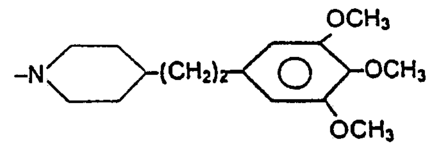 | 4 | O | 2′ | 7.5–6.4 (m, 10H); 4.1–3.9 (m, 2H); 3.86+3.80 (2s, 9H); 3.47 (s, 3H); 3.4–2.6 (m, 8H); 2.4–1.5 (m, 11H); 1.81 (s, 3H); |
| 52 | H | H | $CH_3$ | $CH_3$ | H | 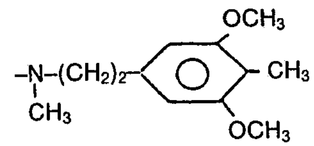 | 4 | O | 2′ | 7.6–6.5 (m, 10H); 4.1–3.9 (m, 2H); 3.84 (s, 6H); 3.47 (s, 3H); 2.9–2.5 (m, 6H); 2.4 (s, 3H); 2.3 (s, 3H); 2.4–1.8 (m, 4H); 1.84 (s, 3H). |
| 53 | H | H | $CH_3$ | $CH_3$ | H | 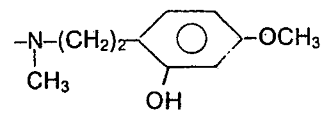 | 4 | O | 2′ | 7.6–6.5 (m, 11H); 4.1–3.9 (m, 2H); 3.82 (s, 3H); 3.46 (s, 3H); 2.9–2.5 (m, 6H); 2.4 (s, 3H); 2.4–1.8 (m, 4H); 1.79 (s, 3H). |
| 54 | H | H | $CH_3$ | $CH_3$ | H | 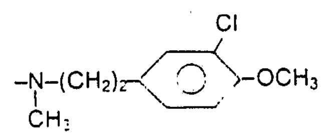 | 4 | O | 2′ | 7.6–6.6 (m, 11H); 4.1–3.9 (m, 2H); 3.86 (s, 3H); 3.51 (s, 3H); 3.2–2.5 (m, 6H); 2.4 (s, 3H); 2.3–1.7 (m, 4H); 1.81 (s, 3H). |
| 55 | H | H | $CH_3$ | $CH_3$ | H | 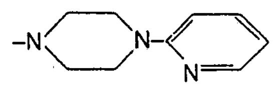 | 4 | O | 2′ | 8.4–6.9 (m, 12H); 4.1–3.9 (m, 2H); 3.50 (s, 3H); 3.5–2.5 (m, 10H); 2.3–1.6 (m, 4H); 1.80 (s, 3H). |
| 56 | H | H | $CH_3$ | $CH_3$ | H | 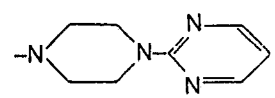 | 4 | O | 2′ | 8.3–7.1 (m, 11H); 4.1–3.9 (m, 2H); 3.46 (s, 3H); 3.5–2.5 (m, 10H); 2.3–1.6 (m, 4H); 1.78 (s, 3H). |
| 57 | H | H | $CH_3$ | $CH_3$ | H | 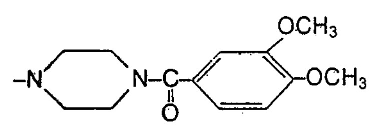 | 4 | O | 2′ | 7.6–6.8 (m, 11H); 4.1–3.7 (m, 6H); 3.51 (s, 3H); 3.5–2.5 (m, 6H); 2.3–1.6 (m, 4H); 1.82 (s, 3H). |

| Bsp.-Nr. | $R^1$ | $R^{1'}$ | $R^2$ | $R^3$ | $R^4$ | $R^6$ | m | X | Seitenkette in Position | NMR (d, ppm) |
|---|---|---|---|---|---|---|---|---|---|---|
| 58 | H | H | $CH_3$ | $CH_3$ | H | 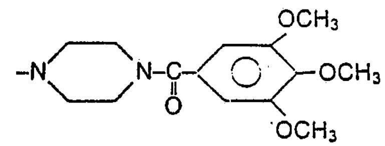 | 4 | O | 2′ | 7.5–6.6 (m, 10H); 4.1–3.7 (m, 6H); 3.51 (s, 3H); 3.4–2.5 (m, 6H); 2.3–1.6 (m, 4H); 1.84 (s, 3H). |
| 59 | 6–Cl | H | $CH_3$ | $CH_3$ | H | 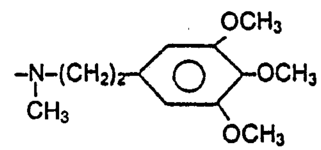 | 4 | O | 2′ | 7.7–6.4 (m, 9H); 4.1–3.9 (m, 2H); 3.47 (s, 3H); 3.84+3.80 (2s, 9H); 3.4–2.6 (m, 6H); 2.41 (s, 3H); 2.3–1.7 (m, 4H); 1.84 (s, 3H). |
| 60 | 6–Cl | H | $CH_3$ | $CH_3$ | H | 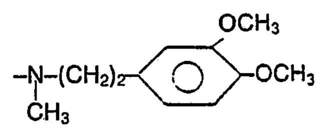 | 4 | O | 2′ | 7.6–6.5 (m, 10H); 4.1–3.9 (m, 2H); 3.83 (s, 6H); 3.47 (s, 3H); 3.4–2.6 (m, 6H); 2.42 (s, 3H); 2.3–1.7 (m, 4H); 1.79 (s, 3H). |
| 61 | 6–Cl | H | $CH_3$ | $CH_3$ | H | 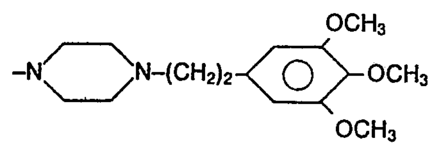 | 4 | O | 2′ | 7.4–6.4 (m, 9H); 4.1–3.9 (m, 2H); 3.86+3.81 (2s, 9H); 3.46 (s, 3H); 3.4–2.5 (m, 14H); 2.4–1.7 (m, 4H); 1.79 (s, 3H). |
| 62 | H | 7–Cl | $CH_3$ | $CH_3$ | H | 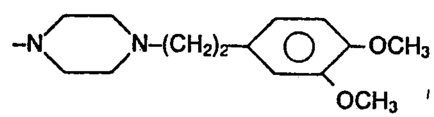 | 4 | O | 2′ | 7.4–6.4 (m, 10H); 4.1–3.9 (m, 2H); 3.87 (s, 6H); 3.47 (s, 3H); 3.4–2.5 (m, 14H); 2.4–1.7 (m, 4H); 1.82 (s, 3H). |
| 63 | H | 7–F | $CH_3$ | $CH_3$ | H | 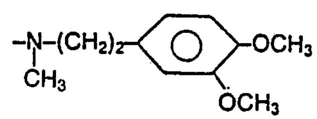 | 4 | O | 2′ | 7.4–6.5 (m, 10H); 4.1–3.9 (m, 2H); 3.86 (s, 6H); 3.47 (s, 3H); 3.4–2.6 (m, 6H); 2.4 (s, 3H); 2.3–1.7 (m, 4H); 1.81 (s, 3H). |

| | | | | | | | | | | |
|---|---|---|---|---|---|---|---|---|---|---|
| 64 | H | 7–F | $CH_3$ | $CH_3$ | H | 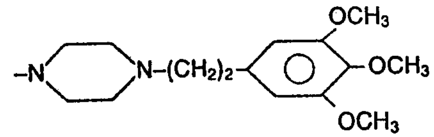 | 4 | O | 2′ | 7.4–6.4 (m, 9H); 4.1–3.9 (m, 2H); 3.86+3.81 (2s, 9H); 3.45 (s, 3H); 3.3–2.5 (m, 14H); 2.4–1.8 (m, 4H); 1.86 (s, 3H). |
| 65 | 6–F | H | $CH_3$ | $CH_3$ | H | 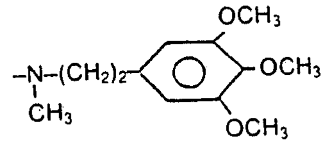 | 4 | O | 2′ | 7.4–6.4 (m, 9H); 4.1–3.9 (m, 2H); 3.87+3.81 (2s, 9H); 3.47 (s, 3H); 3.3–2.4 (m, 6H); 2.43 (s, 3H); 2.3–1.7 (m, 4H); 1.83 (s, 3H). |
| 66 | 6–F | H | $CH_3$ | $CH_3$ | H | 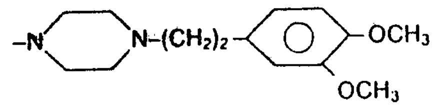 | 4 | O | 2′ | 7.4–6.4 (m, 10H); 4.1–3.9 (m, 2H); 3.82 (s, 6H); 3.44 (s, 3H); 3.4–2.5 (m, 14H); 2.3–1.7 (m, 4H); 1.79 (s, 3H). |
| 67 | 6–$OCH_3$ | H | $CH_3$ | $CH_3$ | H | 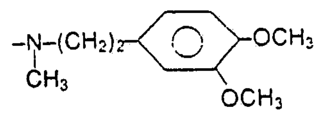 | 4 | O | 2′ | 7.4–6.4 (m, 10H); 4.1–3.9 (m, 2H); 3.84+3.81 (2s, 9H); 3.39 (s, 3H); 3.4–2.5 (m, 6H); 2.44 (s, 3H); 2.3–1.7 (m, 4H); 1.82 (s, 3H). |
| 68 | 6–$OCH_3$ | H | $CH_3$ | $CH_3$ | H | 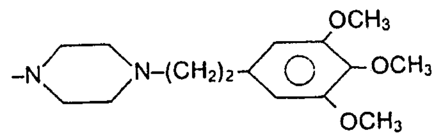 | 4 | O | 2′ | 7.4–6.4 (m, 9H); 4.1–3.9 (m, 2H); 3.9–3.8 (3s, 12H); 3.44 (s, 3H); 3.4–2.5 (m, 14H); 2.3–1.7 (m, 4H); 1.84 (s, 3H). |
| 69 | 6–$CH_3$ | H | $CH_3$ | $CH_3$ | H | 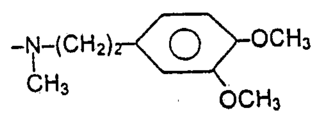 | 4 | O | 2′ | 7.5–6.5 (m, 10H); 4.1–3.9 (m, 2H); 3.82 (s, 6H); 3.51 (s, 3H); 3.4–2.5 (m, 6H); 2.44 (s, 3H); 2.28 (s, 3H); 2.3–1.7 (m, 4H); 1.81 (s, 3H). |
| 70 | 6–$CH_3$ | H | $CH_3$ | $CH_3$ | H | 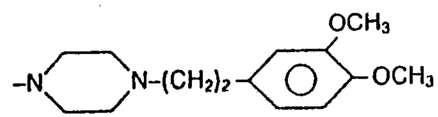 | 4 | O | 2′ | 7.5–6.5 (m, 10H); 4.1–3.9 (m, 2H); 3.84 (s, 6H); 3.50 (s, 3H); 3.4–2.5 (m, 14H); 2.29 (s, 3H); 2.3–1.7 (m, 4H); 1.81 (s, 3H). |
| 71 | 6–$CH_3$ | 7–$CH_3$ | $CH_3$ | $CH_3$ | H | 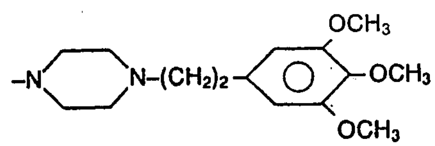 | 4 | O | 2′ | 7.4–6.4 (m, 8H); 4.1–3.9 (m, 2H); 3.87+3.82 (2s, 9H); 3.48 (s, 3H); 3.4–2.6 (m, 14H); 2.31 (s, 6H); 2.3–1.7 (m, 4H); 1.79 (s, 3H); |

| Bsp.-Nr. | $R^1$ | $R^{1'}$ | $R^2$ | $R^3$ | $R^4$ | $R^6$ | m | X | Seitenkette in Position | NMR (δ, ppm) |
|---|---|---|---|---|---|---|---|---|---|---|
| 72 | H | 7–$CH_3$ | $CH_3$ | $CH_3$ | H | 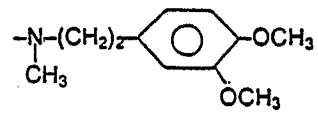 | 4 | O | 2′ | 7.4–6.5 (m, 10H); 4.1–3.9 (m, 2H); 3.81 (s, 6H); 3.51 (s, 3H); 3.4–2.6 (m, 6H); 2.45 (s, 3H); 2.28 (s, 3H); 2.3–1.7 (m, 4H); 1.78 (s, 3H); |
| 73 | H | H | $C_2H_5$ | $C_2H_5$ | H | 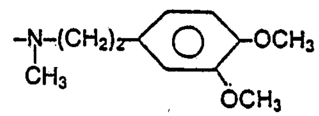 | 4 | O | 2′ | 7.5–6.5 (m, 11H); 4.1–3.7 (m, 4H); 3.83 (s, 6H); 3.4–2.5 (m, 6H); 2.46 (s, 3H); 2.3–1.7 (m, 6H); 1.3 (2t, 6H). |
| 74 | H | H | $C_2H_5$ | $CH_3$ | H | 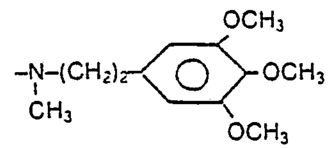 | 4 | O | 2′ | 7.5–6.4 (m, 10H); 4.1–3.7 (m, 4H); 3.87 + 3.81 (2s, 9H); 3.4–2.5 (m, 14H); 2.46 (s, 3H); 2.3–1.7 (m, 4H); 1.81 (s, 3H); 1.3 (t, 3H). |
| 75 | H | H | $CH_3$ | $C_2H_5$ | H | 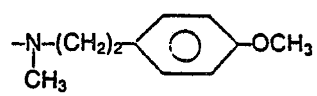 | 4 | O | 2′ | 7.5–6.5 (m, 12H); 4.1–3.9 (m, 2H); 3.81 (s, 3H); 3.51 (s, 3H); 3.4–2.5 (m, 6H); 2.41 (s, 3H); 2.3–1.7 (m, 6H); 1.3 (t, 3H). |
| 76 | H | H | n–$C_3C_7$ | $CH_3$ | H | 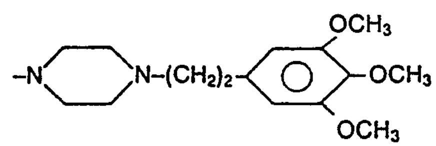 | 4 | O | 2′ | 7.4–6.4 (m, 10H); 4.1–3.7 (m, 4H); 3.86 + 3.81 (2s, 9H); 3.4–2.5 (m, 14H); 2.4–1.8 (m, 6H); 1.81 (s, 3H); 1.0 (t, 3H). |
| 77 | H | H | $C_6H_5$ | $CH_3$ | H | 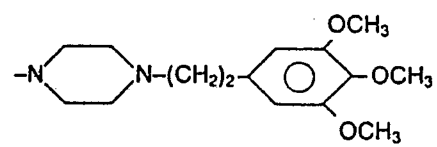 | 4 | O | 2′ | 7.4–6.4 (m, 15H); 4.2–3.9 (m, 2H); 3.87 + 3.81 (2s, 9H); 3.3–2.5 (m, 14H); 2.1–1.6 (m, 4H); 1.84 (s, 3H). |
| 78 | H | H | $C_6H_5$ | $CH_3$ | H | 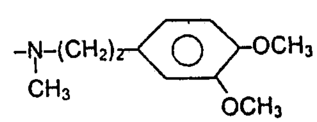 | 4 | O | 2′ | 7.4–6.5 (m, 16H); 4.2–3.9 (m, 2H); 3.82 (s, 6H); 3.3–2.5 (m, 6H); 2.4 (s, 3H); 2.1–1.6 (m, 4H); 1.85 (s, 3H). |

| | | | | | | | | | | |
|---|---|---|---|---|---|---|---|---|---|---|
| 79 | 6–$OCH_3$ | H | $CH_3$ | $C_2H_5$ | H | 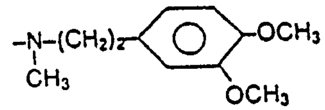 | 4 | O | 2′ | 7.4–6.5 (m, 10H); 4.2–3.9 (m, 2H); 3.81 (s, 9H); 3.51 (s, 3H); 3.4–2.5 (m, 6H); 2.4 (s, 3H); 2.2–1.6 (m, 6H); 1.0 (t, 3H). |
| 80 | 6–Cl | H | $C_6H_5$ | $CH_3$ | H | 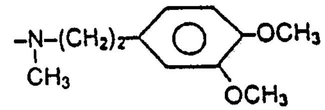 | 4 | O | 2′ | 7.4–6.5 (m, 15H); 4.2–3.9 (m, 2H); 3.82 (s, 6H); 3.4–2.5 (m, 6H); 2.4 (s, 3H); 2.2–1.6 (m, 4H); 1.81 (s, 3H). |
| 81 | 6–$CH_3$ | H | $C_2H_5$ | $CH_3$ | H | 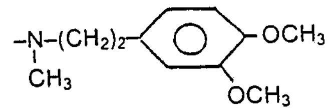 | 4 | O | 2′ | 7.4–6.5 (m, 10H); 4.2–3.7 (m, 4H); 3.84 (s, 6H); 3.4–2.5 (m, 6H); 2.4 (s, 3H); 2.35 (s, 3H); 2.3–1.7 (m, 4H); 1.79 (s, 3H); 1.0 (t, 3H). |
| 82 | H | H | $CH_3$ | $CH_3$ | 4′–$OCH_3$ | 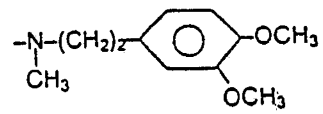 | 4 | O | 2′ | 7.4–6.5 (m, 10H); 4.1–3.9 (m, 2H); 3.87+3.83 (2s, 9H); 3.52 (s, 3H); 3.4–2.5 (m, 6H); 2.4 (s, 3H); 2.3–1.6 (m, 4H); 1.80 (s, 3H). |
| 83 | H | H | $CH_3$ | $CH_3$ | 6′–$OCH_3$ | 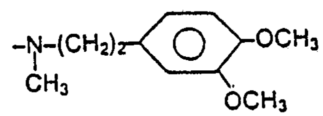 | 4 | O | 2′ | 7.4–6.5 (m, 10H); 4.1–3.9 (m, 2H); 3.86+3.81 (2s, 9H); 3.51 (s, 3H); 3.3–2.5 (m, 6H); 2.48 (s, 3H); 2.4–1.6 (m, 4H); 1.82 (s, 3H). |
| 84 | H | H | $CH_3$ | $CH_3$ | 5′–F | 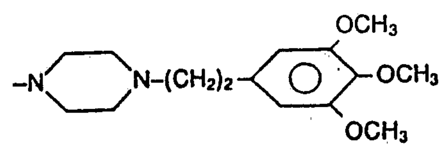 | 4 | O | 2′ | 7.4–6.4 (m, 9H); 4.1–3.9 (m, 2H); 3.86+3.82 (2s, 9H); 3.54 (s, 3H); 3.3–2.5 (m, 14H); 2.4–1.6 (m, 4H); 1.79 (s, 3H). |
| 85 | H | H | $CH_3$ | $CH_3$ | 5′–Cl | 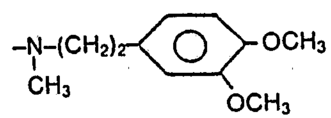 | 4 | O | 2′ | 7.4–6.5 (m, 10H); 4.1–3.9 (m, 2H); 3.82 (s, 6H); 3.53 (s, 3H); 3.3–2.6 (m, 6H); 2.4 (s, 3H); 2.3–1.6 (m, 4H); 1.82 (s, 3H). |
| 86 | H | H | $CH_3$ | $CH_3$ | 5′–$OCH_3$ | 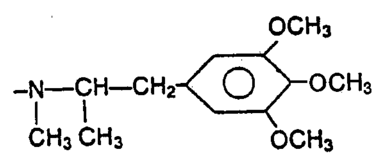 | 4 | O | 2′ | 7.4–6.5 (m, 9H); 4.1–3.9 (m, 2H); 3.9–3.8 (3s, 12H); 3.51 (s, 3H); 3.3–2.7 (m, 5H); 2.4 (s, 3H); 2.3–1.7 (m, 4H); 1.82 (s, 3H); 1.05 (d, 3H). |

| Bsp.-Nr. | $R^1$ | $R^{1'}$ | $R^2$ | $R^3$ | $R^4$ | $R^6$ | m | X | Seitenkette in Position | NMR (δ, ppm) |
|---|---|---|---|---|---|---|---|---|---|---|
| 87 | H | H | $CH_3$ | $CH_3$ | 4'–$NO_2$ | 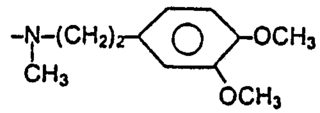 | 4 | O | 2' | 8.1–6.5 (m, 10H); 4.1–3.9 (m, 2H); 3.82 (s, 6H); 3.54 (s, 3H); 3.3–2.6 (m, 6H); 2.4 (s, 3H); 2.3–1.6 (m, 4H); 1.84 (s, 3H). |
| 88 | 6–F | H | $CH_3$ | $CH_3$ | 5'–$OCH_3$ | 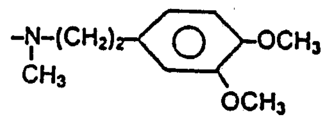 | 4 | O | 2' | 7.4–6.5 (m, 9H); 4.1–3.9 (m, 2H); 3.82 (s, 9H); 3.51 (s, 3H); 3.3–2.6 (m, 6H); 2.4 (s, 3H); 2.3–1.6 (m, 4H); 1.78 (s, 3H). |
| 89 | H | H | 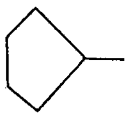 | $CH_3$ | H | 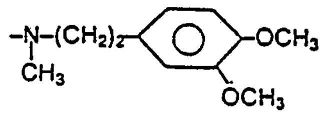 | 4 | O | 2' | 7.4–6.5 (m, 11H); 4.4–3.8 (m, 3H); 3.81 (s, 6H); 3.3–2.4 (m, 7H); 2.4 (s, 3H); 2.3–1.5 (m, 12H); 1.81 (s, 3H). |
| 90 | H | H | $CH_3$ | $CH_2=CHCH_2$ | H | 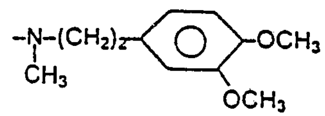 | 4 | O | 2' | 7.5–6.4 (m, 11H); 5.9–5.0 (m, 3H); 4.1–3.9 (m, 2H); 3.82 (s, 6H); 3.51 (s, 3H); 3.3–2.5 (m, 8H); 2.4 (s, 3H). |
| 91 | H | H | $CH_3$ | 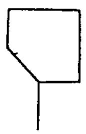 | H | 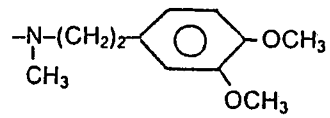 | 4 | O | 2' | 7.5–6.4 (m, 11H); 4.1–3.9 (m, 2H); 3.83 (s, 6H); 3.48 (s, 3H); 3.3–2.5 (m, 7H); 2.4 (s, 3H); 2.3–1.7 (m, 12H). |
| 92 | H | H | $CH_3$ | $C_6H_5CH_2$ | H | 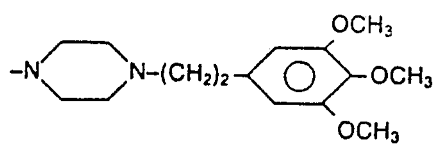 | 4 | O | 2' | 7.5–6.4 (m, 15H); 4.1–3.9 (m, 2H); 3.86+3.81 (2s, 9H); 3.47 (s, 3H); 3.3–2.5 (m, 16H); 2.3–1.7 (m, 4H). |
| 93 | H | H | $CH_3$ | 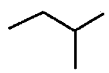 | H | 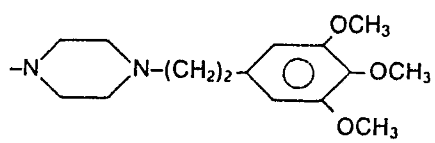 | 4 | O | 2' | 7.5–6.4 (m, 10H); 4.1–3.8 (m, 2H); 3.87+3.82 (2s, 9H); 3.5 (s, 3H); 3.3–2.5 (m, 15H); 2.3–1.7 (m, 4H); 1.1 (d+t, 6H). |
| 94 | H | H | $CH_3$ | $C_6H_5CH_2CH_2$ | H | 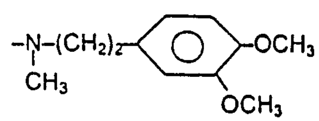 | 4 | O | 2' | 7.4–6.5 (m, 16H); 4.1–3.9 (m, 2H); 3.82 (s, 6H); 3.50 (s, 3H); 3.3–2.4 (m, 8H); 2.35 (s, 3H); 2.2–1.6 (m, 6H). |

Beispiel 95
(−)-2,3-Dihydro-2,4-dimethyl-2-[2-[4-[4-[2-(3,4,5-trimethoxyphenyl)-ethyl]-piperazinyl]-butoxy]-phenyl]-benzothiazin-3-on.Dihydrochlorid

a) 2,3-Dihydro-2,4-dimethyl-2-(3-ethoxycarbonyl)-propoxyphenyl-benzothiazin-3-on

4,4 g (90,5 mmol) Natriumhydrid werden in 200 ml absolutem DMF suspendiert. Nach Zugabe von 17,2 g (60,3 mmol) der Verbindung aus Beispiel 1e) wird 30 min gerührt, dann werden bei 0 °C 10,3 ml (66,4 mmol) 4-Brombuttersäureethylester zugesetzt. Nach 7 Std. Rühren bei Raumtemperatur wird auf Wasser gegossen und mit Methylenchlorid extrahiert. Die organische Phase wird viermal mit Wasser gewaschen, mit Natriumsulfat getrocknet und eingeengt. Chromatographie an 1 kg Kieselgel mit Essigester/Cyclohexan (1 : 3) als Laufmittel ergibt 19,4 g farbloses Öl.

$^1$H-NMR ($CDCl_3$): δ = 1,15 (t,3H); 1,75 (s,3H), 1,9–3,0 (m,4H); 3,35 (s,3H); 3,9 (q,2H); 4,1 (t,2H); 6,4–7,4 (m,8H) ppm.

b) 2,3-Dihydro-2,4-dimethyl-2-(3-carboxy-propoxy)-phenyl-benzothiazin-3-on

19,4 g (48 mmol) der Verbindung aus Beispiel 95a) werden mit 36,3 ml 2 N NaOH versetzt und bis zur klaren Lösung mit Ethanol verdünnt. Die Lösung wird 2,5 Std. bei Raumtemperatur gerührt, das Ethanol abgedampft und mit 2 N HCl sauer gestellt. Extraktion mit Essigester, Trocknen mit Natriumsulfat und Einengen ergibt 16,2 g farblose Kristalle.

c) (−)-2,3-Dihydro-2,4-dimethyl-2-(3-carboxy-propoxy)-phenyl-benzothiazin-3-on

9 g (24,2 mmol) der racemischen Carbonsäure und 10,4 g (24,2 mmol) Brucin werden in ca. 50 ml Aceton gelöst. Es wird Wasser bis zur gerade eintretenden Trübung zugefügt, die Lösung über Nacht stehen gelassen und dann abgesaugt. Das erhaltene Salz wird aus Aceton/Wasser bis zu einem spezifischen Drehwert von −158 Grad (c = 0.99, Methanol) umkristallisiert. 4,1 g dieses Salzes werden in 60 ml heissem Wasser gelöst und mit 7 ml 1 N KOH versetzt. Das ausgefallene Brucin wird nach 3 Stunden abgesaugt, das Filtrat wird mit 5 N HCl angesäuert, mit Methylenchlorid extrahiert, mit Magnesiumsulfat getrocknet und eingeengt. Man erhält nach Verreiben mit Isopropylether 2,05 g farblose Kristalle vom spez. Drehwert $[a]_D^{20}$ = −245 Grad (c = 1, Methanol).

d) (−)-2,3-Dihydro-2,4-dimethyl-2-[2-(4-hydroxy)-butoxy]-phenyl-benzothiazin-3-on

2,05 g (5,5 mmol) der Verbindung aus Beispiel 95c) werden zusammen mit 0,72 g (7 mmol) Triethylamin in 5 ml Toluol gelöst. Eine Lösung von 0,66 g (7 mmol) Chlorameisensäuremethylester in 30 ml Ether werden zugetropft, danach wird 4 Std. am Rückfluss erhitzt, heiss filtriert und eingeengt. Der Rückstand wird in 10 ml THF gelöst, die Lösung wird zu einer Suspension von 0,6 g Natriumborhydrid in 10 ml abs. THF getropft. Nach 12 Stunden Rühren bei Raumtemperatur wird mit 1 N HCl angesäuert, das THF im Vakuum entfernt, der Rückstand in Essigester/Wasser aufgenommen, mit Essigester extrahiert, die organische Lösung mit ges. Kochsalzlösung gewaschen und über Magnesiumsulfat getrocknet. Nach Einengen wird an Kieselgel mit Essigester/Cyclohexan (1 : 1) chromatographiert. Man erhält 0,7 g farblose Kristalle vom Schmp. 88–91 °C $[a]_D^{20}$ = −302 Grad (c = 1, Methanol).

e) (−)-2,3-Dihydro-2,4-dimethyl-2-[2-(4-methansulfonyloxy-butoxy]-phenyl-benzothiazin-3-on

0,7 g (1,96 mmol) der Verbindung aus Beispiel 95d) werden zusammen mit 0,3 g (0,275 mmol) Triethylamin in 10 ml THF vorgelegt. Bei 0 °C werden 0,22 g (1,96 mmol) Methansulfonylchlorid zugetropft. nach 4 Std. bei Raumtemperatur wird auf 1 N HCl gegossen, mit Essigester extrahiert, mit Magnesiumsulfat getrocknet und eingeengt. Man erhält 1,05 g farbloses Öl.

$^1$H-NMR ($CDCl_3$): δ = 1,2 (t,3H); 1,77 (s,3H); 1,7–2,2 (m,4H); 2,9 (s,3H); 3,38 (s,3H); 3,8–4,4 (m,4H); 6,4–7,3 (m,8H) ppm.

f) (−)-2,3-Dihydro-2,4-dimethyl-2-[2-[4-[4-[2-(3,4,5-trimethoxyphenyl)-ethyl]-piperazinyl]-butoxy]-phenyl]-benzothiazin-3-on.Dihydrochlorid

1,05 g des Mesylats aus Beispiel 95e) werden zusammen mit 0,63 g 2-(3,4,5-Trimethoxyphenyl)ethyl-piperazin, 0,5 g Kaliumcarbonat und 0,4 g Natriumjodid in 20 ml Isopropanol 9 Stunden am Rückfluss erhitzt. Nach Abkühlen wird abgesaugt und eingeengt. Chromatographie an Kieselgel mit Methylenchlorid/Methanol (9 : 1) als Laufmittel ergibt 0,68 g farbloses Öl $[a]_D^{20}$ = −114,5 Grad (c = 0,496, Methanol).

Zur Überführung ins Hydrochlorid wird in Aceton aufgenommen, mit 2,5 N ethanolischer HCl angesäuert, eingeengt, in Aceton aufgenommen und abgesaugt. Man erhält 0,7 g farblose Kristalle, Schmp. 241–244 °C (Zersetzung). $[a]_D^{20}$ = −130 Grad (c = 0,4, Methanol).

Beispiel 96
(+)-2,3-Dihydro-2,4-dimethyl-2-[2-[4-[4-[2-(3,4,5-trimethoxyphenyl)-ethyl]-piperazinyl]-butoxy]-phenyl]-benzothiazin-3-on.Dihydrochlorid

a) 2,3-Dihydro-2,4-dimethyl-2-(2-hydroxy-phenyl)-benzothiazin-3-on-(1S)-Bornan-2-on-1-carbonsäureester

5,3 g (25,7 mmol) (1S)-Bornan-2-on-1-carbonsäurechlorid werden zu 5 g (17,5 mmol) der Verbindung aus Beispiel 1e) gelöst in 70 ml Pyridin gegeben. Nach Zufügen von 1 g 4-Dimethylaminopyridin wird 48 Std. bei Raumtemperatur gerührt. Die Lösung wird auf 1 l gesättigte Oxalsäurelösung gegossen und mit Essigester extrahiert (3 ×). Die vereinigten organischen Phasen werden mit ges. NaCl-Lösung gewaschen, über Natriumsulfat getrocknet und eingeengt. Die Isomeren werden am 1 kg Kieselgel mit Essigester/Cyclohexan (1 : 4) getrennt.

Isomer 1: Rf = 0,18
Schmp. 178–181 °C,
$[a]_D^{20}$ = +299 Grad (c = 0,98, $CH_2Cl_2$)
2,8 g
Isomer 2: Rf = 0,15
Schmp. 159 °C
$[a]_D^{20}$ = −302,5 Grad (c = 1,026, $CH_2Cl_2$)
2,2 g

b) (+)-2,3-Dihydro-2,4-dimethyl-2-(2-hydroxyphenyl)-benzothiazin-3-on

0,5 g Natriumhydrid werden in 40 ml Methanol gelöst, 2 g (4,45 mmol) des Isomer 1 aus Beispiel 96a) werden zugesetzt. Nach 3 Stunden am Rückfluss wird auf 200 ml 1 N HCl gegossen, mit Essigester 3× extrahiert, mit gesättigter NaCl-Lösung gewaschen, mit $MgSO_4$ getrocknet und eingeengt. Kristallisation aus Essigester ergibt 1,25 g farblose Kristalle, Schmp. 182–184 °C, $[a]_D^{20}$ = +351 Grad (c = 0,62, $CH_2Cl_2$).

c) (+)-2,3-Dihydro-2,4-dimethyl-2-[2-(4-brombutoxy)-phenyl]-benzothiazin-3-on

1,25 g der Verbindung aus Beispiel 96b) werden zusammen mit 2,85 g 1,4-Dibrombutan und 1,4 g Kaliumcarbonat in 50 ml Butanon-2 3 Std. am Rückfluss erhitzt. Nach Filtrieren wird eingeengt und der Überschuss Dibrombutan am Hochvakuum entfernt.

d) (+)-2,3-Dihydro-2,4-dimethyl-2-[2-[4-[4-[2-(3,4,5-trimethoxyphenyl)-ethyl]-piperazinyl]-butoxy]-phenyl]-benzothiazin-3-on.Dihydrochlorid

1,85 g der Verbindung aus Beispiel 96c) werden mit 2,0 g 2-(3,4,5-Trimethoxyphenyl)-ethyl-piperazin nach dem in Beispiel 95f) angegebenen Verfahren umgesetzt. Man erhält 1,55 g farblose Kristalle der Titelverbindung vom Schmp. 237–240 °C, $[a]_D^{20}$ = +119,5 Grad (c = 0,41, Methanol).

Beispiel 97
2,3-Dihydro-2-ethyl-4-methyl-2-[2-[4-[4-[2-(3,4,5-trimethoxyphenyl)-ethyl]-piperazinyl]-butoxy]-phenyl]-benzothiazin-3-on.Dihydrochlorid

a) 2,3-Dihydro-2-ethyl-4-methyl-2-(2-methoxyphenyl)-benzothiazin-3-on

0,35 g Natriumhydrid (50% in Öl) werden 2× mit Hexan gewaschen und in 10 ml DMF suspendiert. 1,55 g der Verbindung 2,3-Dihydro-4-methyl-2-(2-methoxyphenyl)-benzothiazin-3-on [erhalten nach folgender Vorschrift: 5,42 g (20 mmol) 2,3-Dihydro-2-(2-methoxyphenyl)-benzothiazin-3-on werden in 100 ml Aceton gelöst, 6,9 ml Methyljodid und 6,5 g Kaliumcarbonat werden zugesetzt und 45 Stunden am Rückfluss erhitzt. Nach Filtration wird eingeengt. Das Produkt kristallisiert beim Verreiben mit Isopropylether. Schmelzpunkt: 150–152 °C. Ausbeute: 5,95 g (100%).
$^1H$-NMR ($CDCl_3$): δ = 7,4–6,6 (m,8H); 5,1 (s,1H); 3,83 (s,3H); 3,53 (s,3H) ppm.]
werden zugegeben und 20 min gerührt. Dann werden bei Raumtemperatur 1,27 g Ethyljodid zugetropft. Nach 3 Stunden wird die Lösung auf Wasser gegossen, mit Essigester 3× extrahiert, die vereinigten organischen Phasen mit Wasser 5× gewaschen, mit Magnesiumsulfat getrocknet und eingeengt. Verreiben mit Isopropylether ergibt 1,35 g Kristalle vom Schmp. 98 °C.

b) 2,3-Dihydro-2-ethyl-4-methyl-2-(2-hydroxyphenyl)-benzothiazin-3-on

1,35 g (4,3 mmol) der Verbindung aus Beispiel 97a) werden nach dem in Beispiel 1e) beschriebenen Verfahren mit Bortribromid umgesetzt. Man erhält 1,1 g farblose Kristalle vom Schmp. 165–168 °C.

c) 2,3-Dihydro-2-ethyl-4-methyl-2-[2-[4-[4-[2-(3,4,5-trimethoxyphenyl)-ethyl]-piperazinyl]-butoxy]-phenyl]-benzothiazin-3-on.Dihydrochlorid

1,1 g der Verbindung aus Beispiel 97b) werden mit 2,38 g 1,4-Dibrombutan nach dem in Beispiel 96c) beschriebenen Verfahren umgesetzt. Man erhält 1,66 g des Bromids, das ohne Reinigung weiter umgesetzt wird. 0,83 g (1,91 mmol) werden zusammen mit 0,8 g 2-(3,4,5-Trimethoxyphenyl)-ethyl-piperazin und 0,7 g Kaliumcarbonat nach dem im Beispiel 96f) beschriebenen Verfahren umgesetzt. Man erhält 0,75 g farblose Kristalle vom Schmp. 220–224 °C.

Beispiel 98
2,3-Dihydro-2-ethyl-4-methyl-2-[2-[4-[4-[2-(3,5-dimethoxyphenyl)-ethyl]-piperazinyl]-butoxy]-phenyl]-benzothiazin-3-on.Dihydrochlorid

Analog der in Beispiel 97c) angegebenen Vorschrift werden 0,83 g der Verbindung aus Beispiel 97b) zunächst mit 1,4-Dibrombutan und dann mit 0,72 g (2,87 mmol) 2-(3,5-Dimethoxyphenyl)-ethyl-piperazin umgesetzt. Man erhält 0,43 g farblose Kristalle vom Schmp. 216–218 °C.

Beispiel 99
2,3-Dihydro-2-isopropyl-4-methyl-2-[2-[4-[4-[2-(3,4,5-trimethoxyphenyl)-ethyl]-piperazinyl]-butoxy]-phenyl]-benzothiazin-3-on.Dihydrochlorid

a) 2,3-Dihydro-2-isopropyl-4-methyl-2-(2-methoxyphenyl)-benzothiazin-3-on

0,35 g (7,6 mmol) NaH (50% in Öl) werden 2× mit Hexan gewaschen, in DMF (15 ml) suspendiert und mit 1,55 g 2,3-Dihydro-4-methyl-2-(2-methoxyphenyl)-benzothiazin-3-on versetzt. Nach 30 min bei Raumtemperatur gibt man 1,29 g (7,6 mmol) 2-Jodpropan zu und rührt noch 3 Stunden. Man verteilt zwischen Essigester und Wasser, wäscht die organische Phase 5× mit Wasser und mit ges. NaCl-Lösung und trocknet mit $MgSO_4$. Die Titelverbindung kristallisiert beim Verreiben mit Isopropylether. Schmp. 121 °C.

b) 2,3-Dihydro-2-isopropyl-4-methyl-2-(2-hydroxyphenyl-benzothiazin-3-on

1,35 g der Verbindung aus Beispiel 99a) werden in 20 ml Methylenchlorid gelöst, 5 ml Bortribromid-Lösung in Hexan (1 M) werden zugetropft. Nach 3 Std. Rühren wird auf 150 ml Wasser gegos-

sen, mit $CH_2Cl_2$ extrahiert und über $MgSO_4$ getrocknet. Nach Einengen wird aus Essigester umkristallisiert. Man erhält 0,75 g farblose Kristalle vom Schmp. 189–192 °C.

c) 2,3-Dihydro-2-isopropyl-4-methyl-2-[2-(4-brombutoxy)-phenyl]-benzothiazin-3-on

0,83 g der Verbindung aus Beispiel 99b) werden zusammen mit 1,72 g 1,4-Dibrombutan und 1 g Kaliumcarbonat in 20 ml Methylethylketon 4 Std. am Rückfluss erhitzt. Nach Abfiltrieren wird eingeengt und aus Isopropylether umkristallisiert. Man erhält 1,19 g farblose Kristalle vom Schmp. 84–87 °C.

d) 2,3-Dihydro-2-isopropyl-4-methyl-2-[2-[4-[4-[2-(3,4,5-trimethoxyphenyl)-ethyl]-piperazinyl]-butoxy]-phenyl]-benzothiazin-3-on.Dihydrochlorid

Hergestellt aus 1,91 g der Verbindung aus Beispiel 99c) und 1 g 2-(3,4,5-Trimethoxyphenyl)-ethyl-piperazin analog der in Beispiel 95f) angegebenen Vorschrift. Man erhält 0,75 g farblose Kristalle vom Schmp. 235–238 °C.

Beispiel 100

2,3-Dihydro-2,4-dimethyl-2-[3-methoxy-[2-[4-[4-[2-(3,4,5-trimethoxy-phenyl)-ethyl]-piperazinyl]-butoxy]-phenyl]-benzothiazin-3-on.Dihydrochlorid

a) 2-Benzyloxy-3-methoxy-benzaldehyd

50 g (0,3 Mol) 3-Methoxysalicylaldehyd werden mit 51,3 g (0,3 Mol) Benzylbromid und 48,4 g Kaliumcarbonat in 300 ml Aceton 3 Stunden am Rückfluss gekocht. Nach Filtration wird eingeengt. Man erhält 71,8 g der Titelverbindung als gelbes Öl.

$^1$H-NMR($CDCl_3$): $\delta$ = 3,78 (s,3H); 5,04 (s,2H); 6,5–7,3 (m,3H); 7,17 (s,5H); 10,0 (s,1H) ppm.

b) 2-Benzyloxy-3-methoxy-benzaldehyd-cyanhydrin

71,8 g der Verbindung aus Beispiel 100a) werden mit 125 ml gesättigter Natriumbisulfitlösung und 15 ml Ethanol auf dem Dampfbad erwärmt. Die Lösung wird mit 200 ml Ethanol und 200 ml Isopropylether versetzt und 3 Std. gerührt. Das ausgefallene Bisulfitaddukt wird abgesaugt und an der Luft getrocknet. Nach Aufnehmen in 200 ml Wasser werden bei 0 °C unter Rühren 32,5 g Kaliumcyanid in 125 ml $H_2O$ zugetropft. Nach 2 Std. wird abgesaugt, mit Wasser gewaschen und aus Essigester/Hexan umkristallisiert. Man erhält 51 g farblose Kristalle vom Schmp. 89–91 °C.

c) 2-Benzyloxy-3-methoxy-mandelsäureethylester

5 g der Verbindung aus Beispiel 100b) werden mit 5 ml abs. Ethanol und 50 ml gesättigter etherischer HCl versetzt und 24 Stunden bei 5 °C stehen gelassen. Die gelbe Lösung wird mit Wasser versetzt, 1 Std. gerührt, die Etherphase abgetrennt, mit $MgSO_4$ getrocknet und eingeengt. Chromatographie an Kieselgel mit Essigester/Cyclohexan (1 : 4) als Laufmittel ergibt 3,75 g der Titelverbindung als Öl.

$^1$H-NMR($CDCl_3$): $\delta$ = 1,05 (t,3H); 3,7 (s,3H); 4,05 (q,3H); 4,95 (s,2H); 5,3 (s,1H); 6,6–6,9 (m,3H); 7,0–7,5 (m,5H) ppm.

d) 2-Benzyloxy-3-methoxy-O-methansulfonyl-mandelsäureethylester

3,75 g der Verbindung aus Beispiel 100c) werden zusammen mit 3,5 ml Triethylamin in 20 ml $CH_2Cl_2$ gelöst. Bei –10 °C werden 1,1 ml Methansulfonylchlorid zugetropft. Nach Aufwärmen auf Raumtemperatur wird noch 90 min gerührt, auf 2 N HCl gegossen, die Phasen getrennt und nochmals mit Methylenchlorid extrahiert. Nach Trocknen mit $MgSO_4$ wird eingeengt. Man erhält 4,7 g der Titelverbindung als Öl.

$^1$H-NMR($CDCl_3$): $\delta$ = 1,1 (t,3H); 2,9 (s,3H); 3,75 (s,3H); 4,1 (q,2H); 5,0 (s,2H); 6,17 (s,1H); 6,88 (s,3H); 7,0–7,4 (m,5H) ppm.

e) 2,3-Dihydro-2-(2-benzyloxy-3-methoxy-phenyl)-benzothiazin-3-on

4,7 g des Mesylats aus Beispiel 100d) werden mit 1,53 g 2-Aminothiophenol und 30 ml DMF 18 Std. bei Raumtemperatur und 2 Std. bei 80 °C gerührt, dann auf 200 ml 1 N HCl gegossen, mit Essigester extrahiert, mit Wasser gewaschen, mit $MgSO_4$ getrocknet, eingeengt. Die Titelverbindung kristallisiert aus Essigester/Ether, 1,9 g, Schmp. 151–153 °C.

f) 2,3-Dihydro-2,4-dimethyl-2-(2-benzyloxy-3-methoxyphenyl)-benzothiazin-3-on

1,9 g der Verbindung aus Beispiel 100e) werden zu 0,8 g mit Hexan gewaschenem und in 15 ml DMF suspendiertem NaH gegeben. Nach 30 min Rühren gibt man 1,25 ml Jodmethan zu, rührt 2 Std. und giesst dann auf 1 N HCl. Extraktion mit Essigester, Waschen der organischen Phase mit $H_2O$ und Trocknen mit $MgSO_4$ ergibt 1,95 g der Titelverbindung als gelbes Öl.

g) 2,3-Dihydro-2,4-dimethyl-2-(2-hydroxy-3-methoxyphenyl)-benzothiazin-3-on

1,95 g der Verbindung aus Beispiel 100f) werden in 20 ml Ethanol und 3 ml 2,5 N ethanolischer HCl mit Pd/C und 1 bar $H_2$-Druck hydriert. Chromatographie an Kieselgel mit Essigester/Cyclohexan (1 : 2) als Laufmittel ergibt 0,35 g der Titelverbindung vom Schmp. 153 °C.

h) 2,3-Dihydro-2,4-dimethyl-2-[3-methoxy-[2-[4-[4-[2-(3,4,5-trimethoxy-phenyl)-ethyl]-piperazinyl]-butoxy]-phenyl]-benzothiazin-3-on.Dihydrochlorid

0,35 g der Verbindung aus Beispiel 100g) werden analog dem in Beispiel 100c) angegebenen Verfahren zuerst mit 0,48 g 1,4-Dibrombutan und anschliessend mit 0,84 g 2-(3,4,5-Trimethoxyphenyl)-ethylpiperazin umgesetzt. Man enthält 0,6 g der Titelverbindung als farblose Kristalle vom Schmp. 183–185 °C.

Beispiel 101

2,3-Dihydro-2,4-dimethyl-2-[4-methoxy-[2-[4-[4-[2-(3,4,5-trimethoxy-phenyl)-ethyl]-piperazinyl]-butoxy]-phenyl]-benzothiazin-3-on.Dihydrochlorid

Hergestellt aus 4-Methoxysalicylaldehyd analog den in Beispiel 100a)–h) angegebenen Vorschriften.

$^1H$-NMR (freie Base, $CDCl_3$): $\delta$ = 1,5–2,0 (m,7H); 2,3–2,9 (m,14H); 3,5 (s,3H); 3,7 (s,3H); 3,8 (s,6H); 3,9 (t,3H); 6,4 (s,2H); 6,5–7,4 (m,9H) ppm.

Beispiel 102
2,3-Dihydro-2,4-dimethyl-2-[5-methoxy-[2-[4-[4-[2-(3,4,5-trimethoxy-phenyl)-ethyl]-piperazinyl]-butoxy]-phenyl]-benzothiazin-3-on.Dihydrochlorid

Hergestellt aus 5-Methoxysalicylaldehyd nach den in Beispiel 100a–h) angegebenen Vorschriften, Schmp. 216 °C.

$^1H$-NMR (freie Base, $CDCl_3$): $\delta$ = 1,5–2,0 (m,7H); 2,3–2,9 (m,14H); 3,58 (s,3H); 3,67 (s,3H); 3,8 (s,6H); 3,9 (t,3H); 6,4 (s,2H); 6,5–7,4 (m,9H) ppm.

Beispiel 103
2,3-Dihydro-2,4-dimethyl-2-[5-chlor-[2-[4-[4-[2-(3,4,5-trimethoxy-phenyl)-ethyl]-piperazinyl]-butoxy]-phenyl]-benzothiazin-3-on.Dihydrochlorid

a) 5-Chlor-salicylaldehyd

64,25 g (0,5 Mol) p-Chlorphenol und 55 g (0,2 Mol) Trioctylamin werden in 100 ml Toluol gelöst. Unter $N_2$ werden 13 g Zinntetrachlorid bei 0 °C zugetropft. Nach 20 min bei Raumtemperatur werden 33 g getrockneter Paraformaldehyd zugegeben und 5 Std. auf 100 °C erhitzt. Nach Abkühlen wird auf 2,5 l $H_2O$ gegossen, mit 2 N HCl auf pH 2 gestellt und mit Diethylether extrahiert. Die Etherphase wird mit ges. NaCl-Lösung gewaschen und mit $Na_2SO_4$ getrocknet und eingeengt. Chromatographie an Kieselgel mit Essigester/Cyclohexan (1 : 6) ergibt 24 g der Titelverbindung, Schmp. 99 °C.

b) 5-Chlor-2-methoxy-benzaldehyd

4,3 g 5-Chlor-salicylaldehyd werden zusammen mit 7,8 g Methyljodid und 5,7 g Kaliumcarbonat in 100 ml Aceton am Rückfluss erhitzt (1 Std.). Nach Filtration wird eingeengt. Man erhält 4,6 g der Titelverbindung als Öl.

c) 5-Chlor-2-methoxy-mandelsäure-methylester

Hergestellt aus 4,6 g 5-Chlor-2-methoxy-benzaldehyd nach der in Beispiel 1a) angegebenen Vorschrift, Ausbeute 2,8 g farbloses Öl.

$^1H$-NMR ($CDCl_3$): $\delta$ = 3,64 (s,3H); 3,74 (s,3H); 5,22 (s,1H); 6,6–7,2 (m,3H) ppm.

d) 5-Chlor-2-methoxy-O-methansulfonyl-mandelsäure-methylester

Hergestellt aus 2,8 g der Verbindung aus Beispiel 103c) nach der in Beispiel 100d) angegebenen Vorschrift durch Umsetzung mit 1,37 g Methansulfonsäurechlorid. Man erhält 3,4 g der Titelverbindung als Öl.

$^1H$-NMR ($CDCl_3$): $\delta$ = 3,08 (s,3H); 3,7 (s,3H); 3,78 (s,3H); 6,1 (s,1H); 6,6–7,3 (m,3H) ppm.

e) 2,3-Dihydro-2-(5-chlor-2-methoxy-phenyl)-benzothiazin-3-on

Hergestellt aus 3,4 g der Verbindung aus Beispiel 103d) und 1,56 g 2-Aminothiophenol nach dem in Beispiel 100e) beschriebenen Verfahren. Man erhält 2,5 g der Titelverbindung als blassgelbe Kristalle.

f) 2,3-Dihydro-2,4-dimethyl-2-(5-chlor-2-methoxy-phenyl)-benzothiazin-3-on

Hergestellt aus 2,5 g der Verbindung aus Beispiel 103e) durch Alkylierung mit 1,5 ml Jodmethan nach der in Beispiel 1d) angegebenen Vorschrift. Man erhält 2,33 g farblose Kristalle vom Schmp. 96 °C.

g) 2,3-Dihydro-2,4-dimethyl-2-(5-chlor-2-hydroxy-phenyl)-benzothiazin-3-on

Hergestellt aus 2,33 g der Verbindung aus Beispiel 103f) nach der in Beispiel 1e) angegebenen Vorschrift. Man erhält 2,1 g der Titelverbindung als farblose Kristalle, Schmp. 211 °C.

h) 2,3-Dihydro-2,4-dimethyl-2-[2-(4-brombutoxy)-5-chlor-phenyl]-benzothiazin-3-on

Hergestellt aus 2,1 g der Verbindung aus Beispiel 103g) und 3,8 ml 1,4-Dibrombutan nach der in Beispiel 96c) angegebenen Vorschrift. Man erhält 2,9 g der Titelverbindung als gelbliches Öl.

i) 2,3-Dihydro-2,4-dimethyl-2-[5-chlor-[2-[4-[4-[2-(3,4,5-trimethoxy-phenyl)-ethyl]-piperazinyl]-butoxy]-phenyl]-benzothiazin-3-on.Dihydrochlorid

Hergestellt aus 1,5 g der Verbindung aus Beispiel 103h) und 1,01 g 2-(3,4,5-Trimethoxyphenyl)-ethyl-piperazin nach dem in Beispiel 96d) beschriebenen Verfahren. Man erhält 1,37 g farblose Kristalle vom Schmp. 246 °C.

Beispiel 104
2,3-Dihydro-2,4-dimethyl-2-[5-chlor-[2-[4-[4-[2-(3,5-dimethoxy-phenyl)-ethyl]-piperazinyl]-butoxy]-phenyl]-benzothiazin-3-on.Dihydrochlorid

Hergestellt aus 1,4 g der Verbindung aus Beispiel 103h) und 0,9 g 2-(3,5-Dimethoxyphenyl)-ethyl-piperazin nach der in Beispiel 96d) angegebenen Vorschrift. Man erhält 0,9 g farblose Kristalle vom Schmp. 208 °C.

Beispiel 105
2,3-Dihydro-2,4-dimethyl-2-[5-methyl-[2-[4-[4-[2-(3,4,5-trimethoxy-phenyl)-ethyl]-piperazinyl]-butoxy]-phenyl]-benzothiazin-3-on.Dihydrochlorid

Hergestellt aus p-Kresol und den entsprechenden Reagenzien analog den in Beispiel 103a–i) angegebenen Vorschriften. Man erhält die Titelverbindung als farblose Kristalle vom Schmp. 238 °C (Zersetzung).

Beispiel 106
2,3-Dihydro-2,4-dimethyl-2-[5-methyl-[2-[4-[4-[2-(3,5-dimethoxy-phenyl)-ethyl]-piperazinyl]-butoxy]-phenyl]-benzothiazin-3-on.Dihydrochlorid

Hergestellt aus p-Kresol und den entsprechenden Reagenzien analog den in Beispiel 103a–h) sowie 104) beschriebenen Verfahren. Man erhält die Titelverbindung als farblose Kristalle vom Schmp. 228 °C.

Beispiel 107
2,3-Dihydro-2,4-dimethyl-2-[5-fluor-[2-[4-[4-[2-(3,4,5-trimethoxy-phenyl)-ethyl]-piperazinyl]-butoxy]-phenyl]-benzothiazin-3-on.Dihydrochlorid

Hergestellt aus 4-Fluorphenol analog den in Beispiel 103a–i) angegebenen Vorschriften.

Beispiel 108
2,3-Dihydro-2,4-dimethyl-2-[2-[4-[4-[2-(3-5-dimethoxy-phenyl)-ethyl]-piperazinyl]-butoxy]-phenyl]-benzothiazin-3-on.Dihydrochlorid

Hergestellt aus 1,5 g (3,7 mmol) 2,3-Dihydro-2,4-dimethyl-2-[2-(4-brombutoxy)-phenyl]-benzothiazin-3-on und 1,1 g 2-(3,5-Dimethoxyphenyl)-ethylpiperazin nach dem in Beispiel 96d) beschriebenen Verfahren. Man erhält 0,9 g farblose Kristalle vom Schmp. 234 °C.

Beispiel 109
(+)-2,3-Dihydro-2-ethyl-4-methyl-2-[2-[4-[4-[2-(3,4,5-trimethoxyphenyl)-ethyl]-piperazinyl]-butoxy]-phenyl]-benzothiazin-3-on.Dihydrochlorid

a) 2,3-Dihydro-2-ethyl-4-methyl-2-(2-hydroxyphenyl)-benzothiazin-3-on-(1S)-Bornan-2-on-1-carbonsäureester.

Hergestellt nach der in Beispiel 96a) angegebenen Vorschrift aus 15,2 g der Verbindung aus Beispiel 97b) und 14 g (1S)-Bornan-2-on-1-carbonsäurechlorid. Nach Chromatographie an Kieselgel mit Essigester/Cyclohexan (1 : 6) als Laufmittel erhält man

Isomer 1: farblose Kristalle, Schmp. 197 °C, $[a]_D^{20} = +280$ Grad (c = 1, $CH_2Cl_2$) 7,1 g

Isomer 2: farblose Kristalle, Schmp. 144 °C $[a]_D^{20} = -264$ Grad (c = 1, $CH_2Cl_2$) 6,9 g

b) (+)-2,3-Dihydro-2-ethyl-4-methyl-2-(2-hydroxyphenyl)-benzothiazin-3-on

Hergestellt nach der in Beispiel 96b) angegebenen Vorschrift aus dem Isomer 1 des Beispiels 109a). Man erhält 3,8 g farblose Kristalle vom Schmp. 187 °C, $[a]_D^{20} = +270,6$ Grad (c = 1, $CH_2Cl_2$).

c) (+)-2,3-Dihydro-2-ethyl-4-methyl-2-[2-[4-[4-[2-(3,4,5-trimethoxyphenyl)-ethyl]-piperazinyl]-butoxy]-phenyl]-benzothiazin-3-on.Dihydrochlorid

Hergestellt aus 3,8 g der Verbindung aus Beispiel 109b) und 7,2 g 2-(3,4,5-Trimethoxyphenyl)-ethyl-piperazin nach der in Beispiel 96c) angegebenen Vorschrift. Man erhält 2,8 g farblose Kristalle vom Schmp. 234 °C, $[a]_D^{20} = +131$ Grad (c = 1, $CH_2Cl_2$).

Beispiel 110
(−)-2,3-Dihydro-2-ethyl-4-methyl-2-[2-[4-[4-[2-(3,4,5-trimethoxyphenyl)-ethyl]-piperazinyl]-butoxy]-phenyl]-benzothiazin-3-on.Dihydrochlorid

Hergestellt nach den Vorschriften des Beispiels 109b) und c) aus dem Isomer 2 des Beispiels 109a). Man erhält 2,4 g farblose Kristalle vom Schmp. 239 °C; $[a]_D^{20} = -142,3$ Grad (c = 1, $CH_2Cl_2$).

Beispiel 111
(+)-2,3-Dihydro-2-isopropyl-4-methyl-2-[2-[4-[4-[2-(3,4,5-trimethoxyphenyl)-ethyl]-piperazinyl]-butoxy]-phenyl]-benzothiazin-3-on.Dihydrochlorid

a) 2,3-Dihydro-2-isopropyl-4-methyl-2-(2-hydroxyphenyl)-benzothiazin-3-on-(1S)-Bornan-2-on-1-carbonsäureester

Hergestellt nach der in Beispiel 96a) angegebenen Vorschrift aus 14,7 g der Verbindung aus Beispiel 99b) und 13 g (1S)-Bornan-2-on-1-carbonsäurechlorid. Nach Chromatographie an Kieselgel mit Essigester/Cyclohexan (1 : 6) als Laufmittel erhält man

Isomer 1: farblose Kristalle, Schmp. 196 °C $[a]_D^{20} = +241$ Grad (c = 1, $CH_2Cl_2$) 7,9 g

Isomer 2: farblose Kristalle, Schmp. 160 °C $[a]_D^{20} = -213$ Grad (c = 1, $CH_2Cl_2$) 5,5 g

b) (+)-2,3-Dihydro-2-isopropyl-4-methyl-2-(2-hydroxyphenyl)-benzothiazin-3-on

Hergestellt nach der in Beispiel 96b) angegebenen Vorschrift aus dem Isomer 1 des Beispiels 111a). Man erhält 3,6 g farblose Kristalle vom Schmp. 146 °C, $[a]_D^{20} = +128$ Grad (c = 1, $CH_2Cl_2$).

c) (+)-2,3-Dihydro-2-isopropyl-4-methyl-2-[2-[4-[4-(3,4,5-trimethoxyphenyl)-ethyl]-piperazinyl]-butoxy]-phenyl]-benzothiazin-3-on.Dihydrochlorid

Hergestellt aus 5,1 g der Verbindung aus Beispiel 111b) und 5,0 g 2-(3,4,5-Trimethoxyphenyl)-ethyl-piperazin nach der in Beispiel 96c) angegebenen Vorschrift. Man erhält 3,2 g farblose Kristalle vom Schmp. 240 °C; $[a]_D^{20} = +115,3$ Grad (c = 1, $CH_2Cl_2$).

Beispiel 112
(−)-2,3-Dihydro-2-isopropyl-4-methyl-2-[2-[4-[4-[2-(3,4,5-trimethoxyphenyl)-ethyl]-piperazinyl]-butoxy]-phenyl]-benzothiazin-3-on.Dihydrochlorid

Hergestellt nach den Vorschriften des Beispiels 111b) und c) aus dem Isomer 2 des Beispiels 111a). Man erhält 1,9 g farblose Kristalle vom Schmp. 242 °C; $[a]_D^{20} = -120,1$ Grad (c = 1, $CH_2Cl_2$).

**Patentansprüche für die Vertragsstaaten: BE, CH, DE, FR, GB, IT, LI, LU, NL, SE**

1. Benzothiazin-Derivate der Formel I,

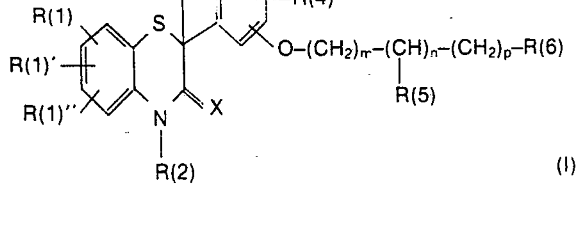

(I)

in welcher

R(1), R(1)' und R(1)'' gleich oder verschieden und voneinander unabhängig Wasserstoff, $(C_1–C_4)$-Alkyl, $(C_1–C_3)$-Alkoxy, Halogen, Nitro, Hydroxy, Acetamido oder Amino,

R(2) Wasserstoff, $(C_1–C_{10})$-Alkyl, geradkettig oder verzweigt, $(C_3–C_{10})$-Alkenyl, geradkettig oder verzweigt, Phenyl, das gegebenenfalls durch einen, zwei oder drei Substituenten der Gruppe $(C_1–C_4)$-Alkyl, $(C_1–C_3)$-Alkoxy, Halogen, $(C_1–C_2)$-Alkylendioxy oder Nitro substituiert sein kann, Phenyl-$(C_1–C_4)$-alkyl, wobei der Phenylring durch einen, zwei oder drei Substituenten der Gruppe $(C_1–C_4)$-Alkyl, $(C_1–C_3)$-Alkoxy, Halogen, $(C_1–C_2)$-Alkylendioxy oder Nitro substituiert sein kann, $(C_4–C_8)$-Cycloalkyl-$(C_1–C_4)$-alkyl, $(C_4–C_8)$-Cycloalkyl,

R(3) $(C_1–C_{10})$-Alkyl, geradkettig oder verzweigt, $(C_3–C_{10})$-Alkenyl, geradkettig oder verzweigt, Phenyl-$(C_1–C_4)$-alkyl, wobei der Phenylrest durch einen, zwei oder drei Substituenten der Gruppe $(C_1–C_4)$-Alkyl, $(C_1–C_3)$-Alkoxy, Halogen, $(C_1–C_2)$-Alkylendioxy oder Nitro substituiert sein kann, oder $(C_4–C_8)$-Cycloalkyl, $(C_4–C_8)$-Cycloalkyl-$(C_1–C_4)$-alkyl,

R(4) und R(4)' gleich oder verschieden und voneinander unabhängig Wasserstoff, $(C_1–C_4)$-Alkyl, $(C_1–C_3)$-Alkoxy, Halogen, Nitro, Hydroxy, Acetamido oder Amino,

R(5) Wasserstoff oder $(C_1–C_3)$-Alkyl,

R(6) eine Teilstruktur aus der folgenden Gruppe,

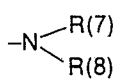
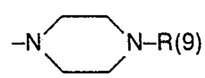
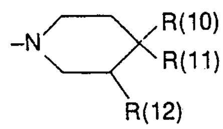
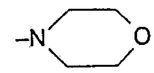

worin

R(7) und R(8) gleich oder verschieden voneinander unabhängig Wasserstoff, $(C_1–C_{10})$-Alkyl, $(C_4–C_8)$-Cycloalkyl, $(C_4–C_8)$-Cycloalkyl-$(C_1–C_4)$-alkyl, Phenyl-$(C_1–C_6)$-alkyl wobei der Phenylrest durch einen, zwei oder drei Reste aus der Gruppe $(C_1–C_4)$-Alkyl, $(C_1–C_4)$Alkoxy, $(C_1–C_2)$-Alkylendioxy, Halogen oder Hydroxy substituiert sein kann, oder Pyridyl-$(C_1–C_4)$-alkyl,

R(9) Wasserstoff $(C_1–C_{10})$-Alkyl, geradkettig oder verzweigt, Phenyl, Phenyl-$(C_1–C_4)$-alkyl, wobei der Phenylrest jeweils durch einen, zwei oder drei Reste aus der Gruppe $(C_1–C_4)$-Alkyl, $(C_1–C_4)$Alkoxy, $(C_1–C_2)$-Alkylendioxy, Halogen oder Hydroxy substituiert sein kann, Pyridyl, Pyrimidinyl, $(C_1–C_5)$-Alkanoyl, Phenyl-$(C_1–C_4)$-alkanoyl, oder Benzoyl, wobei der Phenylrest jeweils durch einen, zwei oder drei Reste aus der Gruppe $(C_1–C_4)$-Alkyl, $(C_1–C_4)$-Alkoxy, $(C_1–C_2)$-Alkylendioxy, Halogen oder Hydroxy substituiert sein kann,

R(10) Wasserstoff, $(C_1–C_{10})$-Alkyl, Phenyl, Phenyl-$(C_1–C_4)$-alkyl wobei der Phenylrest jeweils durch einen, zwei oder drei Reste aus der Gruppe $(C_1–C_4)$-Alkyl, $(C_1–C_4)$-Alkoxy, $(C_1–C_2)$Alkylendioxy, Halogen oder Hydroxy substituiert sein kann,

R(11) Wasserstoff, Hydroxy, $(C_1–C_4)$-Alkoxy oder zusammen mit R(12) eine Bindung, und

R(12) Wasserstoff oder zusammen mit R(11) eine Bindung bedeuten;

in welcher Formel I weiterhin

m 1, 2, 3 oder 4,

n 0 oder 1,

p 0, 1, 2, 3 oder 4, und

X Sauerstoff bedeuten,

sowie die Salze der Verbindungen der Formel I mit physiologisch verträglichen Säuren.

2. Verbindung der Formel I nach Anspruch 1, dadurch gekennzeichnet, dass

R(1) und R(1)' gleich oder verschieden sind und voneinander unabhängig Wasserstoff, Methyl, Ethyl, Methoxy, Ethoxy, Chlor, Brom, Nitro oder Acetamido,

R(1)'' Wasserstoff,

R(2) Wasserstoff, $(C_1–C_6)$-Alkyl, geradkettig oder verzweigt,

Benzyl, Phenethyl, Allyl, Phenyl,
4-Methoxyphenyl, 3-Methoxyphenyl,
3,4-Methylendioxyphenyl, 3,4,5-Trimethoxyphenyl, 3,4-Dimethoxyphenyl, Cyclohexylmethyl,
Cyclopentyl, Cyclohexyl, Cycloheptyl,
4-Methoxybenzyl, 3,4-Dimethoxybenzyl,
3,4,5-Trimethoxybenzyl, 3,4-Methylendioxybenzyl,

R(3) $(C_1–C_6)$-Alkyl, geradkettig oder verzweigt, Benzyl, Phenylethyl, Allyl, Cyclopentyl, Cyclohexyl,

R(4) Wasserstoff, Methyl, Methoxy, Ethoxy, Chlor, Nitro, Hydroxy, Acetamido oder Amino,

R(4)' Wasserstoff,

R(5) Wasserstoff oder Methyl,

R(6) eine Teilstruktur aus der folgenden Gruppe,

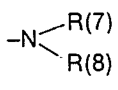
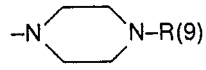
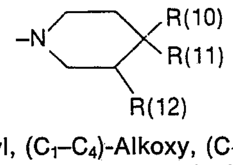

worin

R(7) Wasserstoff, Methyl, Ethyl, Propyl, Isopropyl,

R(8) Wasserstoff, Methyl, Ethyl, Propyl, Isopropyl, Cyclopentylethyl, Cyclohexylethyl, Phenyl-$(C_1–C_4)$-alkyl, wobei der Phenylrest durch einen, zwei oder drei Reste aus der Gruppe $(C_1–C_4)$-Alkyl, $(C_1–C_4)$-Alkoxy, $(C_1–C_2)$-Alkylendioxy, Halogen oder Hydroxy substituiert sein kann, Pyridyl-$(C_1–C_4)$-alkyl,

R(9) wie in Anspruch 1 definiert,

R(10) Phenyl, das gegebenenfalls durch einen, zwei oder drei Reste aus der Gruppe $(C_1–C_4)$-Alkyl, $(C_1–C_4)$-Alkoxy, $(C_1–C_2)$-Alkylendioxy, Halo-

gen oder Hydroxy substituiert sein kann, Phenyl-($C_1$–$C_4$)-alkyl, wobei der Phenylrest durch einen, zwei oder drei Reste aus der Gruppe ($C_1$–$C_4$)-Alkyl, ($C_1$–$C_4$)-Alkoxy, ($C_1$–$C_2$)-Alkylendioxy, Halogen oder Hydroxy substituiert sein kann,

R(11) Wasserstoff und Hydroxy, Methoxy oder zusammen mit R(12) eine Bindung,

R(12) Wasserstoff oder zusammen mit R(11) eine Bindung bedeuten,
in welcher Formel I weiterhin

m 1, 2 oder 3,
n 0 oder 1,
p 1, 2 oder 3, und
X Sauerstoff bedeuten,

sowie die Salze dieser Verbindungen der Formel I mit physiologisch verträglichen Säuren.

3. Verbindung der Formel I nach Anspruch 2, dadurch gekennzeichnet, dass

R(1) Wasserstoff, Methyl, Methoxy, Fluor oder Chlor,
R(1)′ und R(1)″ Wasserstoff,
R(2) Wasserstoff, Methyl, Ethyl, Propyl, Isopropyl, Butyl, sec. Butyl, Isobutyl, Benzyl, Phenethyl, 4-Methoxyphenyl, 3-Methoxyphenyl, 3,4-Dimethoxyphenyl, 3,4,5-Trimethoxyphenyl, Cyclohexylmethyl, Cyclopentyl, Cyclohexyl, Cycloheptyl, 4-Methoxybenzyl, 3,4-Dimethoxybenzyl, 3,4,5-Trimethoxybenzyl, 3,4-Methylendioxybenzyl,
R(3) Methyl, Ethyl, Propyl, Isopropyl, Butyl, sec. Butyl, Isobutyl, Benzyl, Phenylethyl, Allyl, Cyclopentyl, Cyclohexyl,
R(4) Wasserstoff, Methoxy, Methyl, Chlor, Nitro oder Hydroxy,
R(4)′ Wasserstoff,
R(5) Wasserstoff,
R(6) eine Teilstruktur aus der folgenden Gruppe,

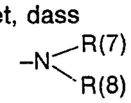 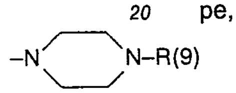 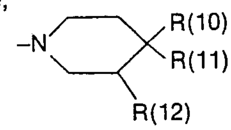

worin
R(7) Wasserstoff oder Methyl,
R(8) Phenyl-($C_1$–$C_4$)-alkyl, wobei der Phenylrest durch einen zwei oder drei Reste aus der Gruppe Methyl, Methoxy, Chlor, Methylendioxy oder Hydroxy substituiert sein kann,
R(9) wie in Anspruch 2 definiert,
R(10) Phenyl, wobei der Phenylrest durch einen zwei oder drei Reste aus der Gruppe Methyl, Methoxy, Chlor, Methylendioxy oder Hydroxy substituiert sein kann,
R(11) Wasserstoff, Hydroxy, Methoxy oder zusammen mit R(12) eine Bindung, und
R(12) Wasserstoff oder zusammen mit R(11) eine Bindung bedeuten;
in welcher Formel I weiterhin
m 1, 2 oder 3,
n 0,
p 0, 1 oder 2, und
X Sauerstoff bedeuten,
sowie die Salze dieser Verbindungen der Formel I mit physiologisch verträglichen Säuren.

4. Verbindung der Formel I nach Anspruch 3, dadurch gekennzeichnet, dass
R(1), R(1)′ und R(1)″ wie in Anspruch 3 definiert,
R(2) Wasserstoff, Methyl, Ethyl, Propyl, Isopropyl, Phenyl,
R(3) Methyl, Ethyl, Propyl, Isopropyl, Butyl, sec. Butyl, Isobutyl, Cyclopentyl, Cyclohexyl,
R(4) Wasserstoff, Methoxy, Methyl oder Chlor
R(4)′ wie in Anspruch 3 definiert,
R(5) wie in Anspruch 3 definiert,
R(6) eine Teilstruktur aus der folgenden Gruppe

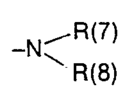 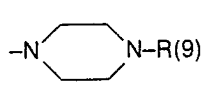

R(7) Methyl
R(8) wie in Anspruch 3 definiert,
R(9) Phenyl-($C_1$–$C_4$)-alkyl, wobei der Phenylrest durch einen, zwei oder drei Reste aus der Gruppe ($C_1$–$C_2$)-Alkoxy, ($C_1$–$C_2$)-Alkylendioxy oder Hydroxy substituiert sein kann,
m 3
X Sauerstoff
p 0 oder 1 sind, sowie die Salze dieser Verbindungen mit physiologisch verträglichen Säuren.

5. Verbindung der Formel I nach Anspruch 4, dadurch gekennzeichnet, dass
R(1) Wasserstoff,
R(1)′ und R(1)″ wie in Anspruch 4 definiert,
R(2) Methyl,
R(3) Methyl, Ethyl, Isopropyl, Isobutyl, sec. Butyl, Cyclopentyl,
R(4), R(4)′ und R(5) wie in Anspruch 4 definiert,

R(6) 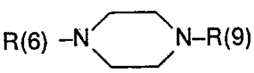

R(9) Phenyl-($C_1$–$C_3$)-alkyl, wobei der Phenylrest durch ein, zwei oder drei Methoxygruppen substituiert sein kann,
X Sauerstoff
bedeuten, sowie deren physiologisch verträgliche Salze.

6. 2,3-Dihydro-2,4-dimethyl-2-[2-[4-[4-[2-(3,4,5-trimethoxyphenyl)-ethyl]-piperazinyl]-butoxy]-phenyl]-benzothiazin-3-on und dessen physiologisch verträgliche Salze.

7. 2,3-Dihydro-2-ethyl-4-methyl-2-[2-[4-[4-[2-(3,4,5-trimethoxyphenyl)-ethyl]-piperazinyl]-butoxy]-phenyl]-benzothiazin-3-on und dessen physiologisch verträgliche Salze.

8. 2,3-Dihydro-2-isopropyl-4-methyl-2-[2-[4-[4-[2-(3,4,5-trimethoxyphenyl)-ethyl]-piperazinyl]-butoxy]-phenyl]-benzothiazin-3-on und dessen physiologisch verträgliche Salze.

9. (+)-2,3-Dihydro-2-isopropyl-4-methyl-2-[2-[4-[4-[2-(3,4,5-trimethoxyphenyl)-ethyl]-piperazinyl]-butoxy]-phenyl]-benzothiazin-3-on und dessen physiologisch verträgliche Salze.

10. Verfahren zur Herstellung von Verbindungen der Formel I gemäss Anspruch 1, dadurch gekennzeichnet, dass man

a) eine Verbindung der Formel II,

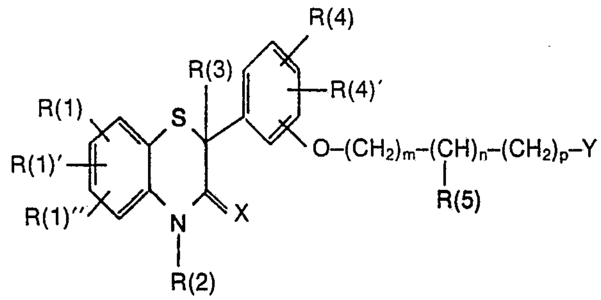

(II)

in welcher R(1), R(1)', R(1)'', R(2), R(3), R(4), R(4)', R(5), X, m, n und p die gleiche Bedeutung wie in Anspruch 1 haben und in welcher Y eine Abgangsgruppe, die nucleophil verdrängt werden kann, insbesondere ein Halogenatom oder einen Sulfonsäurerest, vorzugsweise Methansulfonylrest, einen Benzolsulfonylrest, einen Toluolsulfonylrest oder einen Trifluormethansulfonylrest bedeutet, mit einer der Verbindungen der Formeln IIIa, IIIb, IIIc oder IIId,

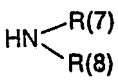

(IIIa)

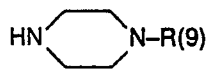

(IIIb)

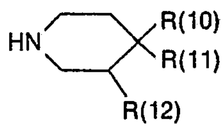

(IIIc)

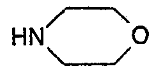

(IIId)

in welchen R(7), R(8), R(9), R(10), R(11) und R(12) die gleiche Bedeutung wie in Anspruch 1 haben, unter Bedingungen einer nucleophilen Substitution, vorzugsweise in einem polaren organischen Lösungsmittel, mit oder ohne Gegenwart einer Hilfsbase zum Abfangen der sich bildenden Säure, bei einer Temperatur zwischen 0 und 160 °C, unsetzt, oder dass man

b) eine Verbindung der Formel IV,

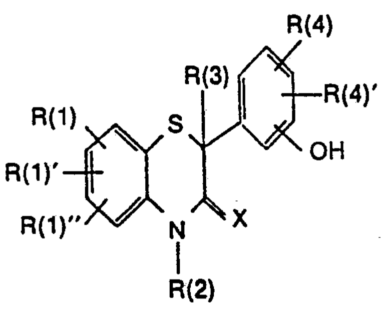

(IV)

in welcher R(1), R(1)', R(1)'', R(2), R(3), R(4), R(4)' und X die gleiche Bedeutung wie in Anspruch 1 haben, mit einer Verbindung der Formel V,

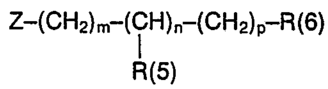

(V)

in welcher Z gleich wie Y unter a) definiert ist und in welcher R(5), R(6), R(7), R(8), R(9), R(10), R(11), R(12), m, n und p die gleiche Bedeutung wie in Anspruch 1 haben, entweder in einem polaren aprotischen Lösungsmittel in Gegenwart einer starken Base bei einer Temperatur zwischen −40 und +60 °C, oder in einem protischen oder aprotischen polaren organischen Lösungsmittel in Gegenwart einer schwachen bis mittelstarken Base bei einer Temperatur zwischen 0 und 160 °C, umsetzt.

11. Verbindungen der allgemeinen Formel II

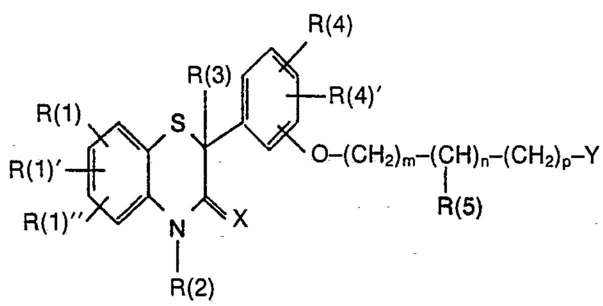

(II)

in welcher R(1), R(1)', R(1)'', R(2), R(3), R(4), R(4)', R(5), X, m, n und p die gleiche Bedeutung wie in Anspruch 1 haben und in welcher Y eine Abgangsgruppe, die nucleophil verdrängt werden kann, insbesondere ein Halogenatom, einen Sulfonsäurerest, vorzugsweise Methansulfonylrest, einen Benzolsulfonylrest, einen Toluolsulfonylrest oder einen Trifluormethansulfonylrest bedeutet.

12. Arzneimittel, dadurch gekennzeichnet, dass es eine Verbindung der Formel I nach Anspruch 1

oder ein Salz dieser Verbindung mit einer physiologisch verträglichen Säure enthält oder daraus besteht.

13. Verwendung einer Verbindung der Formel I nach Anspruch 1 oder eines ihrer Salze mit einer physiologisch verträglichen Säure zum Herstellen eines Medikaments zur Behandlung von Störungen im Calciumhaushalt eines menschlichen oder tierischen Organismus.

**Patentansprüche für den Vertragsstaat: AT**

1. Verfahren zum Herstellen von Verbindungen der Formel I

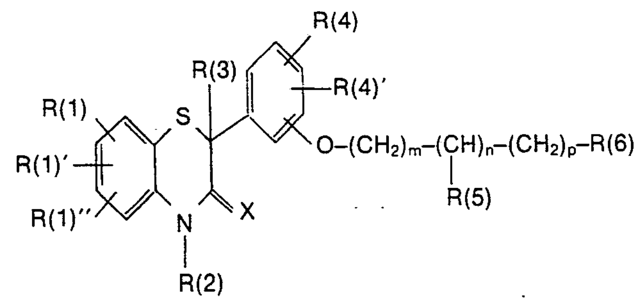

(I)

in welcher

R(1), R(1)′ und R(1)″ gleich oder verschieden und voneinander unabhängig Wasserstoff, ($C_1$–$C_4$)-Alkyl, ($C_1$–$C_3$)-Alkoxy, Halogen, Nitro, Hydroxy, Acetamido oder Amino,

R(2) Wasserstoff, ($C_1$–$C_{10}$)-Alkyl, geradkettig oder verzweigt, ($C_3$–$C_{10}$)-Alkenyl, geradkettig oder verzweigt, Phenyl, das gegebenenfalls durch einen, zwei oder drei Substituenten der Gruppe ($C_1$–$C_4$)-Alkyl, ($C_1$–$C_3$)-Alkoxy, Halogen, ($C_1$–$C_2$)-Alkylendioxy oder Nitro substituiert sein kann, Phenyl-($C_1$–$C_4$)-alkyl, wobei der Phenylring durch einen, zwei oder drei Substituenten der Gruppe ($C_1$–$C_4$)-Alkyl, ($C_1$–$C_3$)-Alkoxy, Halogen, ($C_1$–$C_2$)-Alkylendioxy oder Nitro substituiert sein kann, ($C_4$–$C_8$)-Cycloalkyl-($C_1$–$C_4$)-alkyl, ($C_4$–$C_8$)-Cycloalkyl,

R(3) ($C_1$–$C_{10}$)-Alkyl, geradkettig oder verzweigt, ($C_3$–$C_{10}$)-Alkenyl, geradkettig oder verzweigt, Phenyl-($C_1$–$C_4$)-alkyl, wobei der Phenylrest durch einen, zwei oder drei Substituenten der Gruppe ($C_1$–$C_4$)-Alkyl, ($C_1$–$C_3$)-Alkoxy, Halogen, ($C_1$–$C_2$)-Alkylendioxy oder Nitro substituiert sein kann, oder ($C_4$–$C_8$)-Cycloalkyl, ($C_4$–$C_8$)-Cycloalkyl-($C_1$–$C_4$)-alkyl,

R(4) und R(4)′ gleich oder verschieden und voneinander unabhängig Wasserstoff, ($C_1$–$C_4$)-Alkyl, ($C_1$–$C_3$)-Alkoxy, Halogen, Nitro, Hydroxy, Acetamido oder Amino,

R(5) Wasserstoff oder ($C_1$–$C_3$)-Alkyl,

R(6) eine Teilstruktur aus der folgenden Gruppe,

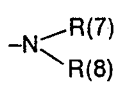 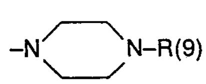 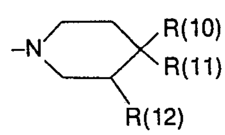 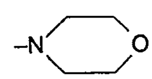

worin

R(7) und R(8) gleich oder verschieden voneinander unabhängig Wasserstoff, ($C_1$–$C_{10}$)-Alkyl, ($C_4$–$C_8$)-Cycloalkyl, ($C_4$–$C_8$)-Cycloalkyl-($C_1$–$C_4$)-alkyl, Phenyl-($C_1$–$C_6$)-alkyl wobei der Phenylrest durch einen, zwei oder drei Reste aus der Gruppe ($C_1$–$C_4$)-Alkyl, ($C_1$–$C_4$)-Alkoxy, ($C_1$–$C_2$)-Alkylendioxy, Halogen oder Hydroxy substituiert sein kann, oder Pyridyl-($C_1$–$C_4$)-alkyl,

R(9) Wasserstoff, ($C_1$–$C_{10}$)-Alkyl, geradkettig oder verzweigt, Phenyl, Phenyl-($C_1$–$C_4$)-alkyl, wobei der Phenylrest jeweils durch einen, zwei oder drei Reste aus der Gruppe ($C_1$–$C_4$)-Alkyl, ($C_1$–$C_4$)Alkoxy, ($C_1$–$C_2$)-Alkylendioxy, Halogen oder Hydroxy substituiert sein kann, Pyridyl, Pyrimidinyl, ($C_1$–$C_5$)-Alkanoyl, Phenyl-($C_1$–$C_4$)-alkanoyl, oder Benzoyl, wobei der Phenylrest jeweils durch einen, zwei oder drei Reste aus der Gruppe ($C_1$–$C_4$)-Alkyl, ($C_1$–$C_4$)-Alkoxy, ($C_1$–$C_2$)-Alkylendioxy, Halogen oder Hydroxy substituiert sein kann,

R(10) Wasserstoff, ($C_1$–$C_{10}$)-Alkyl, Phenyl, Phenyl-($C_1$–$C_4$)-alkyl wobei der Phenylrest jeweils durch einen, zwei oder drei Reste aus der Gruppe ($C_1$–$C_4$)-Alkyl, ($C_1$–$C_4$)-Alkoxy, ($C_1$–$C_2$)-Alkylendioxy, Halogen oder Hydroxy substituiert sein kann,

R(11) Wasserstoff, Hydroxy, ($C_1$–$C_4$)-Alkoxy oder zusammen mit R(12) eine Bindung, und

R(12) Wasserstoff oder zusammen mit R(11) eine Bindung bedeuten;

in welcher Formel I weiterhin

m 1, 2, 3 oder 4,

n 0 oder 1,

p 0, 1, 2, 3 oder 4, und

X Sauerstoff bedeuten,

dadurch gekennzeichnet, dass man

a) eine Verbindung der Formel II,

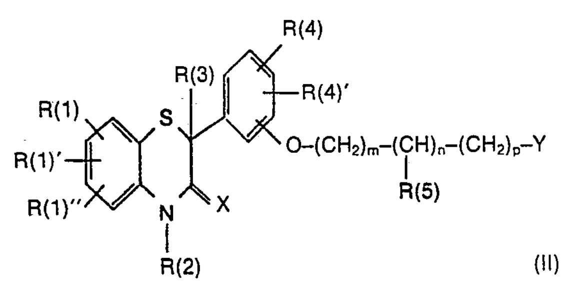

(II)

in welcher R(1), R(1)′, R(1)″, R(2), R(3), R(4), R(4)′, R(5), X, m, n und p die gleiche Bedeutung wie obengenannt haben und in welcher Y eine Abgangsgruppe, die nucleophil verdrängt werden kann, insbesondere ein Halogenatom oder einen Sulfonsäurerest, vorzugsweise Methansulfonylrest, einen Benzolsulfonylrest, einen Toluolsulfonylrest oder einen Trifluormethansulfonylrest bedeutet,

mit einer der Verbindungen der Formeln IIIa, IIIb, IIIc oder IIId,

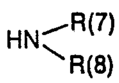
(IIIa)

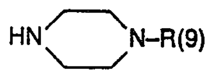
(IIIb)

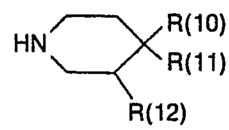
(IIIc)

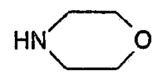
(IIId)

in welchen R(7), R(8), R(9), R(10), R(11) und R(12) die gleiche Bedeutung wie obengenannt haben, unter Bedingungen einer nucleophilen Substitution, vorzugsweise in einem polaren organischen Lösungsmittel, mit oder ohne Gegenwart einer Hilfsbase zum Abfangen der sich bildenden Säure, bei einer Temperatur zwischen 0 und 160 °C, umsetzt, oder dass man

b) eine Verbindung der Formel IV,

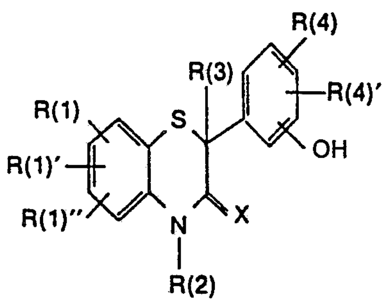
(IV)

in welcher R(1), R(1)′, R(1)″, R(2), R(3), R(4), R(4)′ und X die gleiche Bedeutung wie obengenannt haben, mit einer Verbindung der Formel V,

$$Z-(CH_2)_m-\underset{\displaystyle R(5)}{\underset{|}{(CH)_n}}-(CH_2)_p-R(6) \qquad (V)$$

in welcher Z gleich wie Y unter a) definiert ist und in welcher R(5), R(6), R(7), R(8), R(9), R(10), R(11), R(12), m, n und p die gleiche Bedeutung wie obengenannt haben, entweder in einem polaren aprotischen Lösungsmittel in Gegenwart einer starken Base bei einer Temperatur zwischen −40 und +60 °C, oder in einem protischen oder aprotischen polaren organischen Lösungsmittel in Gegenwart einer schwachen bis mittelstarken Base bei einer Temperatur zwischen 0 und 160 °C, umsetzt.

2. Verfahren nach Anspruch 1, dadurch gekennzeichnet, dass

R(1) und R(1)′ gleich oder verschieden sind und voneinander unabhängig Wasserstoff, Methyl, Ethyl, Methoxy, Ethoxy, Chlor, Brom, Nitro oder Acetamido,

R(1)″ Wasserstoff,

R(2) Wasserstoff, ($C_1$–$C_6$)-Alkyl, geradkettig oder verzweigt,
Benzyl, Phenethyl, Allyl, Phenyl, 4-Methoxyphenyl, 3-Methoxyphenyl, 3,4-Methylendioxyphenyl, 3,4,5-Trimethoxyphenyl,
3,4-Dimethoxyphenyl, Cyclohexylmethyl,
Cyclopentyl, Cyclohexyl, Cycloheptyl,
4-Methoxybenzyl, 3,4-Dimethoxybenzyl,
3,4,5-Trimethoxybenzyl, 3,4-Methylendioxybenzyl,

R(3) ($C_1$–$C_6$)-Alkyl, geradkettig oder verzweigt, Benzyl, Phenylethyl, Allyl, Cyclopentyl, Cyclohexyl,

R(4) Wasserstoff, Methyl, Methoxy, Ethoxy, Chlor, Nitro, Hydroxy, Acetamido oder Amino,

R(4)′ Wasserstoff,

R(5) Wasserstoff oder Methyl,

R(6) eine Teilstruktur aus der folgenden Gruppe,

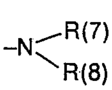

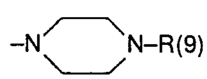

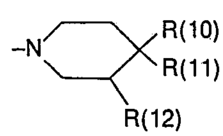

worin

R(7) Wasserstoff, Methyl, Ethyl, Propyl, Isopropyl,

R(8) Wasserstoff, Methyl, Ethyl, Propyl, Isopropyl, Cyclopentylethyl, Cyclohexylethyl, Phenyl-($C_1$–$C_4$)-alkyl, wobei der Phenylrest durch einen, zwei oder drei Reste aus der Gruppe ($C_1$–$C_4$)-Alkyl, ($C_1$–$C_4$)-Alkoxy, ($C_1$–$C_2$)-Alkylendioxy, Halogen oder Hydroxy substituiert sein kann, Pyridyl-($C_1$–$C_4$)-alkyl,

R(9) wie in Anspruch 1 definiert,

R(10) Phenyl, das gegebenenfalls durch einen, zwei oder drei Reste aus der Gruppe ($C_1$–$C_4$)-Alkyl, ($C_1$–$C_4$)-Alkoxy, ($C_1$–$C_2$)-Alkylendioxy, Halogen oder Hydroxy substituiert sein kann, Phenyl-($C_1$–$C_4$)-alkyl, wobei der Phenylrest durch einen, zwei oder drei Reste aus der Gruppe ($C_1$–$C_4$)-Alkyl, ($C_1$–$C_4$)-Alkoxy, ($C_1$–$C_2$)-Alkylendioxy, Halogen oder Hydroxy substituiert sein kann,

R(11) Wasserstoff und Hydroxy, Methoxy oder zusammen mit R(12) eine Bindung,

R(12) Wasserstoff oder zusammen mit R(11) eine Bindung bedeuten;

in welcher Formel I weiterhin

m 1, 2 oder 3,

n 0 oder 1,

p 1, 2 oder 3, und

X Sauerstoff bedeuten.

3. Verfahren nach Anspruch 2, dadurch gekennzeichnet, dass

R(1) Wasserstoff, Methyl, Methoxy, Fluor oder Chlor,

R(1)′ und R(1)″ Wasserstoff,

R(2) Wasserstoff, Methyl, Ethyl, Propyl, Isopropyl, Butyl, sec. Butyl, Isobutyl, Benzyl, Phenethyl, 4-Methoxyphenyl, 3-Methoxyphenyl, 3,4-Dimethoxyphenyl, 3,4,5-Trimethoxyphenyl, Cyclohexylmethyl, Cyclopentyl, Cyclohexyl,

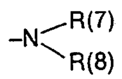 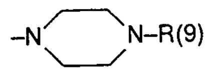 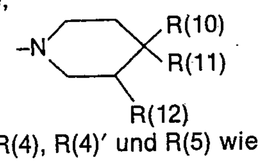

worin

R(7) Wasserstoff oder Methyl,

R(8) Phenyl-($C_1$–$C_4$)-alkyl, wobei der Phenylrest durch einen zwei oder drei Reste aus der Gruppe Methyl, Methoxy, Chlor, Methylendioxy oder Hydroxy substituiert sein kann,

R(9) wie in Anspruch 2 angegeben definiert ist,

R(10) Phenyl, wobei der Phenylrest durch einen, zwei oder drei Reste aus der Gruppe Methyl, Methoxy, Chlor, Methylendioxy oder Hydroxy substituiert sein kann,

R(11) Wasserstoff, Hydroxy, Methoxy oder zusammen mit R(12) eine Bindung, und

R(12) Wasserstoff oder zusammen mit R(11) eine Bindung bedeuten;

in welcher Formel I weiterhin

m 1, 2 oder 3,

n 0,

p 0, 1 oder 2, und

X Sauerstoff bedeuten.

4. Verfahren nach Anspruch 3, dadurch gekennzeichnet, dass

R(1), R(1)′ und R(1)″ wie in Anspruch 3 definiert,

R(2) Wasserstoff, Methyl, Ethyl, Propyl, Isopropyl, Phenyl,

R(3) Methyl, Ethyl, Propyl, Isopropyl, Butyl, sec. Butyl, Isobutyl, Cyclopentyl, Cyclohexyl

R(4) Wasserstoff, Methoxy, Methyl oder Chlor

R(4)′ und R(5) wie in Anspruch 3 definiert,

R(6) eine Teilstruktur aus der folgenden Gruppe

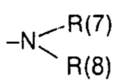 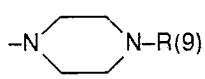

R(7) Methyl

R(8) wie in Anspruch 3 definiert,

R(9) Phenyl, ($C_1$–$C_4$)-alkyl, wobei der Phenylrest durch einen, zwei oder drei Reste aus der Gruppe ($C_1$–$C_2$)-Alkoxy, ($C_1$–$C_2$)-Alkylendioxy oder Hydroxy substituiert sein kann,

m 3

X Sauerstoff

p 0 oder 1 sind.

5. Verfahren nach Anspruch 4, dadurch gekennzeichnet, dass

R(1) Wasserstoff,

R(1)′ und R(1)″ wie in Anspruch 4 definiert,

R(2) Methyl,

R(3) Methyl, Ethyl, Isopropyl, Isobutyl, sec. Butyl, Cyclopentyl, Cycloheptyl, 4-Methoxybenzyl, 3,4-Dimethoxybenzyl, 3,4,5-Trimethoxybenzyl, 3,4-Methylendioxybenzyl,

R(3) Methyl, Ethyl, Propyl, Isopropyl, Butyl, sec. Butyl, Isobutyl, Benzyl, Phenylethyl, Allyl, Cyclopentyl, Cyclohexyl,

R(4) Wasserstoff, Methoxy, Methyl, Chlor, Nitro oder Hydroxy,

R(4)′ Wasserstoff,

R(5) Wasserstoff,

R(6) eine Teilstruktur aus der folgenden Gruppe,

R(4), R(4)′ und R(5) wie in Anspruch 4 definiert,

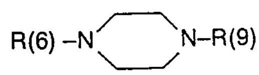

R(9) Phenyl-($C_1$–$C_3$)-alkyl, wobei der Phenylrest durch ein, zwei oder drei Methoxygruppen substituiert sein kann,

X Sauerstoff

bedeuten.

6. Verfahren nach Anspruch 1, dadurch gekennzeichnet, dass

2,3-Dihydro-2,4-dimethyl-2-[2-[4-[4-[2-(3,4,5-trimethoxyphenyl)-ethyl]-piperazinyl]-butoxy]-phenyl]-benzothiazin-3-on

hergestellt wird.

7. Verfahren nach Anspruch 1, dadurch gekennzeichnet, dass

2,3-Dihydro-2-ethyl-4-methyl-2-[2-[4-[4-[2-(3,4,5-trimethoxyphenyl)-ethyl]-piperazinyl]-butoxy]-phenyl]-benzothiazin-3-on

hergestellt wird.

8. Verfahren nach Anspruch 1, dadurch gekennzeichnet, dass

2,3-Dihydro-2-isopropyl-4-methyl-2-[2-[4-[4-[2-(3,4,5-trimethoxyphenyl)-ethyl]-piperazinyl]-butoxy]-phenyl]-benzothiazin-3-on

hergestellt wird.

9. Verfahren nach Anspruch 1, dadurch gekennzeichnet, dass

(+)-2,3-Dihydro-2-isopropyl-4-methyl-2-[2-[4-[4-[2-(3,4,5-trimethoxyphenyl)-ethyl]-piperazinyl]-butoxy]-phenyl]-benzothiazin-3-on

hergestellt wird.

**Claims for the contracting States: BE, CH, DE, FR, GB, IT, LI, LU, NL, SE**

1. A benzothiazine derivative of the formula I

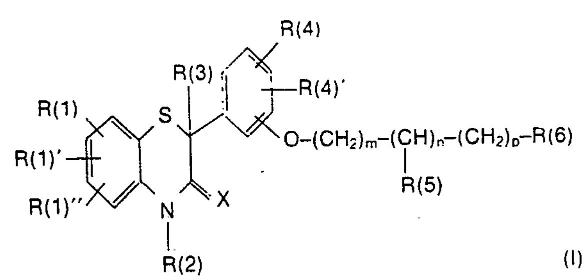

(I)

in which

R(1), R(1)′ and R(1)″ denote, identically or differently and independently of one another, hydrogen, $(C_1-C_4)$-alkyl, $(C_1-C_3)$-alkoxy, halogen, nitro, hydroxyl, acetamido or amino,

R(2) denotes hydrogen, $(C_1-C_{10})$-alkyl, straight-chain or branched, $(C_3-C_{10})$-alkenyl, straight-chain or branched, phenyl which can optionally be substituted by one, two or three substituents from the group comprising $(C_1-C_4)$-alkyl, $(C_1-C_3)$-alkoxy, halogen, $(C_1-C_2)$-alkylenedioxy or nitro, phenyl-$(C_1-C_4)$-alkyl, it being possible for the phenyl ring to be substituted by one, two or three substituents from the group comprising $(C_1-C_4)$-alkyl, $(C_1-C_3)$-alkoxy, halogen, $(C_1-C_2)$-alkylenedioxy or nitro, $(C_4-C_8)$-cycloalkyl-$(C_1-C_4)$-alkyl, or $(C_4-C_8)$-cycloalkyl,

R(3) denotes $(C_1-C_{10})$-alkyl, straight-chain or branched, $(C_3-C_{10})$-alkenyl, straight-chain or branched, phenyl-$(C_1-C_4)$-alkyl, it being possible for the phenyl radical to be substituted by one, two or three substituents from the group comprising $(C_1-C_4)$-alkyl, $(C_1-C_3)$-alkoxy, halogen, $(C_1-C_2)$-alkylenedioxy or nitro, or denotes $(C_4-C_8)$-cycloalkyl, or $(C_4-C_8)$-cycloalkyl-$(C_1-C_4)$-alkyl,

R(4) and R(4)′ denote, identically or differently and independently of one another, hydrogen, $(C_1-C_4)$-alkyl, $(C_1-C_3)$-alkoxy, halogen, nitro, hydroxyl, acetamido or amino,

R(5) denotes hydrogen or $(C_1-C_3)$-alkyl,

R(6) denotes a part structure from the following group,

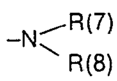

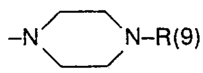

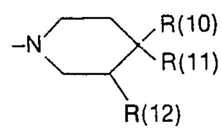

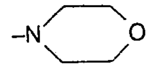

in which

R(7) and R(8) denote, identically or differently and independently of one another, hydrogen, $(C_1-C_{10})$-alkyl, $(C_4-C_8)$-cycloalkyl, $(C_4-C_8)$-cycloalkyl-$(C_1-C_4)$-alkyl, phenyl-$(C_1-C_6)$-alkyl, it being possible for the phenyl radical to be substituted by one, two or three radicals from the group comprising $(C_1-C_4)$-alkyl, $(C_1-C_4)$-alkoxy, $(C_1-C_2)$-alkylenedioxy, halogen or hydroxyl, or denote pyridyl-$(C_1-C_4)$-alkyl,

R(9) denotes hydrogen, $(C_1-C_{10})$-alkyl, straight-chain or branched, phenyl, phenyl-$(C_1-C_4)$-alkyl, it being possible for the phenyl radical in each case to be substituted by one, two or three radicals from the group comprising $(C_1-C_4)$-alkyl, $(C_1-C_4)$-alkoxy, $(C_1-C_2)$-alkylenedioxy, halogen or hydroxyl, or denotes pyridyl, pyrimidinyl, $(C_1-C_5)$-alkanoyl, phenyl-$(C_1-C_4)$-alkanoyl, or benzoyl, it being possible for the phenyl radical in each case to be substituted by one, two or three radicals from the group comprising $(C_1-C_4)$-alkyl, $(C_1-C_4)$-alkoxy, $(C_1-C_2)$-alkylenedioxy, halogen or hydroxyl,

R(10) denotes hydrogen, $(C_1-C_{10})$-alkyl, phenyl, phenyl-$(C_1-C_4)$-alkyl, it being possible for the phenyl radical in each case to be substituted by one, two or three radicals from the group comprising $(C_1-C_4)$-alkyl, $(C_1-C_4)$-alkoxy, $(C_1-C_2)$-alkylenedioxy, halogen or hydroxyl,

R(11) denotes hydrogen, hydroxyl, $(C_1-C_4)$-alkoxy or, together with R(12), denotes a bond, and

R(12) denotes hydrogen or, together with R(11), denotes a bond;

in which formula I in addition

m denotes 1, 2, 3 or 4,

n denotes 0 or 1,

p denotes 0, 1, 2, 3 or 4, and

X denotes oxygen,

and the salts of the compound of the formula I with physiologically tolerated acids.

2. A compound of the formula I as claimed in claim 1, wherein

R(1) and R(1)′ are identical or different and, independently of one another, denote hydrogen, methyl, ethyl, methoxy, ethoxy, chlorine, bromine, nitro or acetamido,

R(1)″ denotes hydrogen,

R(2) denotes hydrogen, $(C_1-C_6)$-alkyl, straight-chain or branched,
benzyl, phenethyl, allyl, phenyl,
4-methoxyphenyl, 3-methoxyphenyl,
3,4-methylenedioxyphenyl,
3,4,5-trimethoxyphenyl, 3,4-dimethoxyphenyl,
cyclohexylmethyl, cyclopentyl, cyclohexyl,
cycloheptyl, 4-methoxybenzyl,
3,4-dimethoxybenzyl, 3,4,5-trimethoxybenzyl or
3,4-methylenedioxybenzyl,

R(3) denotes $(C_1-C_6)$-alkyl, straight-chain or branched, benzyl, phenylethyl, allyl, cyclopentyl or cyclohexyl,

R(4) denotes hydrogen, methyl, methoxy, ethoxy, chlorine, nitro, hydroxyl, acetamido or amino,

R(4)′ denotes hydrogen,

R(5) denotes hydrogen or methyl,

R(6) denotes a part structure from the following group,

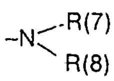

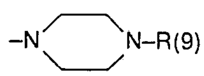

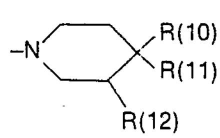

in which

R(7) denotes hydrogen, methyl, ethyl, propyl or isopropyl,

R(8) denotes hydrogen, methyl, ethyl, propyl or isopropyl, cyclopentylethyl, cyclohexylethyl, phenyl-$(C_1-C_4)$-alkyl, it being possible for the phenyl radical to be substituted by one, two or three radicals from the group comprising $(C_1-C_4)$-alkyl, $(C_1-C_4)$-alkoxy, $(C_1-C_2)$-alkylenedioxy, halogen or hydroxyl, or denotes pyridyl-$(C_1-C_4)$-alkyl,

R(9) denotes as defined in claim 1,

R(10) denotes phenyl which can optionally be substituted by one, two or three radicals from the group comprising ($C_1$–$C_4$)-alkyl, ($C_1$–$C_4$)-alkoxy, ($C_1$–$C_2$)-alkylenedioxy, halogen or hydroxyl, or phenyl-($C_1$–$C_4$)-alkyl, it being possible for the phenyl radical to be substituted by one, two or three radicals from the group comprising ($C_1$–$C_4$)-alkyl, ($C_1$–$C_4$)-alkoxy, ($C_1$–$C_2$)-alkylenedioxy, halogen or hydroxyl,

R(11) denotes hydrogen and hydroxyl, methoxy or, together with R(12), denotes a bond,

R(12) denotes hydrogen or, together with R(11), denotes a bond;

in which formula I in addition

m denotes 1, 2 or 3,

n denotes 0 or 1,

p denotes 1, 2 or 3, and

X denotes oxygen,

and the salts of this compound of the formula I with physiologically tolerated acids.

3. A compound of the formula I as claimed in claim 2, wherein

R(1) denotes hydrogen, methyl, methoxy, fluorine or chlorine,

R(1)′ and R(1)″ denote hydrogen,

R(2) denotes hydrogen, methyl, ethyl, propyl, isopropyl, butyl, sec.-butyl, isobutyl, benzyl, phenethyl, 4-methoxyphenyl, 3-methoxyphenyl, 3,4-dimethoxyphenyl, 3,4,5-trimethoxyphenyl, cyclohexylmethyl, cyclopentyl, cyclohexyl, cycloheptyl, 4-methoxybenzyl, 3,4-dimethoxybenzyl, 3,4,5-trimethoxybenzyl or 3,4-methylenedioxybenzyl,

R(3) denotes methyl, ethyl, propyl, isopropyl, butyl, sec.-butyl, isobutyl, benzyl, phenylethyl, allyl, cyclopentyl or cyclohexyl,

R(4) denotes hydrogen, methoxy, methyl, chlorine, nitro or hydroxyl,

R(4)′ denotes hydrogen,

R(5) denotes hydrogen,

R(6) denotes a part structure from the following group,

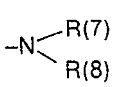
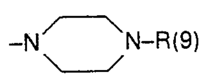
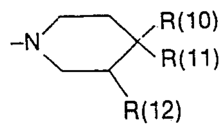

in which

R(7) denotes hydrogen or methyl,

R(8) denotes phenyl-($C_1$–$C_4$)-alkyl, it being possible for the phenyl radical to be substituted by one, two or three radicals from the group comprising methyl, methoxy, chlorine, methylenedioxy or hydroxyl,

R(9) denotes as defined in claim 2,

R(10) denotes phenyl, it being possible for the phenyl radical to be substituted by one, two or three radicals from the group comprising methyl, methoxy, chlorine, methylenedioxy or hydroxyl,

R(11) denotes hydrogen, hydroxyl, methoxy or, together with R(12), denotes a bond, and

R(12) denotes hydrogen or, together with R(11), denotes a bond;

in which formula I in addition

m denotes 1, 2 or 3,

n denotes 0,

p denotes 0, 1 or 2, and

X denotes oxygen,

and the salts of this compound of the formula I with physiologically tolerated acids.

4. A compound of the formula I as claimed in claim 3, wherein

R(1), R(1)′ and R(1)″ denote as defined in claim 3,

R(2) is hydrogen, methyl, ethyl, propyl, isopropyl or phenyl,

R(3) is methyl, ethyl, propyl, isopropyl, butyl, sec.-butyl, isobutyl, cyclopentyl or cyclohexyl,

R(4) is hydrogen, methoxy, methyl or chlorine,

R(4)′ is as defined in claim 3,

R(5) is as defined in claim 3,

R(6) is a part structure from the following group

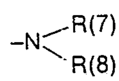
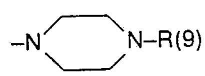

in which

R(7) is methyl,

R(8) is as defined in claim 3,

R(9) is phenyl-($C_1$–$C_4$)-alkyl, it being possible for the phenyl radical to be substituted by one, two or three radicals from the group comprising ($C_1$–$C_2$)-alkoxy, ($C_1$–$C_2$)-alkylenedioxy or hydroxyl,

m is 3,

X is oxygen,

p is 0 or 1, and the salts of this compound with physiologically tolerated acids.

5. A compound of the formula I as claimed in claim 4, wherein

R(1) denotes hydrogen,

R(1)′ and R(1)″ denote as defined in claim 4,

R(2) denotes methyl,

R(3) denotes methyl, ethyl, isopropyl, isobutyl, sec.-butyl or cyclopentyl,

R(4), R(4)′ and R(5) denote as defined in claim 4,

R(6) denotes 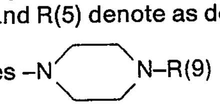

and

R(9) denotes phenyl-($C_1$–$C_3$)-alkyl, it being possible for the phenyl radical to be substituted by one, two or three methoxy groups,

X denotes oxygen,

and its physiologically tolerated salts.

6. 2,3-Dihydro-2,4-dimethyl-2-[2-[4-[4-[2-(3,4,5-trimethoxyphenyl)ethyl]piperazinyl]butoxy]phenyl]benzothiazin-3-one

and its physiologically tolerated salts.

7. 2,3-Dihydro-2-ethyl-4-methyl-2-[2-[4-[4-[2-(3,4,5-trimethoxyphenyl)ethyl]piperazinyl]butoxy]phenyl]benzothiazin-3-one
and its physiologically tolerated salts.

8. 2,3-Dihydro-2-isopropyl-4-methyl-2-[2-[4-[4-[2-(3,4,5-trimethoxyphenyl)ethyl]piperazinyl]-butoxy]phenyl]benzothiazin-3-one
and its physiologically tolerated salts.

9. (+)-2,3-Dihydro-2-isopropyl-4-methyl-2-[2-[4-[4-[2-(3,4,5-trimethoxyphenyl)piperazinyl]butoxy]phenyl]benzothiazin-3-one
and its physiologically tolerated salts.

10. A process for the preparation of a compound of the formula I as claimed in claim 1, which comprises

a) reacting a compound of the formula II

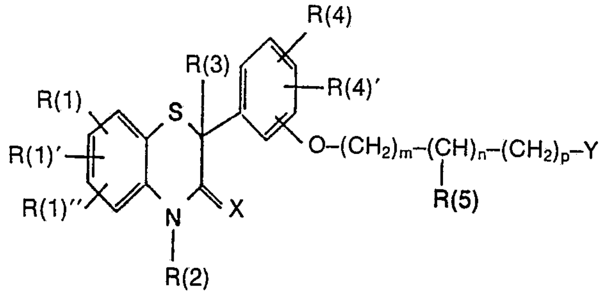

(II)

in which R(1), R(1)′, R(1)″, R(2), R(3), R(4), R(4)′, R(5), X, m, n and p have the same meaning as in claim 1, and in which Y denotes a leaving group which can undergo nucleophilic displacement, in particular a halogen atom or a sulfonic acid radical, preferably methanesulfonyl radical, a benzenesulfonyl radical, a toluenesulfonyl radical or a trifluoromethanesulfonyl radical, with one of the compounds of the formulae IIIa, IIIb, IIIc or IIId,

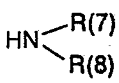

(IIIa)

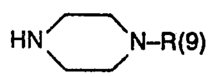

(IIIb)

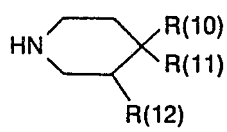

(IIIc)

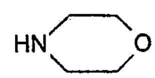

(IIId)

in which R(7), R(8), R(9), R(10), R(11) and R(12) have the same meaning as in claim 1, under the conditions of nucleophilic substitution, preferably in a polar organic solvent, with or without the presence of an auxiliary base to capture the acid which is formed, at a temperature between 0 and 160 °C, or which comprises

b) reacting a compound of the formula IV

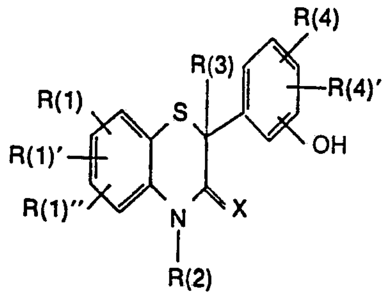

(IV)

in which R(1), R(1)′, R(1)″, R(2), R(3), R(4), R(4)″ and X have the same meaning as in claim 1, with a compound of the formula V

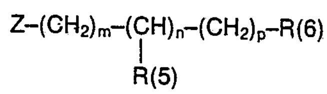

(V)

in which Z is defined identically to Y under a), and in which R(5), R(6), R(7), R(8), R(9), R(10), R(11), R(12), m, n and p have the same meaning as in claim 1, either in a polar aprotic solvent in the presence of a strong base at a temperature between −40 and +60 °C, or in a protic or aprotic polar organic solvent in the presence of a weak to moderately strong base at a temperature between 0 and 160 °C.

11. A compound of the formula II

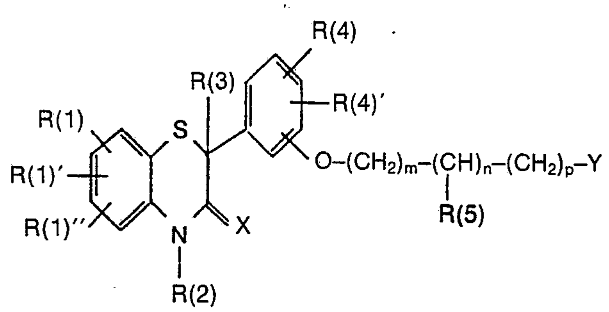

(II)

in which R(1), R(1)′, R(1)″, R(2), R(3), R(4), R(4)′, R(5), X, m, n and p have the same meaning as in claim 1, and in which Y denotes a leaving group which can undergo nucleophilic displacement, in particular a halogen atom, a sulfonic acid radical, preferably methanesulfonyl radical, a benzenesulfonyl radical, a toluenesulfonyl radical or a trifluoromethanesulfonyl radical.

12. A medicament which contains or is composed of a compound of the formula I as claimed in claim 1 or a salt of this compound with a physiologically tolerated acid.

13. The use of a compound of the formula I as claimed in claim 1 or one of its salts with a physiologically tolerated acid for the preparation of a medicament for the treatment of disturbances of the calcium balance of a human or animal body.

**Claims for the contracting state: AT**

1. A process for the preparation of a compound of the formula I

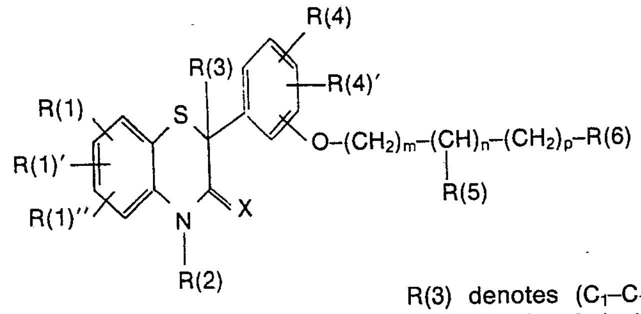

(I)

in which

R(1), R(1)′ and R(1)″ denote, identically or differently and independently of one another, hydrogen, ($C_1$–$C_4$)-alkyl, ($C_1$–$C_3$)-alkoxy, halogen, nitro, hydroxyl, acetamido or amino,

R(2) denotes hydrogen, ($C_1$–$C_{10}$)-alkyl, straight-chain or branched, ($C_3$–$C_{10}$)-alkenyl, straight-chain or branched, phenyl which can optionally be substituted by one, two or three substituents from the group comprising ($C_1$–$C_4$)-alkyl, ($C_1$–$C_3$)-alkoxy, halogen, ($C_1$–$C_2$)-alkylenedioxy or nitro, phenyl-($C_1$–$C_4$)-alkyl, it being possible for the phenyl ring to be substituted by one, two or three substituents from the group comprising ($C_1$–$C_4$)-alkyl, ($C_1$–$C_3$)-alkoxy, halogen, ($C_1$–$C_2$)-alkylenedioxy or nitro, ($C_4$–$C_8$)-cycloalkyl-($C_1$–$C_4$)-alkyl, or ($C_4$–$C_8$)-cycloalkyl,

R(3) denotes ($C_1$–$C_{10}$)-alkyl, straight-chain or branched, ($C_3$–$C_{10}$)-alkenyl, straight-chain or branched, phenyl-($C_1$–$C_4$)-alkyl, it being possible for the phenyl radical to be substituted by one, two or three substituents from the group comprising ($C_1$–$C_4$)-alkyl, ($C_1$–$C_3$)-alkoxy, halogen, ($C_1$–$C_2$)-alkylenedioxy or nitro, or denotes ($C_4$–$C_8$)-cycloalkyl or ($C_4$–$C_8$)-cycloalkyl-($C_1$–$C_4$)-alkyl,

R(4) and R(4)′ denote, identically or differently and independently of one another, hydrogen, ($C_1$–$C_4$)-alkyl, ($C_1$–$C_3$)-alkoxy, halogen, nitro, hydroxyl, acetamido or amino,

R(5) denotes hydrogen or ($C_1$–$C_3$)-alkyl,

R(6) denotes a part structure from the following group,

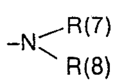

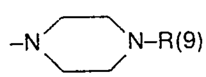

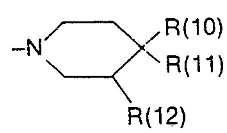

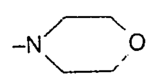

in which

R(7) and R(8) denote, identically or differently and independently of one another, hydrogen, ($C_1$–$C_{10}$)-alkyl, ($C_4$–$C_8$)-cycloalkyl, ($C_4$–$C_8$)-cycloalkyl-($C_1$–$C_4$)-alkyl, phenyl-($C_1$–$C_6$)-alkyl, it being possible for the phenyl radical to be substituted by one, two or three radicals from the group comprising ($C_1$–$C_4$)-alkyl, ($C_1$–$C_4$)-alkoxy, ($C_1$–$C_2$)-alkylenedioxy, halogen or hydroxyl, or denote pyridyl-($C_1$–$C_4$)-alkyl,

R(9) denotes hydrogen, ($C_1$–$C_{10}$)-alkyl, straight-chain or branched, phenyl, phenyl-($C_1$–$C_4$)-alkyl, it being possible for the phenyl radical in each case to be substituted by one, two or three radicals from the group comprising ($C_1$–$C_4$)-alkyl, ($C_1$–$C_4$)-alkoxy, ($C_1$–$C_2$)-alkylenedioxy, halogen or hydroxyl, or denotes pyridyl, pyrimidinyl, ($C_1$–$C_5$)-alkanoyl, phenyl-($C_1$–$C_4$)-alkanoyl, or benzoyl, it being possible for the phenyl radical in each case to be substituted by one, two or three radicals from the group comprising ($C_1$–$C_4$)-alkyl, ($C_1$–$C_4$)-alkoxy, ($C_1$–$C_2$)-alkylenedioxy, halogen or hydroxyl,

R(10) denotes hydrogen, ($C_1$–$C_{10}$)-alkyl, phenyl, phenyl-($C_1$–$C_4$)-alkyl, it being possible for the phenyl radical in each case to be substituted by one, two or three radicals from the group comprising ($C_1$–$C_4$)-alkyl, ($C_1$–$C_4$)-alkoxy, ($C_1$–$C_2$)-alkylenedioxy, halogen or hydroxyl,

R(11) denotes hydrogen, hydroxyl, ($C_1$–$C_4$)-alkoxy or, together with R(12), denotes a bond, and

R(12) denotes hydrogen or, together with R(11), denotes a bond;

in which formula I in addition

m denotes 1, 2, 3 or 4,

n denotes 0 or 1,

p denotes 0, 1, 2, 3 or 4, and

X denotes oxygen,

which comprises

a) reacting a compound of the formula II

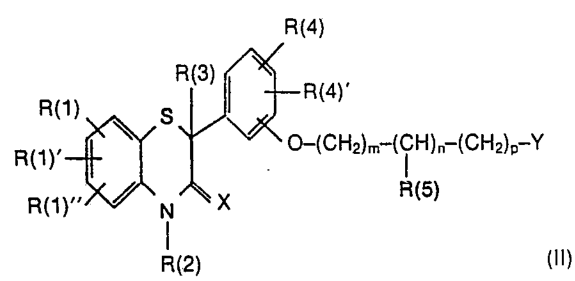

(II)

in which R(1), R(1)′, R(1)″, R(2), R(3), R(4), R(4)′, R(5), X, m, n and p have the same meaning as abovementioned, and in which Y denotes a leaving group which can undergo nucleophilic displacement, in particular a halogen atom or a sulfonic acid radical, preferably methanesulfonyl radical, a benzenesulfonyl radical, a toluenesulfonyl radical or a trifluoromethanesulfonyl radical, with one of the compounds of the formulae IIIa, IIIb, IIIc or IIId,

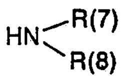

(IIIa)

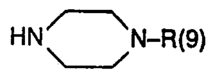

(IIIb)

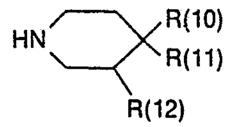

(IIIc)

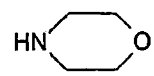

(IIId)

in which R(7), R(8), R(9), R(10), R(11) and R(12) have the same meaning as abovementioned, under the conditions of nucleophilic substitution, preferably in a polar organic solvent, with or without the presence of an auxiliary base to capture the acid which is formed, at a temperature between 0 and 160 °C, or which comprises

b) reacting a compound of the formula IV

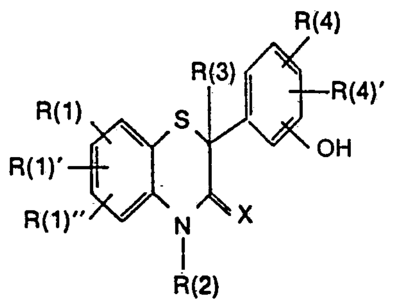

(IV)

in which R(1), R(1)′, R(1)″, R(2), R(3), R(4), R(4)′ and X have the same meaning as abovementioned, with a compound of the formula V

$$Z-(CH_2)_m-\underset{\displaystyle R(5)}{\underset{|}{(CH)_n}}-(CH_2)_p-R(6) \qquad (V)$$

in which Z is defined identically to Y under a), and in which R(5), R(6), R(7), R(8), R(9), R(10), R(11), R(12), m, n and p have the same meaning as abovementioned, either in a polar aprotic solvent in the presence of a strong base, at a temperature between −40 and +60 °C, or in a protic or aprotic polar organic solvent in the presence of a weak to moderately strong base, at a temperature between 0 and 160 °C.

2. The process as claimed in claim 1, wherein

R(1) and R(1)′ are identical or different and, independently of one another, denote hydrogen, methyl, ethyl, methoxy, ethoxy, chlorine, bromine, nitro or acetamido,

R(1)″ denotes hydrogen,

R(2) denotes hydrogen, ($C_1$–$C_6$)-alkyl, straight-chain or branched,
benzyl, phenethyl, allyl, phenyl,
4-methoxyphenyl, 3-methoxyphenyl,
3,4-methylenedioxyphenyl,
3,4,5-trimethoxyphenyl,
3,4-dimethoxyphenyl, cyclohexylmethyl,
cyclopentyl, cyclohexyl, cycloheptyl,
4-methoxybenzyl, 3,4-dimethoxybenzyl,
3,4,5-trimethoxybenzyl or
3,4-methylenedioxybenzyl,

R(3) denotes ($C_1$–$C_6$)-alkyl, straight-chain or branched, benzyl, phenylethyl, allyl, cyclopentyl or cyclohexyl,

R(4) denotes hydrogen, methyl, methoxy, ethoxy, chlorine, nitro, hydroxyl, acetamido or amino,

R(4)′ denotes hydrogen,

R(5) denotes hydrogen or methyl,

R(6) denotes a part structure from the following group,

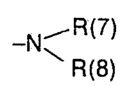

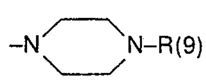

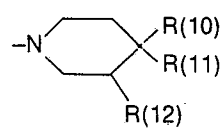

in which

R(7) denotes hydrogen, methyl, ethyl, propyl or isopropyl,

R(8) denotes hydrogen, methyl, ethyl, propyl, isopropyl, cyclopentylethyl, cyclohexylethyl, phenyl-($C_1$–$C_4$)-alkyl, it being possible for the phenyl radical to be substituted by one, two or three radicals from the group comprising ($C_1$–$C_4$)-alkyl, ($C_1$–$C_4$)-alkoxy, ($C_1$–$C_2$)-alkylenedioxy, halogen or hydroxyl, or pyridyl-($C_1$–$C_4$)-alkyl,

R(9) denotes as defined in claim 1,

R(10) denotes phenyl which can optionally be substituted by one, two or three radicals from the group comprising ($C_1$–$C_4$)-alkyl, ($C_1$–$C_4$)-alkoxy, ($C_1$–$C_2$)-alkylenedioxy, halogen or hydroxyl, or phenyl-($C_1$–$C_4$)-alkyl, it being possible for the phenyl radical to be substituted by one, two or three radicals from the group comprising ($C_1$–$C_4$)-alkyl, ($C_1$–$C_4$)-alkoxy, ($C_1$–$C_2$)-alkylenedioxy, halogen or hydroxyl,

R(11) denotes hydrogen and hydroxyl, methoxy or, together with R(12), denotes a bond,

R(12) denotes hydrogen or, together with R(11), denotes a bond;

in which formula I in addition

m denotes 1, 2 or 3,

n denotes 0 or 1,

p denotes 1, 2 or 3, and

X denotes oxygen.

3. The process as claimed in claim 2, wherein R(1) denotes hydrogen, methyl, methoxy, fluorine or chlorine,

R(1)′ and R(1)″ denote hydrogen,

R(2) denotes hydrogen, methyl, ethyl, propyl, isopropyl, butyl, sec.-butyl, isobutyl, benzyl, phenethyl, 4-methoxyphenyl, 3-methoxyphenyl, 3,4-dimethoxyphenyl, 3,4,5-trimethoxyphenyl, cyclohexylmethyl, cyclopentyl, cyclohexyl, cycloheptyl, 4-methoxybenzyl, 3,4-dimethoxybenzyl, 3,4,5-trimethoxybenzyl or 3,4-methylenedioxybenzyl,

R(3) denotes methyl, ethyl, propyl, isopropyl, butyl, sec.-butyl, isobutyl, benzyl, phenylethyl, allyl, cyclopentyl or cyclohexyl,

R(4) denotes hydrogen, methoxy, methyl, chlorine, nitro or hydroxyl,

R(4)′ denotes hydrogen,

R(5) denotes hydrogen,

R(6) denotes a part structure from the following group,

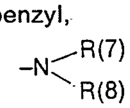

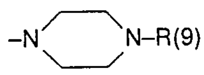

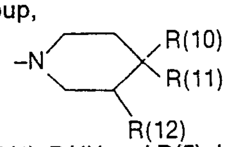

in which

R(7) denotes hydrogen or methyl,

R(8) denotes phenyl-($C_1$–$C_4$)-alkyl, it being possible for the phenyl radical to be substituted by one, two or three radicals from the group comprising methyl, methoxy, chlorine, methylenedioxy or hydroxyl,

R(9) is defined as indicated in claim 2,

R(10) denotes phenyl, it being possible for the phenyl radical to be substituted by one, two or three radicals from the group comprising methyl, methoxy, chlorine, methylenedioxy or hydroxyl,

R(11) denotes hydrogen, hydroxyl, methoxy or, together with R(12), denotes a bond, and

R(12) denotes hydrogen or, together with R(11), denotes a bond;

in which formula I in addition

m denotes 1, 2 or 3,

n denotes 0,

p denotes 0, 1 or 2, and

X denotes oxygen.

4. The process as claimed in claim 3, wherein R(1), R(1)′ and R(1)″ denote as defined in claim 3,

R(2) is hydrogen, methyl, ethyl, propyl, isopropyl or phenyl,

R(3) is methyl, ethyl, propyl, isopropyl, butyl, sec.-butyl, isobutyl, cyclopentyl or cyclohexyl,

R(4) is hydrogen, methoxy, methyl or chlorine,

R(4)′ and R(5) denote as defined in claim 3,

R(6) is a part structure from the following group

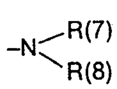

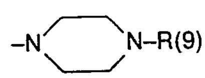

in which

R(7) is methyl,

R(8) denotes as defined in claim 3,

R(9) is phenyl-($C_1$–$C_4$)-alkyl, it being possible for the phenyl radical to be substituted by one, two or three radicals from the group comprising ($C_1$–$C_2$)-alkoxy, ($C_1$–$C_2$)-alkylenedioxy or hydroxyl,

m is 3,

X is oxygen,

p is 0 or 1.

5. The process as claimed in claim 4, wherein R(1) denotes hydrogen,

R(1)′ and R(1)″ denote as defined in claim 4,

R(2) denotes methyl,

R(3) denotes methyl, ethyl, isopropyl, isobutyl, sec.-butyl, cyclopentyl,

R(4), R(4)′ and R(5) denote as defined in claim 4,

R(6) denotes 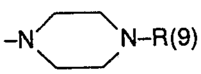

and

R(9) denotes phenyl-($C_1$–$C_3$)-alkyl, it being possible for the phenyl radical to be substituted by one, two or three methoxy groups,

X denotes oxygen.

6. The process as claimed in claim 1, wherein 2,3-dihydro-2,4-dimethyl-2-[2-[4-[4-[2-(3,4,5-trimethoxyphenyl)ethyl]piperazinyl]butoxy]phenyl]benzothiazin-3-one is prepared.

7. The process as claimed in claim 1, wherein 2,3-dihydro-2-ethyl-4-methyl-2-[2-[4-[4-[2-(3,4,5-trimethoxyphenyl)ethyl]piperazinyl]butoxy]phenyl]benzothiazin-3-one is prepared.

8. The process as claimed in claim 1, wherein, 2,3-dihydro-2-isopropyl-4-methyl-2-[2-[4-[4-[2-(3,4,5-trimethoxyphenyl)ethyl]piperazinyl]butoxy]phenyl]benzothiazin-3-one is prepared.

9. The process as claimed in claim 1, wherein (+)-2,3-dihydro-2-isopropyl-4-methyl-2-[2-[4-[4-[2-(3,4,5-trimethoxyphenyl)ethyl]piperazinyl]-butoxy]phenyl]benzothiazin-3-one is prepared.

**Revendications pour les Etats contractants: BE, CH, DE, FR, GB, IT, LI, LU, NL, SE**

1. Dérivés de benzothiazine de formule I

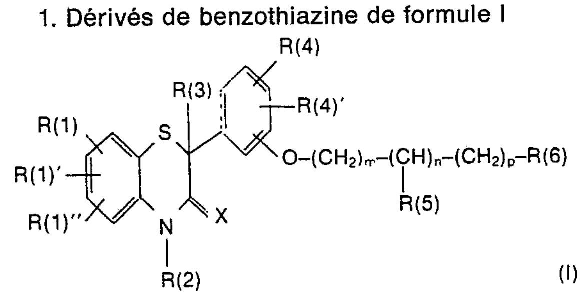

(I)

dans laquelle

R(1), R(1)′ et R(1)″ sont identiques ou différents, et représentent indépendamment les uns des autres un atome d'hydrogène ou d'halogène, ou un groupe alkyle en $C_1$–$C_4$, alcoxy en $C_1$–$C_3$, nitro, hydroxy, acétamido ou amino;

R(2) représente un atome d'hydrogène ou un radical alkyle en $C_1$–$C_{10}$ à chaîne droite ou ramifiée, alcényle en $C_3$–$C_{10}$ à chaîne droite ou ramifiée, phényle qui peut éventuellement être substitué par un, deux ou trois substituants choisis parmi des atomes d'halogène et des groupes alkyle en $C_1$–$C_4$, alcoxy en $C_1$–$C_3$, alkylène-($C_1$–$C_2$)-dioxy ou nitro; un radical phényl-alkyle-($C_1$–$C_4$), le noyau phényle pouvant être substitué par un, deux ou trois substituants choisi parmi des atomes d'halogène et des groupes alkyle en $C_1$–$C_4$, alcoxy en $C_1$–$C_3$, alkylène($C_1$–$C_2$)-dioxy ou nitro; un radical cycloalkyl($C_4$–$C_8$)-alkyle($C_1$–$C_4$), cycloalkyle en $C_4$–$C_8$;

R(3) représente un radical alkyle en $C_1$–$C_{10}$ à chaîne droite ou ramifiée, alcényle en $C_3$–$C_{10}$ à chaîne droite ou ramifiée, phényl-alkyle($C_1$–$C_4$), le reste phényle pouvant être substitué par un, deux ou trois substituants choisis parmi des atomes d'halogène et des groupes alkyle en $C_1$–$C_4$, alcoxy en $C_1$–$C_3$, alkylène($C_1$–$C_2$)dioxy ou nitro; un radical cycloalkyle en $C_4$–$C_8$, cycloalkyle($C_4$–$C_8$)-alkyle($C_1$–$C_4$);

R(4) et R(4)′ sont identiques ou différents, et représentent, indépendamment l'un de l'autre, un atome d'hydrogène ou d'halogène, ou un groupe alkyle en $C_1$–$C_4$, alcoxy en $C_1$–$C_3$, nitro, hydroxy, acétamido ou amino;

R(5) représente un atome d'hydrogène ou un groupe alkyle en $C_1$–$C_3$;

R(6) représente une structure élémentaire choisie parmi les suivantes

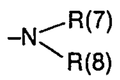 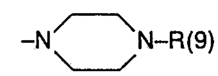 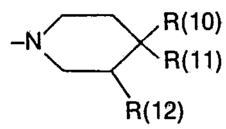 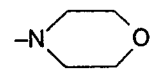

dans lesquelles

R(7) et R(8), identiques ou différents, représentent, indépendamment l'un de l'autre, un atome d'hydrogène ou un radical alkyle en $C_1$–$C_{10}$, cycloalkyle en $C_4$–$C_8$, cycloalkyl($C_4$–$C_8$)-alkyle($C_1$–$C_4$), phényl-alkyle($C_1$–$C_6$), le reste phényle pouvant être substitué par un, deux ou trois substituants choisis parmi des atomes d'halogène et des groupes alkyle en $C_1$–$C_4$, alcoxy en $C_1$–$C_4$, alkylène($C_1$–$C_2$)-dioxy ou hydroxy; ou un radical pyridyl-alkyle($C_1$–$C_4$);

R(9) représente un atome d'hydrogène ou un radical alkyle en $C_1$–$C_{10}$ à chaîne droite ou ramifiée, phényle, phényl-alkyle($C_1$–$C_4$), le reste phényle pouvant dans chaque cas être substitué par un, deux ou trois substituants choisis parmi des atomes d'halogène et des groupes alkyle en $C_1$–$C_4$, alcoxy en $C_1$–$C_4$, alkylène($C_1$–$C_2$)-dioxy ou hydroxy; un radical pyridyle, pyrimidinyle, alcanoyle en $C_1$–$C_5$, phényl-alcanoyle ($C_1$–$C_4$), benzoyle, le reste phényle pouvant dans chaque cas être substitué par un, deux ou trois substituants choisis parmi des atomes d'halogène et des groupes alkyle en $C_1$–$C_4$, alcoxy en $C_1$–$C_4$, alkylène($C_1$–$C_2$)-dioxy ou hydroxy;

R(10) représente un atome d'hydrogène ou un radical alkyle en $C_1$–$C_{10}$, phényle, phényl-alkyle($C_1$–$C_4$), le reste phényle pouvant dans chaque cas être substitué par un, deux ou trois substituants choisis parmi des atomes d'halogène et des groupes alkyle en $C_1$–$C_4$, alcoxy en $C_1$–$C_4$, alkylène($C_1$–$C_2$)-dioxy ou hydroxy;

R(11) représente un atome d'hydrogène ou un groupe hydroxy, alcoxy en $C_1$–$C_4$ ou, conjointement avec $R_{12}$, une liaison; et

R(12) représente un atome d'hydrogène ou, conjointement avec R(11), une liaison;
dans laquelle formule I, en outre

m vaut 1, 2, 3 ou 4,
n vaut 0 ou 1,
p vaut 0, 1, 2, 3 ou 4, et
X représente un atome d'oxygène,
et les sels des composés de formule I avec des acides physiologiquement acceptables.

2. Composé de formule I selon la revendication 1, caractérisé en ce que

R(1) et R(1)′ sont identiques ou différents, et représentent, indépendamment l'un de l'autre, un atome d'hydrogène ou de chlore ou de brome, ou un groupe méthyle, éthyle, méthoxy, éthoxy, nitro ou acétamido;

R(1)″ représente un atome d'hydrogène;

R(2) représente un atome d'hydrogène ou un radical alkyle en $C_1$–$C_6$ à chaîne droite ou ramifiée,
benzyle, phénéthyle, allyle, phényle,
4-méthoxyphényle, 3-méthoxyphényle,
3,4-méthylènedioxyphényle,
3,4,5-triméthoxyphényle, 3,4-diméthoxyphényle,
cyclohexylméthyle, cyclopentyle, cyclohexyle,
cycloheptyle, 4-méthoxybenzyle,
3,4-diméthoxybenzyle, 3,4,5-triméthoxybenzyle,
3,4-méthylènedioxybenzyle;

R(3) représente un radical alkyle en $C_1$–$C_6$ à chaîne droite ou ramifiée, benzyle, phényléthyle, allyle, cyclopentyle, cyclohexyle;

R(4) représente un atome d'hydrogène ou de chlore, ou un groupe méthyle, méthoxy, éthoxy, nitro, hydroxy, acétamido ou amino;

R(4)′ représente un atome d'hydrogène;

R(5) représente un atome d'hydrogène ou le groupe méthyle;

R(6) représente une structure élémentaire choisie parmi les suivantes

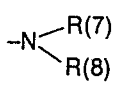 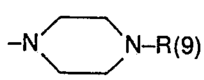 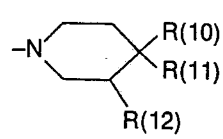

dans lesquelles

R(7) représente un atome d'hydrogène ou un groupe méthyle, éthyle, propyle, isopropyle;

R(8) représente un atome d'hydrogène ou un radical méthyle, éthyle, propyle, isopropyle, cyclopentylheptyle, cyclohexyléthyle, phényl-alkyle($C_1$–$C_4$), le reste phényle pouvant être substitué par un, deux ou trois substituants choisis parmi des atomes d'halogène et des groupes alkyle en $C_1$–$C_4$, alcoxy en $C_1$–$C_4$, alkylène($C_1$–$C_2$)-dioxy et hydroxy; ou un radical pyridyl-alkyle($C_1$–$C_4$);

R(9) est tel que défini dans la revendication 1;

R(10) représente un radical phényle qui peut éventuellement être substitué par un, deux ou trois substituants choisis parmi des atomes d'halogène et des groupes alkyle en $C_1$–$C_4$, alcoxy en $C_1$–$C_4$, alkylène($C_1$–$C_2$)-dioxy ou hydroxy; un radical phényl-alkyle($C_1$–$C_4$), le reste phényle pouvant être substitué par un, deux ou trois substituants choisis parmi des atomes d'halogène et des groupes alkyle en $C_1$–$C_4$, alcoxy en $C_1$–$C_4$, alkylène($C_1$–$C_2$)-dioxy et hydroxy;

R(11) représente un atome d'hydrogène ou un groupe hydroxy, méthoxy ou, conjointement avec R(12), une liaison;

R(12) représente un atome d'hydrogène ou, conjointement avec R(11), une liaison;

dans laquelle formule I, en outre

m vaut 1, 2 ou 3,

n vaut 0 ou 1,

p vaut 1, 2 ou 3, et

X représente un atome d'oxygène;

et les sels de ce composé de formule I avec des acides physiologiquement acceptables.

3. Composé de formule I selon la revendication 2, caractérisé en ce que

R(1) représente un atome d'hydrogène, de fluor ou de chlore ou un groupe méthyle ou méthoxy;

R(1)′ et R(1)″ représentent chacun un atome d'hydrogène;

R(2) représente un atome d'hydrogène ou un groupe
méthyle, éthyle, propyle, isopropyle, butyle, sec-butyle, isobutyle, benzyle, phénéthyle, 4-méthoxyphényle, 3-méthoxyphényle, 3,4-diméthoxyphényle, 3,4,5-triméthoxyphényle, cyclohexylméthyle, cyclopentyle, cyclohexyle, cycloheptyle, 4-méthoxybenzyle, 3,4-diméthoxybenzyle, 3,4,5-triméthoxybenzyle, 3,4-méthylènedioxybenzyle;

R(3) représente un groupe méthyle, éthyle, propyle, isopropyle, butyle, sec-butyle, isobutyle, benzyle, phénéthyle, allyle, cyclopentyle, cyclohexyle;

R(4) représente un atome d'hydrogène ou de chlore, ou un groupe méthoxy, méthyle, nitro ou hydroxy;

R(4)′ représente un atome d'hydrogène;

R(5) représente un atome d'hydrogène;

R(6) représente une structure élémentaire choisie parmi les suivantes

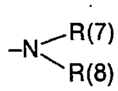

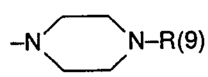

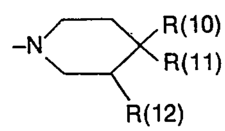

dans lesquelles

R(7) représente un atome d'hydrogène ou le groupe méthyle;

R(8) représente un radical phényl-alkyle($C_1$–$C_4$), le reste phényle pouvant être substitué par un, deux ou trois substituants choisis parmi un atome de chlore ou un groupe méthyle, méthoxy, méthylènedioxy ou hydroxy;

R(9) est tel que défini dans la revendication 2;

R(10) représente un radical phényle, le radical phényle pouvant être substitué par un, deux ou trois substituants choisis parmi un atome de chlore ou un groupe méthyle, méthoxy, méthylènedioxy ou hydroxy;

R(11) représente un atome d'hydrogène ou un groupe hydroxy, méthoxy, ou, conjointement avec R(12), une liaison; et

R(12) représente un atome d'hydrogène ou, conjointement avec R(11), une liaison;

dans laquelle formule I, en outre

m vaut 1, 2 ou 3,

n vaut 0,

p vaut 0, 1 ou 2, et

X représente un atome d'oxygène;

et les sels de ce composé de formule I avec des acides physiologiquement acceptables.

4. Composé de formule I selon la revendication 3, caractérisé en ce que

R(1), R(1)′ et R(1)″ sont tels que définis dans la revendication 3;

R(2) représente un atome d'hydrogène ou un groupe méthyle, éthyle, propyle, isopropyle, phényle;

R(3) représente un groupe méthyle, éthyle, propyle, isopropyle, butyle, sec-butyle, isobutyle, cyclopentyle, cyclohexyle;

R(4) représente un atome d'hydrogène ou de chlore, ou un groupe méthoxy ou méthyle;

R(4)′ est tel que défini dans la revendication 3;

R(5) est tel que défini dans la revendication 3;

R(6) représente une structure élémentaire choisie parmi les suivantes

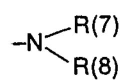

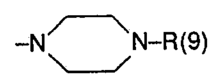

R(7) représente le groupe méthyle;

R(8) est tel que défini dans la revendication 3;

R(9) représente un radical phényl-alkyle($C_1$–$C_4$), le reste phényle pouvant être substitué par un, deux ou trois substituants choisis parmi des groupes alcoxy en $C_1$–$C_2$, alkylène($C_1$–$C_2$)-dioxy ou hydroxy;

m vaut 3,

X représente un atome d'oxygène,

p vaut 0 ou 1,

de ce composé avec des acides physiologiquement acceptables.

5. Composé de formule I selon la revendication 4, caractérisé en ce que

R(1) représente un atome d'hydrogène;

R(1)' et R(1)'' sont tels que définis dans la revendication 4;

R(2) représente le groupe méthyle;

R(3) représente un groupe méthyle, éthyle, isopropyle, isobutyle, sec-butyle, cyclopentyle;

R(4), R(4)' et R(5) sont tels que définis dans la revendication 4;

R(6) représente 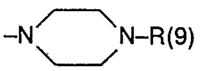

R(9) représente un radical phényl-alkyle($C_1$–$C_3$), le reste phényle pouvant être substitué par un, deux ou trois groupes méthoxy;

X représente un atome d'oxygène;

et physiologiquement acceptables de celui-ci.

6. 2,3-dihydro-2,4-diméthyl-2-[2-[4-[4-[2-(3,4,5-triméthoxyphényl)-éthyl]-pipérazinyl]-butoxy]-phényl]-benzothiazine-3-one et sels physiologiquement acceptables de celle-ci.

7. 2,3-dihydro-2-éthyl-4-méthyl-2-[2-[4-[4-[2-(3,4,5-triméthoxyphényl)-éthyl]-pipérazinyl]-butoxy]-phényl]-benzothiazine-3-one et sels physiologiquement acceptables de celle-ci.

8. 2,3-dihydro-2-isopropyl-4-méthyl-2-[2-[4-[4-[2-(3,4,5-triméthoxyphényl)-éthyl]-pipérazinyl]-butoxy]-phényl]-benzothiazine-3-one et sels physiologiquement acceptables de celle-ci.

9. (+)-2,3-dihydro-2-isopropyl-4-méthyl-2-[2-[4-[4-[2-(3,4,5-triméthoxyphényl)-éthyl]-pipérazinyl]-butoxy]-phényl]-benzothiazine-3-one et sels physiologiquement acceptables de celle-ci.

10. Procédé pour la préparation de composés de formule I selon la revendication 1, caractérisé en ce que

a) on fait réagir un composé de formule II

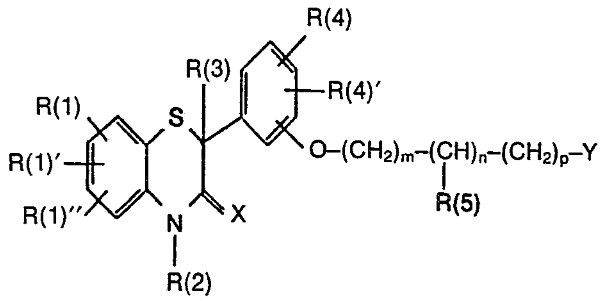

(II)

dans laquelle R(1), R(1)', R(1)'', R(2), R(3), R(4), R(4)', R(5), X, m, n et p ont les mêmes significations que dans la revendication 1, et dans laquelle Y représente un groupe séparable qui peut être séparé par déplacement nucléophile, en particulier un atome d'halogène, un groupe sulfonyle, de préférence un groupe méthanesulfonyle, un groupe benzènesulfonyle, un groupe toluènesulfonyle ou un groupe trifluorométhanesulfonyle, avec un des composés de formules IIIa, IIIb, IIIc ou IIId,

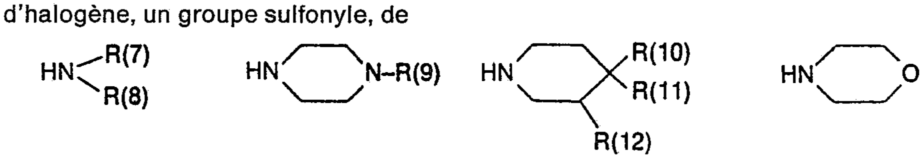

(IIIa) (IIIb) (IIIc) (IIId)

dans lesquelles R(7), R(8), R(9), R(10), R(11) et R(12) ont les mêmes significations que dans la revendication 1, dans des conditions d'une substitution nucléophile, de préférence dans un solvant organique polaire, en présence ou non d'une base auxiliaire pour la fixation de l'acide formé, à une température comprise entre 0 et 160 °C, de préférence entre 20 et 120 °C, ou en ce que

b) on fait réagir un composé de formule IV

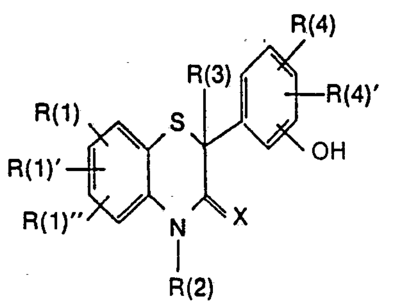

(IV)

dans laquelle R(1), R(1)', R(1)'', R(2), R(3), R(4), R(4)' et X ont les mêmes significations que dans la revendication 1, avec un composé de formule V

$$Z{-}(CH_2)_m{-}(\underset{\displaystyle R(5)}{\underset{|}{C}}H)_n{-}(CH_2)_p{-}R(6) \qquad (V)$$

dans laquelle Z a la même définition que Y en a), et dans laquelle R(5), R(6), R(7), R(8), R(9), R(10), R(11), R(12), m, n et p ont les mêmes significations que dans la revendication 1, soit dans un solvant aprotique polaire, en présence d'une base forte, à une température comprise entre −40 et +60 °C, soit dans un solvant organique polaire protique ou aprotique, en présence d'une base faible à moyennement forte, à une température comprise entre 0 et 160 °C.

11. Composés de formule générale II

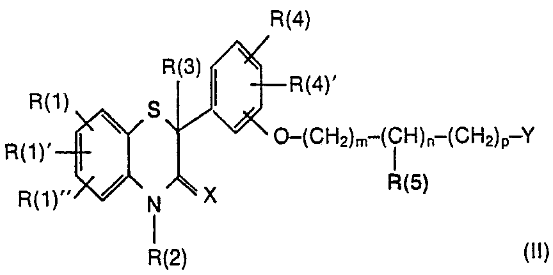

(II)

dans laquelle R(1), R(1)', R(1)'', R(2), R(3), R(4), R(4)', R(5), X, m, n et p ont les mêmes significations que dans la revendication 1, et dans laquelle Y représente un groupe séparable qui peut être séparé par déplacement nucléophile, en particulier un atome d'halogène, un groupe sulfonyle, de préférence un groupe méthanesulfonyle, un groupe benzènesulfonyle, un groupe toluènesulfonyle ou un groupe trifluorométhanesulfonyle.

12. Médicament caractérisé en ce qu'il contient ou consiste en un composé de formule I selon la revendication 1, ou un sel de ce composé avec un acide physiologiquement acceptable.

13. Utilisation d'un composé de formule I selon la revendication 1, ou d'un de ses sels avec un acide physiologiquement acceptable, pour la préparation d'un médicament pour le traitement de troubles du bilan calcique d'un organisme humain ou animal.

**Revendications pour l'Etat Contractant: AT**

1. Procédé pour la préparation de composés de formule I

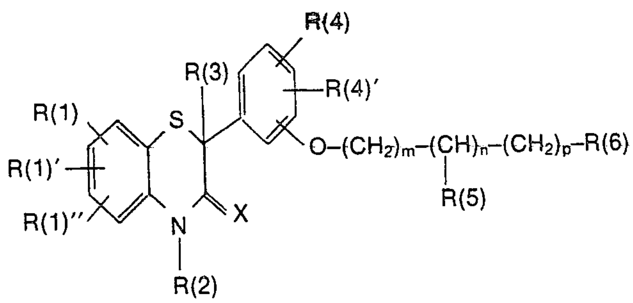

(I)

dans laquelle

R(1), R(1)' et R(1)'' sont identiques ou différents, et représentent, indépendamment les uns des autres, un atome d'hydrogène ou d'halogène, ou un groupe alkyle en $C_1$–$C_4$, alcoxy en $C_1$–$C_3$, nitro, hydroxy, acétamido ou amino;

R(2) représente un atome d'hydrogène ou un radical alkyle en $C_1$–$C_{10}$ à chaîne droite ou ramifiée, alcényle en $C_3$–$C_{10}$ à chaîne droite ou ramifiée, phényle qui peut éventuellement être substitué par un, deux ou trois substituants choisis parmi des atomes d'halogène et des groupes alkyle en $C_1$–$C_4$, alcoxy en $C_1$–$C_3$, alkylène($C_1$–$C_2$)-dioxy ou nitro; un radical phényl-alkyle($C_1$–$C_4$), le noyau phényle pouvant être substitué par un, deux ou trois substituants choisi parmi des atomes d'halogène et des groupes alkyle en $C_1$–$C_4$, alcoxy en $C_1$–$C_3$, alkylène($C_1$–$C_2$)-dioxy ou nitro; un radical cycloalkyl($C_4$–$C_8$)-alkyle($C_1$–$C_4$), cycloalkyle en $C_4$–$C_8$;

R(3) représente un radical alkyle en $C_1$–$C_{10}$ à chaîne droite ou ramifiée, alcényle en $C_3$–$C_{10}$ à chaîne droite ou ramifiée, phényl-alkyle($C_1$–$C_4$), le reste phényle pouvant être substitué par un, deux ou trois substituants choisis parmi des atomes d'halogène et des groupes alkyle en $C_1$–$C_4$, alcoxy en $C_1$–$C_3$, alkylène($C_1$–$C_2$)dioxy ou nitro; un radical cycloalkyle en $C_4$–$C_8$, cycloalkyle($C_4$–$C_8$)-alkyle($C_1$–$C_4$);

R(4) et R(4)' sont identiques ou différents, et représentent, indépendamment l'un de l'autre, un atome d'hydrogène ou d'halogène, ou un groupe alkyle en $C_1$–$C_4$, alcoxy en $C_1$–$C_3$, nitro, hydroxy, acétamido ou amino;

R(5) représente un atome d'hydrogène ou un groupe alkyle en $C_1$–$C_3$);

R(6) représente une structure élémentaire choisie parmi les suivantes

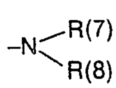

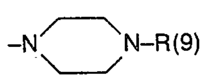

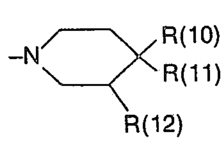

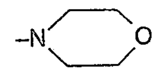

dans lesquelles

R(7) et R(8), identiques ou différents, représentent, indépendamment l'un de l'autre, un atome d'hydrogène ou un radical alkyle en $C_1$–$C_{10}$, cycloalkyle en $C_4$–$C_8$, cycloalkyl($C_4$–$C_8$)-alkyle($C_1$–$C_4$), phényl-alkyle($C_1$–$C_6$), le reste phényle pouvant être substitué par un, deux ou trois substituants choisis parmi des atomes d'halogène et des groupes alkyle en $C_1$–$C_4$, alcoxy en $C_1$–$C_4$, alkylène($C_1$–$C_2$)-dioxy ou hydroxy; ou un radical pyridyl-alkyle($C_1$–$C_4$);

R(9) représente un atome d'hydrogène ou un radical alkyle en $C_1$–$C_{10}$ à chaîne droite ou ramifiée, phényle, phényl-alkyle($C_1$–$C_4$), le reste phényle pouvant dans chaque cas être substitué par un, deux ou trois substituants choisis parmi des atomes d'halogène et des groupes alkyle en $C_1$–$C_4$, alcoxy en $C_1$–$C_4$, alkylène($C_1$–$C_2$)-dioxy ou hydroxy; un radical pyridyle, pyrimidinyle, alcanoyle en $C_1$–$C_5$, phényl-alcanolyle($C_1$–$C_4$), benzoyle, le reste phényle pouvant dans chaque cas être substitué par un, deux ou trois substituants

choisis parmi des atomes d'halogène et des groupes alkyle en $C_1$–$C_4$, alcoxy en $C_1$–$C_4$, alkylène($C_1$–$C_2$)-dioxy ou hydroxy;

R(10) représente un atome d'hydrogène ou un radical alkyle en $C_1$–$C_{10}$, phényle, phényl-alkyle($C_1$–$C_4$), le reste phényle pouvant être substitué par un, deux ou trois substituants choisis parmi des atomes d'halogène et des groupes alkyles en $C_1$–$C_4$, alcoxy en $C_1$–$C_4$, alkylène($C_1$–$C_2$)-dioxy ou hydroxy;

R(11) représente un atome d'hydrogène ou un groupe hydroxy, alcoxy en $C_1$–$C_4$ ou, conjointement avec R(12), une liaison; et

R(12) représente un atome d'hydrogène ou, conjointement avec R(11), une liaison;

dans laquelle formule I, en outre

m vaut 1, 2, 3 ou 4,

n vaut 0 ou 1,

p vaut 0, 1, 2, 3 ou 4, et

X représente un atome d'oxygène,

caractérisé en ce que

a) on fait réagir un composé de formule II

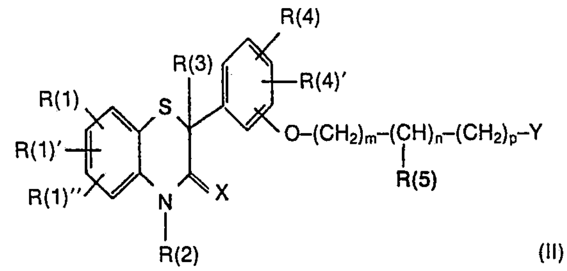

(II)

dans laquelle R(1), R(1)′, R(1)″, R(2), R(3), R(4), R(4)′, R(5), X, m, n et p ont les mêmes significations qu'indiqué plus haut, et dans laquelle Y représente un groupe séparable qui peut être séparé par déplacement nucléophile, en particulier un atome d'halogène, un groupe sulfonyle, de préférence un groupe méthanesulfonyle, un groupe benzènesulfonyle, un groupe toluènesulfonyle ou un groupe trifluorométhanesulfonyle, avec un des composés de formules IIIa, IIIb, IIIc ou IIId,

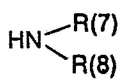

(IIIa)

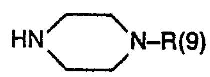

(IIIb)

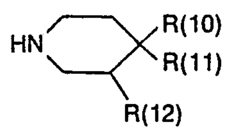

(IIIc)

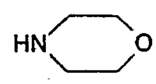

(IIId)

dans lesquelles R(7), R(8), R(9), R(10), R(11) et R(12) ont les mêmes significations qu'indiqué plus haut, dans des conditions d'une substitution nucléophile, de préférence dans un solvant organique polaire, en présence ou non d'une base auxiliaire pour la fixation de l'acide formé, à une température comprise entre 0 et 160 °C, ou en ce que

b) on fait réagir un composé de formule IV

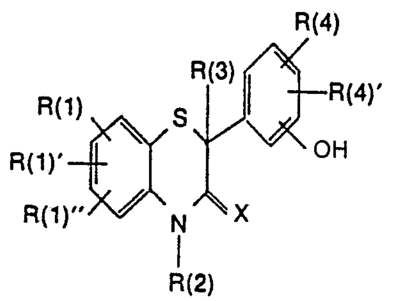

(IV)

dans laquelle R(1), R(1)′, R(1)″, R(2), R(3), R(4), R(4)′ et X ont les mêmes significations que celles données plus haut, avec un composé de formule V

$$Z-(CH_2)_m-(\underset{\substack{|\\R(5)}}{C}H)_n-(CH_2)_p-R(6) \qquad (V)$$

dans laquelle Z a la même définition que Y en a), et dans laquelle R(5), R(6), R(7), R(8), R(9), R(10), R(11), R(12), m, n et p ont les mêmes significations que celles données plus haut, soit dans un solvant aprotique polaire, en présence d'une base forte, à une température comprise entre −40 et +60 °C, soit dans un solvant organique polaire protique ou aprotique, en présence d'une base faible à moyennement forte, à une température comprise entre 0 et 160 °C.

2. Procédé selon la revendication 1, caractérisé en ce que

R(1) et R(1)′ sont identiques ou différents, et représentent, indépendamment l'un de l'autre, un atome d'hydrogène ou de chlore ou de brome, ou un groupe méthyle, éthyle, méthoxy, éthoxy, nitro ou acétamido;

R(1)″ représente un atome d'hydrogène,

R(2) représente un atome d'hydrogène ou un radical alkyle en $C_1$–$C_6$ à chaîne droite ou ramifiée,
benzyle, phénéthyle, allyle, phényle,
4-méthoxyphényle, 3-méthoxyphényle,
3,4-méthylènedioxyphényle,
3,4,5-triméthoxyphényle, 3,4-diméthoxyphényle,
cyclohexylméthyle, cyclopentyle, cyclohexyle,
cycloheptyle, 4-méthoxybenzyle,
3,4-diméthoxybenzyle, 3,4,5-triméthoxybenzyle,
3,4-méthylènedioxybenzyle;

R(3) représente un radical alkyle en $C_1$–$C_6$ à chaîne droite ou ramifiée, benzyle, phényléthyle, allyle, cyclopentyle, cyclohexyle;

R(4) représente un atome d'hydrogène ou de chlore, ou un groupe méthyle, méthoxy, éthoxy, nitro, hydroxy, acétamido ou amino;

R(4)′ représente un atome d'hydrogène,

R(5) représente un atome d'hydrogène ou le groupe méthyle;

R(6) représente une structure élémentaire choisie parmi les suivantes

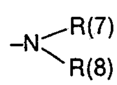
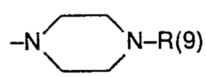
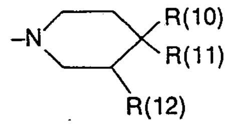

dans lesquelles

R(7) représente un atome d'hydrogène ou un groupe méthyle, éthyle, propyle, isopropyle;

R(8) représente un atome d'hydrogène ou un radical méthyle, éthyle, propyle, isopropyle, cyclopentylheptyle, cyclohexyléthyle, phényl-alkyle($C_1$–$C_4$), le reste phényle pouvant être substitué par un, deux ou trois substituants choisis parmi des atomes d'halogène et des groupes alkyle en $C_1$–$C_4$, alcoxy en $C_1$–$C_4$, alkylène($C_1$–$C_2$)-dioxy ou hydroxy; ou un radical pyridyl-alkyle($C_1$–$C_4$);

R(9) est tel que défini dans la revendication 1;

R(10) représente un radical phényle qui peut éventuellement être substitué par un, deux ou trois substituants choisis parmi des atomes d'halogène et des groupes alkyle en $C_1$–$C_4$, alcoxy en $C_1$–$C_4$, alkylène($C_1$–$C_2$)-dioxy ou hydroxy; un radical phényl-alkyle($C_1$–$C_4$), le reste phényle pouvant être substitué par un, deux ou trois substituants choisis parmi des atomes d'halogène et des groupes alkyle en $C_1$–$C_4$, alcoxy en $C_1$–$C_4$, alkylène($C_1$–$C_2$)-dioxy ou hydroxy;

R(11) représente un atome d'hydrogène ou un groupe hydroxy, méthoxy, ou, conjointement avec R(12), une liaison;

R(12) représente un atome d'hydrogène ou, conjointement avec R(11), une liaison;

dans laquelle formule I, en outre,

m vaut 1, 2 ou 3,

n vaut 0 ou 1,

p vaut 1, 2 ou 3, et

X représente un atome d'oxygène.

3. Procédé selon la revendication 2, caractérisé en ce que

R(1) représente un atome d'hydrogène, de fluor ou de chlore ou un groupe méthyle ou méthoxy;

R(1)′ et R(1)″ représentent chacun un atome d'hydrogène;

R(2) représente un atome d'hydrogène ou un groupe
méthyle, éthyle, propyle, isopropyle, butyle,
sec-butyle, isobutyle, benzyle, phénéthyle,
4-méthoxyphényle, 3-méthoxyphényle,
3,4-diméthoxyphényle, 3,4,5-triméthoxyphényle,
cyclohexylméthyle, cyclopentyle, cyclohexyle,
cycloheptyle, 4-méthoxybenzyle,
3,4-diméthoxybenzyle, 3,4,5-triméthoxybenzyle,
3,4-méthylènedioxybenzyle;

R(3) représente un groupe méthyle, éthyle, propyle, isopropyle, butyle, sec-butyle, isobutyle, benzyle, phénéthyle, allyle, cyclopentyle, cyclohexyle;

R(4) représente un atome d'hydrogène ou de chlore, ou un groupe méthoxy, méthyle, nitro ou hydroxy;

R(4)′ représente un atome d'hydrogène;

R(5) représente un atome d'hydrogène;

R(6) représente une structure élémentaire choisie parmi les suivantes

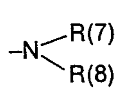
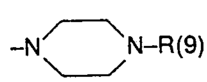
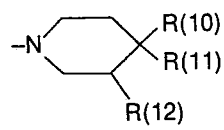

dans lesquelles

R(7) représente un atome d'hydrogène ou le groupe méthyle;

R(8) représente un radical phényl-alkyle($C_1$–$C_4$), le reste phényle pouvant être substitué par un, deux ou trois substituants choisis parmi un atome de chlore ou un groupe méthyle, méthoxy, méthylènedioxy ou hydroxy;

R(9) est tel que défini dans la revendication 2;

R(10) représente un radical phényle, le radical phényle pouvant être substitué par un, deux ou trois substituants choisis parmi un atome de chlore ou un groupe méthyle, méthoxy, méthylènedioxy ou hydroxy;

R(11) représente un atome d'hydrogène ou un groupe hydroxy, méthoxy, ou, conjointement avec R(12), une liaison; et

R(12) représente un atome d'hydrogène ou, conjointement avec R(11), une liaison;

dans laquelle formule I, en outre

m vaut 1, 2 ou 3,

n vaut 0,

p vaut 0, 1 ou 2, et

X représente un atome d'oxygène.

4. Procédé selon la revendication 3, caractérisé en ce que

R(1), R(1)′ et R(1)″ sont tels que définis dans la revendication 3;

R(2) représente un atome d'hydrogène ou un groupe méthyle, éthyle, propyle, isopropyle, phényle;

R(3) représente un groupe méthyle, éthyle, propyle, isopropyle, butyle, sec-butyle, isobutyle, cyclopentyle, cyclohexyle;

R(4) représente un atome d'hydrogène ou de chlore, ou un groupe méthoxy ou méthyle;

R(4)′ et R(5) sont tels que définis dans la revendication 3;

R(6) représente une structure élémentaire choisie parmi les suivantes

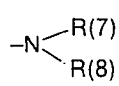
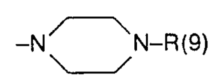

R(7) représente le groupe méthyle;

R(8) est tel que défini dans la revendication 3;

R(9) représente un radical phényl-alkyle($C_1$–$C_4$), le reste phényle pouvant être substitué par un,

deux ou trois substituants choisis parmi des groupes alcoxy en $C_1$–$C_2$, alkylène($C_1$–$C_2$)-dioxy ou hydroxy;

m vaut 3,

X représente un atome oxygène,

p vaut 0 ou 1.

5. Procédé selon la revendication 4, caractérisé en ce que

R(1) représente un atome d'hydrogène;

R(1)′ et R(1)″ sont tels que définis dans la revendication 4;

R(2) représente le groupe méthyle;

R(3) représente un groupe méthyle, éthyle, isopropyle, isobutyle, sec-butyle, cyclopentyle;

R(4), R(4)′ et R(5) sont tels que définis dans la revendication 4;

R(6) représente 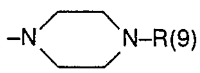

R(9) représente un radical phényl-alkyle($C_1$–$C_3$), le reste phényle pouvant être substitué par un, deux ou trois groupes méthoxy;

X représente un atome d'oxygène.

6. Procédé selon la revendication 1, caractérisé en ce que l'on prépare la 2,3-dihydro-2,4-diméthyl-2-[2-[4-[4-[2-(3,4,5-triméthoxyphényl)-éthyl]-pipérazinyl]-butoxy]-phényl]-benzothiazine-3-one.

7. Procédé selon la revendication 1, caractérisé en ce que l'on prépare la 2,3-dihydro-2-éthyl-4-méthyl-2-[2-[4-[4-[2-(3,4,5-triméthoxyphényl)-éthyl]-pipérazinyl]-butoxy]-phényl]-benzothiazine-3-one.

8. Procédé selon la revendication 1, caractérisé en ce que l'on prépare la 2,3-dihydro-2-isopropyl-4-méthyl-2-[2-[4-[4-[2-(3,4,5-triméthoxyphényl)-éthyl]-pipérazinyl]-butoxy]-phényl]-benzothiazine-3-one.

9. Procédé selon la revendication 1, caractérisé en ce que l'on prépare la (+)-2,3-dihydro-2-isopropyl-4-méthyl-2-[2-[4-[4-[2-(3,4,5-triméthoxyphényl)-éthyl]-pipérazinyl]-butoxy]-phényl]-benzothiazine-3-one.